# EUROPEAN PATENT SPECIFICATION

(11) **EP 2 024 511 B1**
(45) Date of publication and mention of the grant of the patent: **16.03.2011**
(21) Application number: 07777420.6
(22) Date of filing: 06.06.2007
(51) Int. Cl.: C12Q 1/68, G01N 33/53

(54) **CHOLESTEROL-REGULATING COMPLEX OF SIRT1 AND LXR AND METHODS OF USE**
CHOLESTERINREGULIERENDER KOMPLEX AUS SIRT1 UND LXR UND SEINE VERWENDUNG
COMPLEXE DE SIRT1 ET LXR REGULANT LE CHOLESTEROL ET PROCEDES D'UTILISATION

(30) Priority: 06.06.2006 US 811154 P
(43) Date of publication of application: 18.02.2009
(73) Proprietor: Massachusetts Institute of Technology, Cambridge, MA 02139 (US)
(72) Inventor: GUARENTE, Leonard, Newton, MA 02466-3005 (US); KRIEGER, Monty, Needham, MA 02494-2122 (US); LI, Xiaoling, Chapel Hill, NC 27516 (US)
(74) Representative: Fleck, Barbara
(86) International application number: PCT/US2007/013364
(87) International publication number: WO 2007/146023

(56) References cited:
- US-A- 5 998 131
- US-A1- 2003 228 607
- US-A1- 2005 136 429
- US-B1- 6 835 563
- "Interventions in Aging and Age-Related Diseases: The Present and the Future" AGE, vol. 28, no. 1, March 2006 (2006-03), pages 1-75, XP019411239
- ABEDIN S A ET AL: "Environmental sensing capacity by VDR and related nuclear receptors" ANTICANCER RESEARCH, vol. 25, no. 3B, 2005, page 2279, XP002561483 & 2ND INTERNATIONAL SYMPOSIUM ON VITAMIN D ANALOGS IN CANCER PREVENTION AND THERAPY; LUBECK, GERMANY

## Description

### BACKGROUND OF THE INVENTION

Cholesterol homeostasis is maintained by a balance between dietary intake, *de novo* synthesis, transport, metabolism and excretion. Low levels of high density lipoprotein (HDL) and high levels of low density lipoprotein (LDL) are associated with hypercholesterolemia, hypertriglyceridemia, and increased risk of cardiovascular disease¹ and Alzheimer's disease ²⁻⁶, age-associated diseases that are major causes of mortality in middle-aged and older people. Both genetic factors and the environment contribute to the progression of cardiovascular disease and Alzheimer's disease, and the risk of these disorders rises with age. However, the underlying mechanisms by which genetic factors sense the environment to mediate these ageing-associated diseases are poorly understood.

Silent information regulator 2 (Sir2) is a critical regulator of life span in response to environmental changes. The Sir2 gene is a longevity determinant in yeast, *C. elegans⁷,* and Drosophila ^{8,9}. In both yeast ^{10,11} and Drosophila ^{8,9}, Sir2 activity is also required for lifespan extension provided by calorie restriction. Biochemically, Sir2 and its homologues (sirtuins) are a group of highly conserved NAD⁺-dependent protein deacetylases ¹²⁻¹⁴. The requirement for NAD⁺ may enable sirtuins to monitor cellular metabolism and modulate cellular processes that affect ageing. In mammals, SIRT1, a mammalian orthologue of the Sir2 protein, has multiple protein substrates and is able to regulate ageing-related processes, such as cell cycle, apoptosis, oxidative stress response, and neurodegeneration ¹⁵⁻²⁰. In these processes, SIRT1 is able to shift the balance between cell death and cell survival, thereby providing stress resistance ²¹.

Cholesterol and fat metabolism are regulated by many common cellular and environmental factors. Peroxisome Proliferator-Activated Receptors (PPARs) and Liver X Receptors (LXRs), two subclasses of lipid-sensing nuclear receptors, play critical roles in lipid and carbohydrate metabolism. Recent studies showed that in white fat tissue, SIRT1 interacts with the nuclear receptor PPARγ through nuclear receptor co-repressor (N-CoR) and promotes the fat mobilization upon food deprivation ²². SIRT1 is also able to interact with and modify the PPARγ coactivator PGC-1α to regulate hepatic glucose homeostasis ^{23,24}. U. S. Patent 6,048,903 reports that the level of heavy density lipoproteins (HDL) in the blood of a human subject can be in eased by administering trans-resveratrol to the subject, and that this substance reduces the level of light density lipoproteins (LDL) in the blood of the subject.

The involvement of SIRT1 in fat metabolism and glucose homeostasis affords the potential that this protein could also regulate cholesterol homeostasis in mammals. The present invention addresses these novel mechanisms of cholesterol homeostasis.

### SUMMARY OF THE INVENTION

The present invention relates generally to compositions and methods useful in the field of cholesterol homeostasis and reverse cholesterol transport.

In a first aspect the invention provides an isolated complex that contains a mammalian SIRT1 protein and a mammalian LXR protein. In certain embodiments this complex additionally contains an LXR response element.

In a related aspect the invention provides a method of forming a complex that contains a mammalian SIRT1 protein and a mammalian LXR protein. The method includes combining compositions containing a mammalian SIRT1 protein, a mammalian LXR protein and a fragment of a cellular nucleic acid that includes a LXR response element.

In still a further aspect the invention provides a method of identifying an agent that modulates formation of a complex comprising a mammalian SIRT1 protein and a mammalian LXR protein. This method includes steps of
combining compositions that contain a mammalian SIRT1 protein, a mammalian LXR protein and a fragment of a cellular nucleic acid that includes a LXR response element, thus providing a complex composition;
further either contacting one of the composition containing the mammalian SIRT1 protein, the composition containing the mammalian LXR protein, or the composition containing the cellular nucleic acid fragment with a fourth composition including the agent prior to the combining step, or contacting the complex composition with the fourth composition including the agent after the combining step; and
determining whether formation of the complex is modulated by the agent.
In certain embodiments of this method the agent increases formation of the complex. In additional embodiments, the determining is conducted by comparison with a control composition not containing the agent.

US6,835,563 relates to ABC1 polypeptides and methods for increasing cholesterol efflux. US2005/136429 discloses evaluating a SRIT1 molecule in a subject.

Also described is a method of increasing the ratio of cholesterol bound to high density lipoprotein (HDL) to total cholesterol in the plasma of a mammal wherein the method includes administering to the mammal an agent that stimulates SIRT1 activity. For example the agent includes T0901317.

Also described is a method of increasing the ratio of cholesterol bound to high density lipoprotein (HDL) to total cholesterol in the plasma of a mammal wherein the method includes administering to the mammal an agent that promotes formation of a complex containing a mammalian SIRT1 protein and a mammalian LXR protein. For example, the agent includes 22(R)-hydroxycholesterol or 9-cis retinoic acid or both.

Also described is a method of promoting ABCA1-mediated cholesterol efflux from a mammalian cell wherein the method includes introducing into the cell a nucleic acid that contains a sequence encoding a protein deacetylase. For example the protein deacetylase is a eukaryotic Sir2 or a mammalian SIRT1. Other examples of this method include a step of further contacting the cell with an agent that stimulates SIRT1 activity, such as T0901317. The cell may be further contacted with an agent that promotes formation of a complex containing a mammalian SIRT1 protein and a mammalian LXR protein. For example the complex-promoting includes 22(R)-hydroxycholesterol or 9-cis retinoic acid, or both.

Also described is a method of treating a subject deemed to have a level of SIRT1 activity that is below normal. Such a subject exhibits a level of HDL-cholesterol, and a ratio of HDL-cholesterol to LDL-cholesterol, that are deemed to be below normal. The method includes administering a nucleic acid encoding species-specific SIRT1 to the subject wherein the nucleic acid is effective to express a therapeutically effective amount of SIRT1 in a cell of the subject.

Also described is a method for assessing whether a candidate substance modulates an LXR-dependent process. The method includes transfecting a cell with a plasmid harboring a reporter gene operably driven by an LXRE; contacting the cell with the candidate; and determining whether the candidate modulates the expression of the reporter gene in comparison with a cell not contacted with the candidate; such that a difference detected between the presence and absence of the candidate indicates that the candidate modulates the LXR-dependent process.

In an additional aspect the invention provides a method for assessing whether a candidate substance modulates an SIRT1-dependent effect of an LXR. This method includes transfecting a cell with a plasmid harboring an SIRT1 gene; further transfecting the cell with a plasmid harboring a reporter gene operably driven by an LXRE promoter; contacting the cell with the candidate; and determining whether the candidate modulates the expression of the reporter gene in comparison with a cell not contacted with the candidate; whereby a difference detected between the presence and absence of the candidate indicates that the candidate modulates the SIRT1-dependent effect of an LXR.

In a further aspect the invention provides a method for assessing whether a candidate substance modulates the interaction of SIRT1 with an LXR. This method includes transfecting a cell with a vector harboring a sequence encoding an epitope-tagged LXR; further transfecting the cell with a vector harboring a-sequence encoding an SIRT1; contacting the cell with the candidate substance; lysing the cells, contacting the cell lysates with an antibody specific for the epitope tag, and recovering immunoprecipitates comprising a complex of the SIRT1 and the LXR with an antibody-specific adsorbent; carrying out a Western blot procedure using antibodies specific for SIRT1 and an LXR; whereby a difference in complex formation detected in the presence of the candidate compared with the absence of the candidate indicates that the candidate modulates the interaction of the SIRT1 with the LXR.

### BRIEF DESCRIPTION OF THE FIGURES

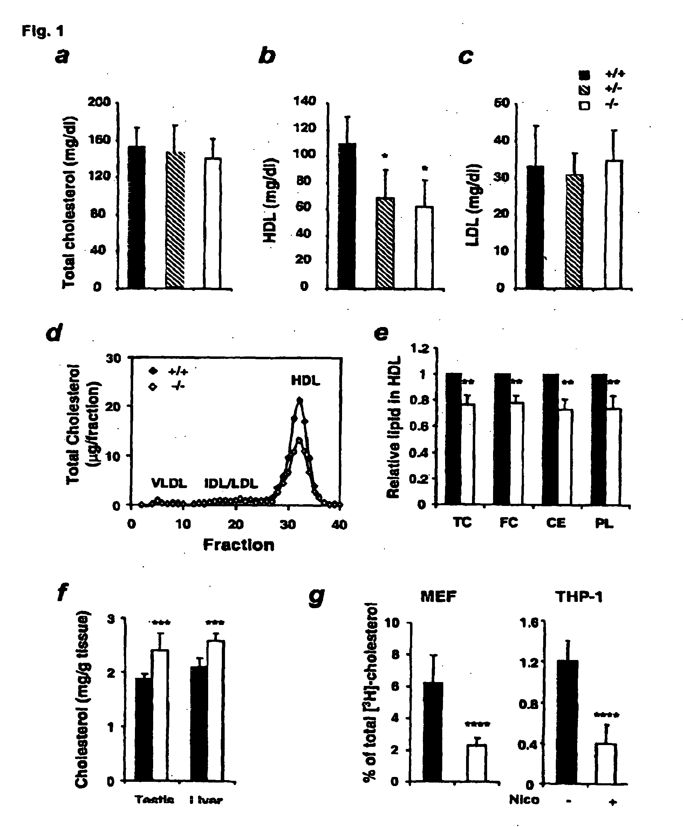

Figure 1. Loss of function of SIRT1 results in altered cholesterol homeostasis.
   (*a-c*) Plasma samples from wildtype (filled bar), *SIRT1*^{*+*/}*⁻* (bar with slanted shading), and *SIRT1*^{*+*/}*⁻* (open bar) mice were analyzed for (*a*) total cholesterol, (*b*) HDL cholesterol, (*c*) LDL cholesterol.
   (*d*) FPLC plasma lipoprotein cholesterol profiles in wildtype (filled diamond) and *SIRT1*^{*-*/}*⁻* (open diamond) mice.
   (*e*) Relative lipid contents in HDL fractions of wildtype (filled bar) and *SIRT1*^{*-*/}*⁻* (open bar) mice.
   (*f*) Total cholesterol from wildtype control (filled bar) and littermate *SIRT1*^{*-*/}*⁻* (open bar) testes and livers.
   (*g*) ApoAI-mediated efflux of [³H]cholesterol from primary mouse embryonic fibroblasts (MEFs) (left panel) or THP-1 human monocytes (right panel), with or without pretreatment with nicotinamide.

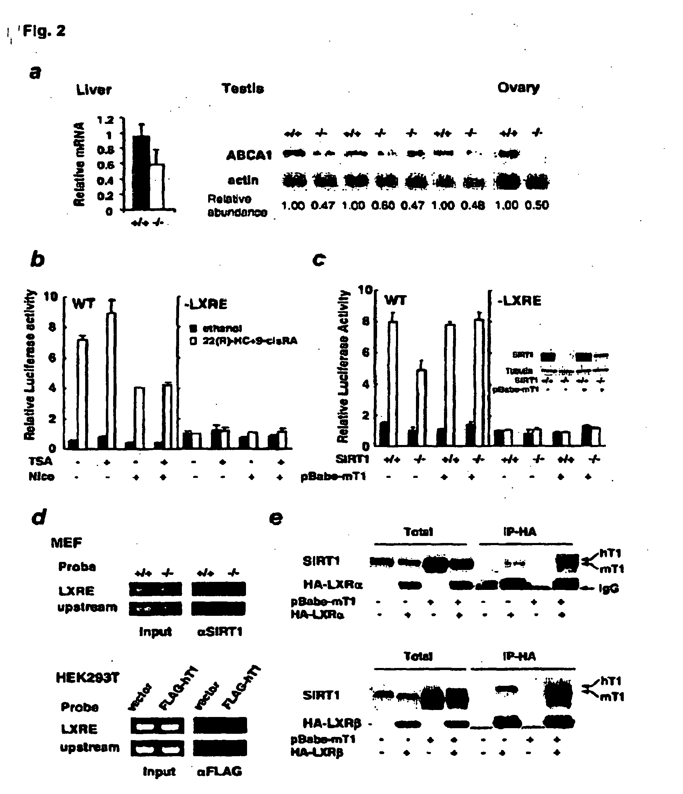

Figure 2. Expression of ABCA1 and interaction with LXR nuclear receptors.
   (*a*) Expression of ABCA1 mRNA in livers (left panel, determined by quantitative real-time PCR), and in testes and ovaries (right panel, determined by northern blots of wildtype (+/+) and *SIRT1*^{*-*/}*⁻* (-/-) animals).
   (*b*) Inhibition of SIRT1 activity reduces ABCA1 promoter activity.
   (*c*) Effect of SIRT1 deficiency on ABCA1 promoter activity in MEFs.
   (*d*) Interaction of SIRT1 with the LXRE of ABCA1 promoter.
   (*e*) SIRT1 interacts with LXRα and LXRβ.

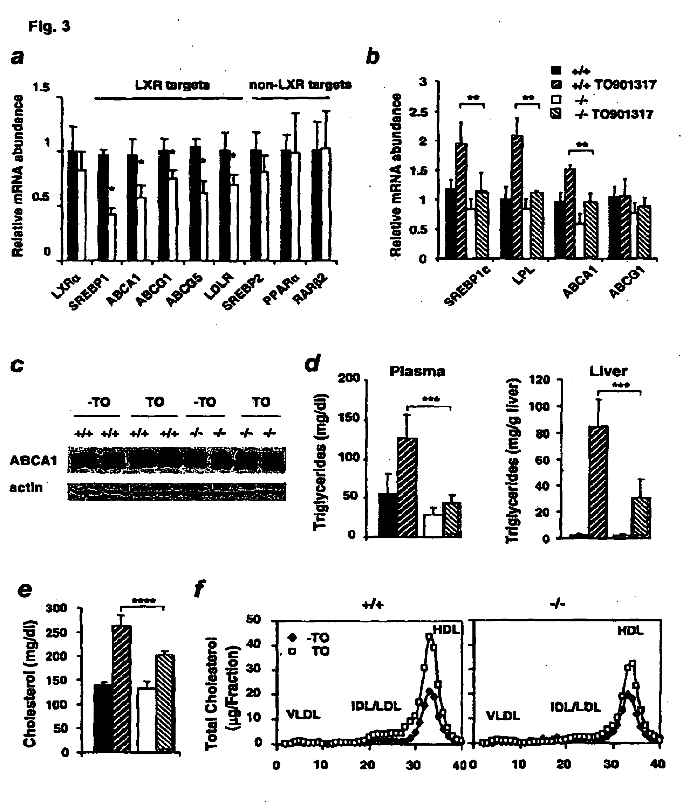

Figure 3. SIRT1 deficiency compromises the normal responses to a LXR agonist *in vivo.*
   (*a*) LXR target mRNAs in livers of *SlRT1*^{*-*/}*⁻* mice.
   (*b*) Relative levels of LXR target mRNAs in livers of mice treated with the LXR agonist T0901317.
   (*c*) ABCA1 protein levels in wildtype and *SIRT1*^{*-*/}*⁻* mice without or with T0901317.
   (*d*) Triglyceride levels after T0901317 administration. in *SIRT1*^{*-*/}*⁻* mice accumulate lower levels of triglycerides in plasma and liver.
   (*e*) Induction of cholesterol in plasma after T0901317 feeding.
   (*f*) Representative FPLC plasma lipoprotein profiles in wildtype and *SIRT1*^{*-*/}*⁻* mice before (filled square) and after (open square) T0901317 feeding.

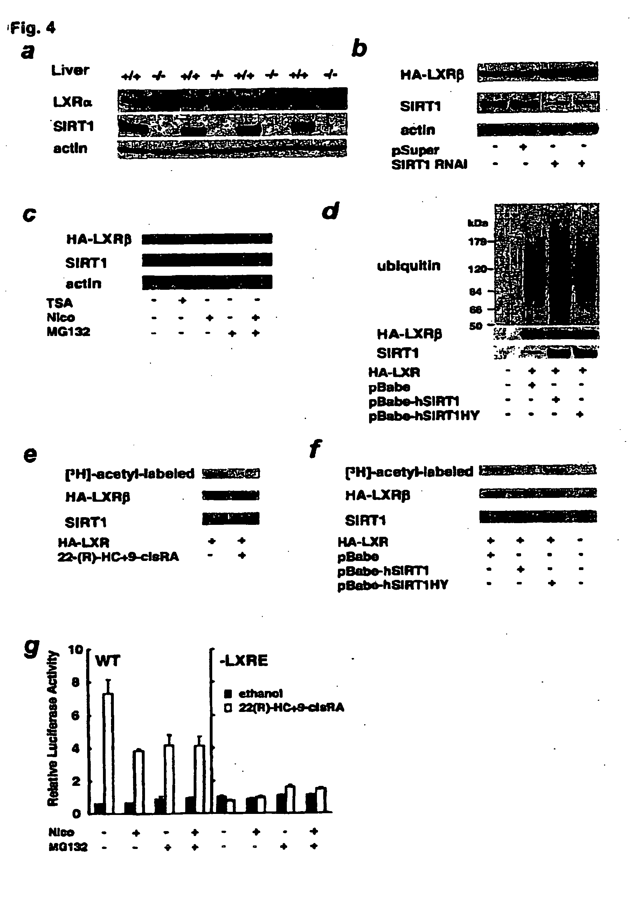

Figure 4. SIRT1 regulates LXR stability and activity by deacetylating the LXR protein.
   (*a*) Effect of SIRT1 on LXR Protein Expression *SIRT1.*
   (*b*) Effect of knockdown of SIRT1 by RNA interference on the level of LXR.
   (*c*) Effect of inhibitors on protein expression of LXR
   (*d*) SIRT1 promotes the ubiquitination of LXR.
   (*e*) Acetylation ofLXR *in vivo* and activation ofLXR by 22(R)-HC and 9-cisRA.
   (*f*) SIRT1 deacetylates HA-LXRβ.

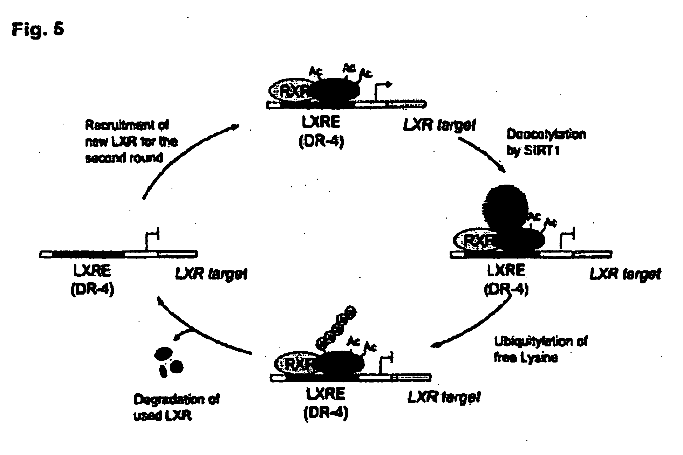

Figure 5. Schematic representation of the effect of inhibition of SIRT1 or proteasome-mediated LXR degradation on the transcription activity ofLXR on the ABCA1 promoter.

### DETAILED DESCRIPTION OF THE INVENTION

### Table of Sequences

**Table 1. Table of Sequences in the Disclosure**

| SEQ ID NO: | IDENTIFICATION | GENBANK ACC. NO. | TYPE |
|---|---|---|---|
| 1 | Human SIRT1 | NP 036370 | Prot |
| 2 | Murine SIRT1 | NP 062786 | Prot |
| 3 | Human Liver X Receptor alpha | NP 062786 | Prot |
| 4 | Human Liver X Receptor beta | NP 009052.3 | Prot |
| 5 | Murine Liver X Receptor alpha. | NP 038867.1 | Prot |
| 6 | Murine Liver X Receptor beta | NP 033499.1 | Prot |
| 7 | Human SIRT1 | NM 012238 | mRNA |
| 8 | Murine SIRT1 | NM 019812 | mRNA |
| 9 | Human liver X receptor alpha | NM 005693 | mRNA |
| 10 | Human liver X receptor beta | NM 007121 | mRNA |
| 11 | Murine liver X receptor alpha | NM 013839 | mRNA |
| 12 | Murine liver X receptor beta | NM 009473 | mRNA |
| 13 | Primer | | DNA |
| 14 | Primer | | DNA |
| 15 | Primer | | DNA |
| 16 | Primer | | DNA |
| 17 | Primer | | DNA |
| 18 | Primer | | DNA |
| 19 | Primer | | DNA |
| 20 | Primer | | DNA |

As used herein, HDL (high-density lipoprotein) relates to a class of plasma lipoprotein with a high proportion of protein, including apolipoproteins A, C, D and E. HDL incorporates and transports cholesterol, whether free or esterified, in the plasma as an HDL-cholesterol complex. The term HDL may be used in a context-dependent manner to designate cholesterol bound to HDL particles.

As used herein, LDL (low-density lipoprotein) relates to a class of plasma lipoprotein with a high proportion of lipid, including cholesterol, cholesterol esters and triglycerides. It includes primarily apolipoprotein B-100 and apolipoprotein E. LDL incorporates and transports cholesterol in the plasma. The term LDL may be used in a context-dependent manner to designate cholesterol bound to LDL particles.

As used herein, the term "isolated" and similar terms relate to a substance that has been purified from a naturally occurring state by the removal of at least one component with which the substance occurs in the natural state. The term "purified" may be used synonymously with "isolated". It is understood that these terms do not require that the substance have any predetermined level of purity.

As used herein, the term "a complex" and similar terms relate to a combination of two or more substances combined with each other sufficiently stably to undergo an isolation procedure and be characterized as being so combined. The components of the complex may be bound directly to each other, or the combination may include an intermediary facilitating component such that the identified components are not directly in contact with one another. A complex may be formed by noncovalent interactions binding the components or by covalent bonds between them.

As used herein the term "cholesterol" generally refers to total occurrence of cholesterol as both cholesterol and cholesteryl esters. In certain contexts which will be apparent to the worker of skill in the art, "cholesterol" may be used herein to refer only to free cholesterol.

As used herein, the term "treatment" and similar terms and phrases relate to the application or administration of a therapeutic agent to a subject having a disease or condition, a symptom of disease, or a predisposition toward a disease, or application or administration of a therapeutic agent to an isolated tissue or cell line from the subject. Treatment is intended to promote curing or healing thereof, or to alleviate, relieve, alter, remedy, ameliorate, improve, or affect the disease, the symptoms of disease, or the predisposition toward disease.

As used herein, the phrases "therapeutically effective amount" and "prophylactically effective amount" refer to an amount that provides a therapeutic benefit in the treatment of a disease, or an effect providing prevention or diminishing the severity of the disease, respectively. The specific amount that is therapeutically effective can be readily determined by an ordinary medical practitioner employing assessment of response in a treated subject, and may vary depending on factors known in the art, such as the nature of the disease, the subject's history and age, the stage of disease, and the administration of other therapeutic agents.

As used herein, a "pharmaceutical composition" relates to a composition that includes a pharmacologically effective amount of a polynucleotide and a pharmaceutically acceptable carrier. As used herein, "pharmacologically effective amount," "therapeutically effective amount" or simply "effective amount" refers to that amount of an inhibitory polynucleotide effective to produce the intended pharmacological, therapeutic or preventive result. For example, if a given clinical treatment is considered effective when there is at least a minimal measurable change in a clinical parameter associated with a disease or disorder, a therapeutically effective amount of a drug for the treatment of that disease or disorder is the amount necessary to effect at least extent of change in the parameter.

The term "pharmaceutically acceptable carrier" refers to a composition for administration of a therapeutic agent that is at least both physiologically acceptable and approvable by a regulatory agency.

### SIRT1 Polypeptides

As used herein, the terms an "SIRT1 polypeptide", an "SIRT1 protein", and related terms and phrases, relate to wild type SIRT1, to a mutant SIRT1, a variant SIRT1, and to biologically active fragments and mature forms thereof. An important SIRT1 protein to be used in the present invention is human SIRT1. The amino acid sequence of human SIRT1 is given in GenBank Acc. No. NP_036370, disclosed as being composed of 747 amino acid residues, is shown in Table 2 using the conventional one-letter amino acid code (International Union Of Biochemistry And Molecular Biology, Recommendations on Biochemical & Organic Nomenclature, Symbols & Terminology etc., Part 1, Section A: Amino-Acid Nomenclature, Section 3AA-1. Names Of Common Alpha-Amino Acids, http://www.chem.qmul.ac.uk/iubmb/ and J. Biol. Chem., 1985, 260,14-42).

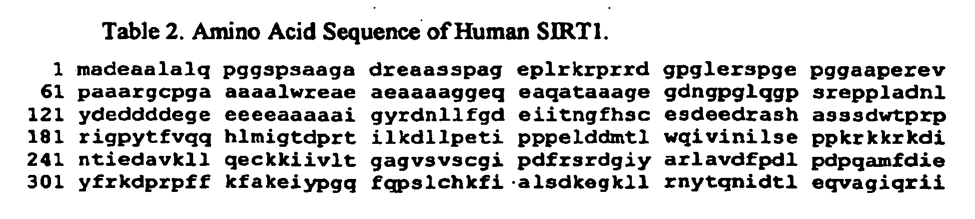

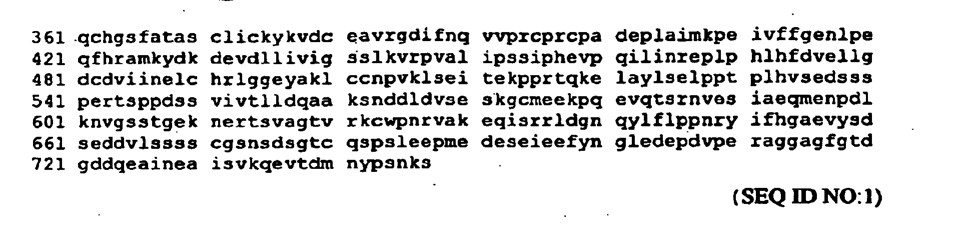

The amino acid sequence of murine SIRT1 is given in GenBank Acc. No. NP_062786, disclosed as being composed of 737 amino acid residues, is shown in Table 3.

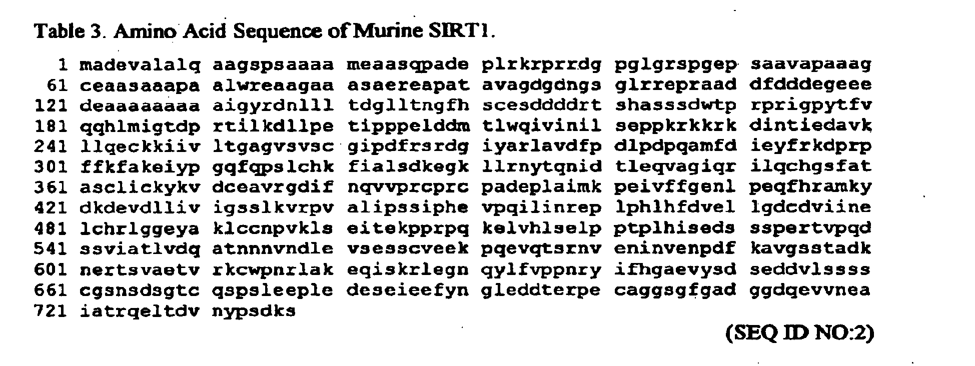

The amino acid sequence of human liver X receptor alpha is given in GenBank Acc. No. NP_062786, disclosed as being composed of 447 amino acid residues, is shown in Table 4.

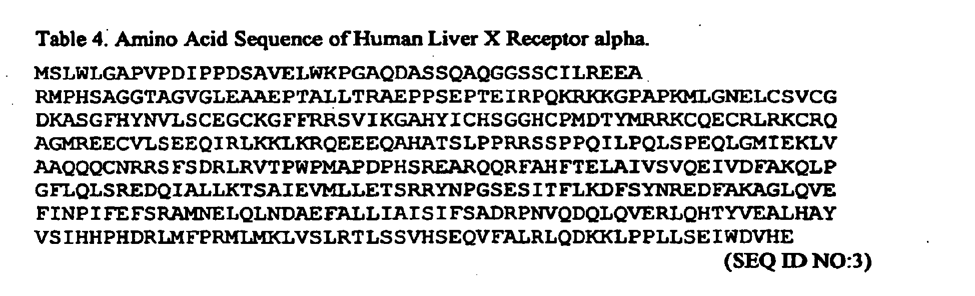

The amino acid sequence of human liver X receptor beta is given in GenBank Acc. No. NP_009052.3, disclosed as being composed of 461 amino acid residues, is shown in Table 5.

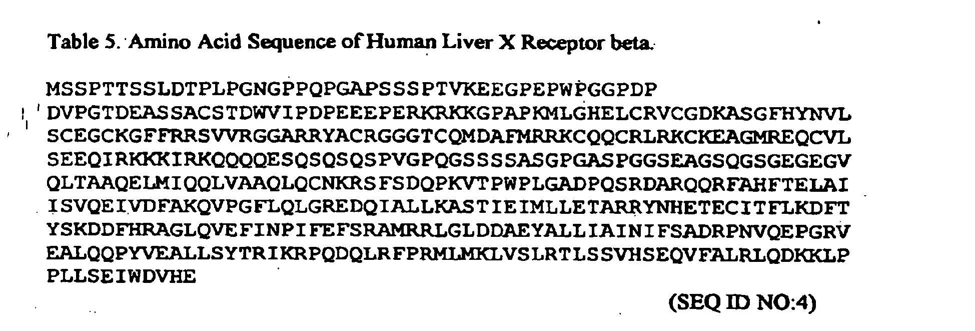

The amino acid sequence of murine liver X receptor alpha is given in GenBank Acc. No., NP_038867.1 disclosed as being composed of 445 amino acid residues, is shown in Table 6.

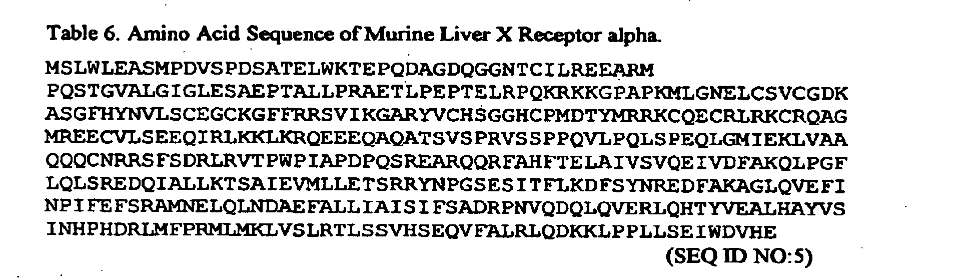

The amino acid sequence of murine liver X receptor beta is given in GenBank Acc. No. NP_033499.1, disclosed as being composed of 446 amino acid residues, is shown in Table 7.

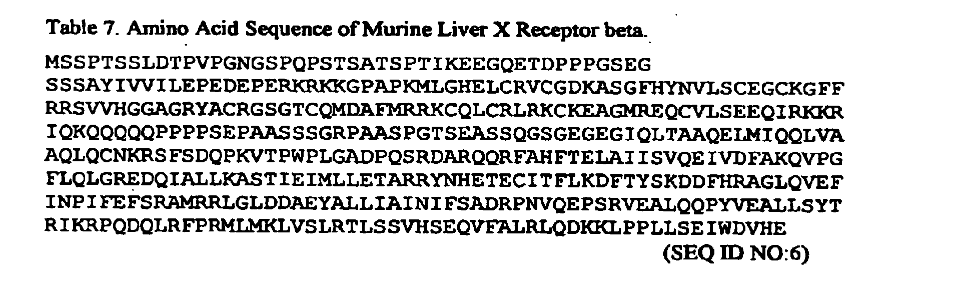

In general, an "SIRT1 polypeptide" and various "LXR polypeptides" as employed in the methods and compositions of the present invention, includes wild type human SIRT1 and LXR polypeptides or wild type murine SIRT1 and murine LXR polypeptides such as shown in Tables 2-7, as well as wild type vertebrate orthologs thereof, and domains, motifs and fragments thereof. In addition, an these terms include recombinant mutant polypeptides, domains, motifs and fragments in which at least one amino acid residue has been changed to a different amino acid residue; or one or more residues may be deleted; or one or more residues may be inserted between neighboring residues in an original sequence. A mutant or variant SIRT1 polypeptide may have from 1 amino acid residue up to 1% of the residues changed, or up to 2%, or up to 5% or up to 8%, or up to 10%, or up to 15%, or up to 20%, or somewhat higher percent, of the residues changed from a wild type or reference sequence. The recombinant mutant or variant polypeptides, domains, motifs and fragments of SIRT1 are used in the present methods and compositions as long as they demonstrably exhibit at least one biological activity or function of wild type SIRT1 and LXRs. Possession of a biological activity or function may be determined by a worker of skill in the fields related to the present invention, including, by way of nonlimiting example, molecular biology, cell biology, pathology, clinical medicine and the like. Such workers of skill in the fields of the invention may assay recombinant mutant SIRT1 and LXR polypeptides, domains, motifs and fragments at least by methods described in the Examples of the present invention.

It will be recognized in the art that an amino acid sequence of an SIRT1 or LXR polypeptide can be varied without significant effect on the structure or function of the protein. If such differences in sequence are contemplated, it should be remembered that there will be certain areas on the protein that are important for its activity. In general, it is possible to replace residues that form the tertiary structure, provided that residues providing a similar function are used. In other instances, the type of residue may be completely unimportant if the alteration occurs at a non-important region of the protein.

### Nucleic Acids

As used herein, the term "SIRT1 polynucleotide" or "SIRT1 nucleic acid", or "LXR polynucleotide" or "LXR nucleic acid" or related terms and phrases, relates to any polynucleotide that encodes any SIRT1 polypeptide as described herein. In general, any nucleotide sequence that encodes an SIRT1 polypeptide described above is encompassed within the present invention. In some embodiments, a nucleic acid encoding a polypeptide having the amino acid sequence of a human SIRT1 shown in Table 2 includes a coding sequence of the mRNA nucleic acid sequence disclosed in GenBank Acc. No. NM_012238, shown in Table 8, or a fragment thereof. In Table 8, the coding sequence extends from position 54 to position 2297.

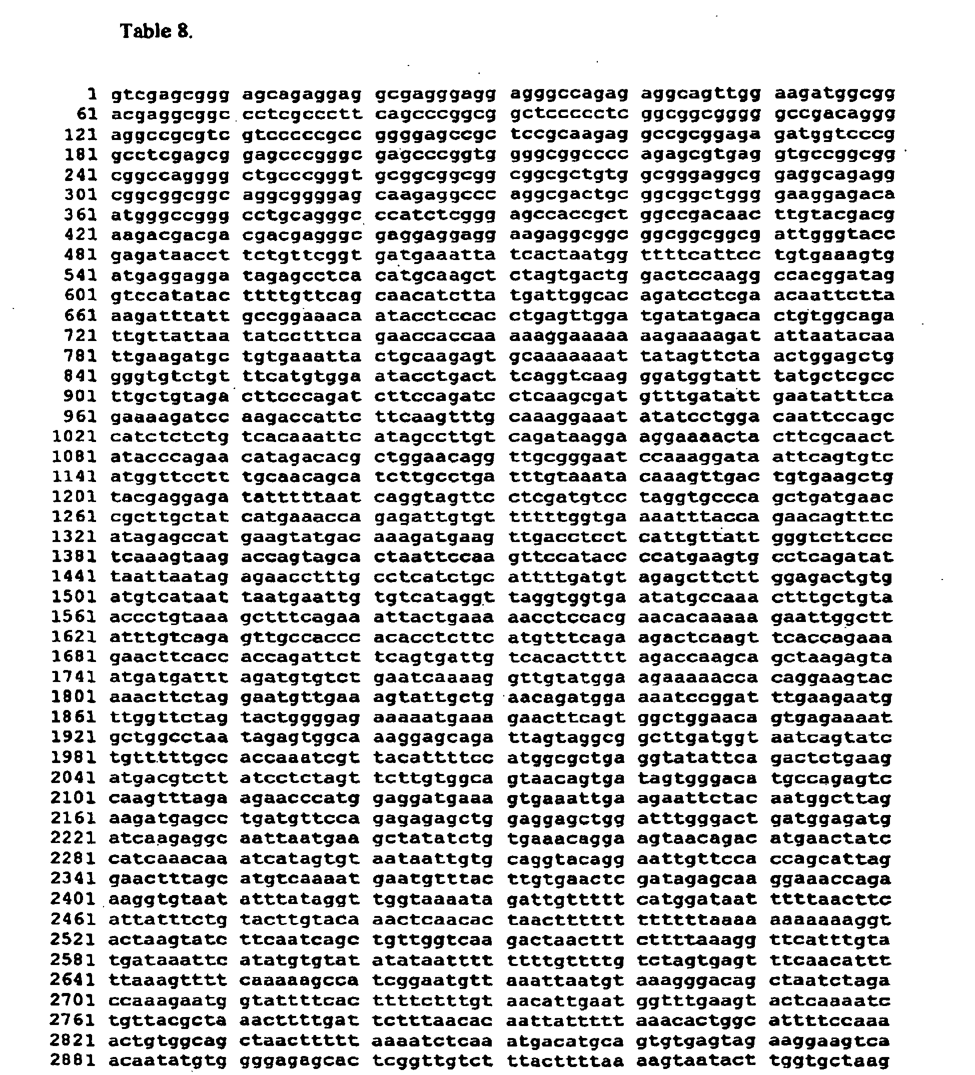

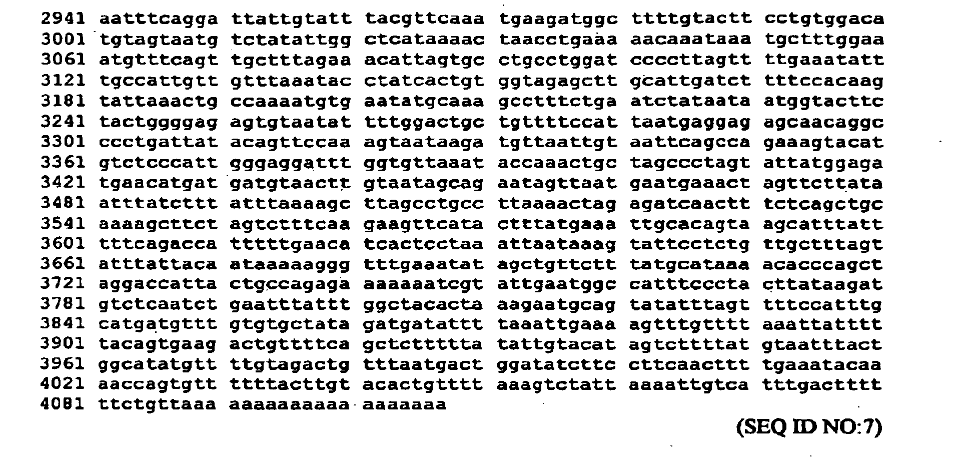

In some embodiments, a nucleic acid encoding a polypeptide having the amino acid sequence of a murine SIRTI shown in Table 3 includes a coding sequence of the mRNA nucleic acid sequence disclosed in GenBank Acc. No. NM_019812, shown in Table 9, or a fragment thereof. In Table 9, the coding sequence extends from position 48 to position 2261.

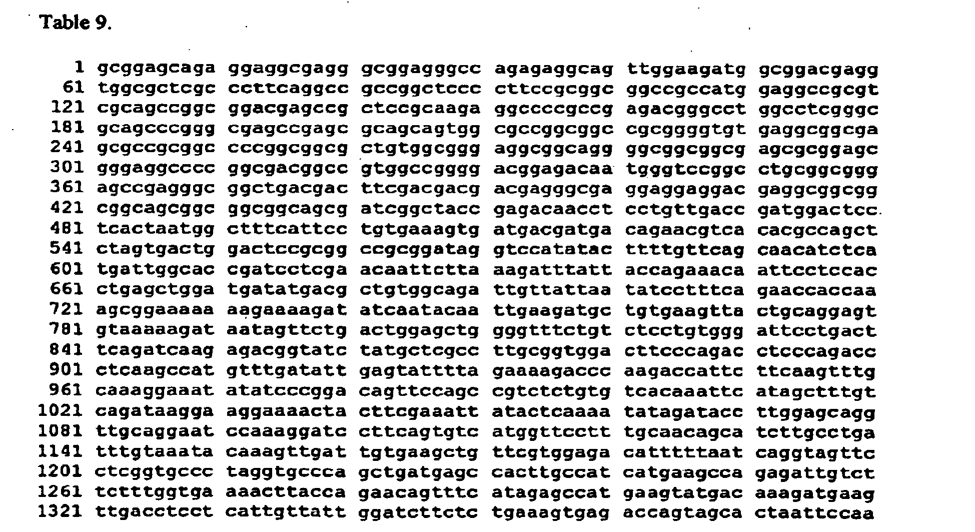

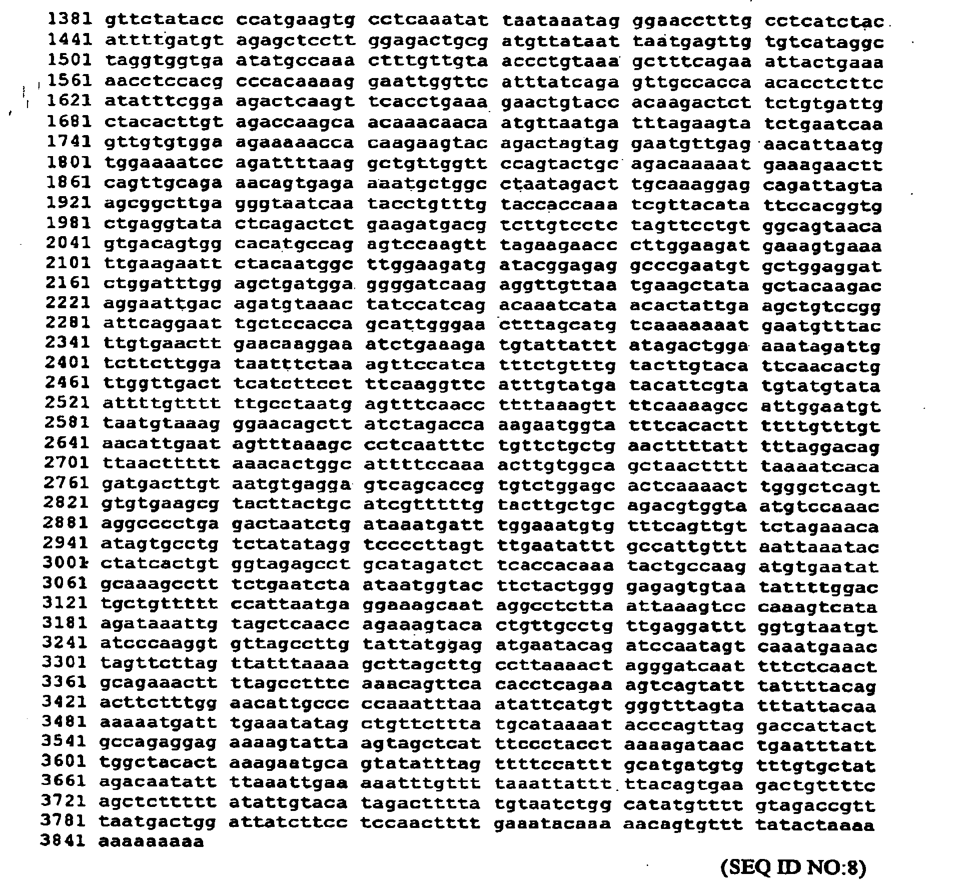

In some embodiments, a nucleic acid encoding a polypeptide having the amino acid sequence of a human liver X receptor alpha shown in Table 4 includes a coding sequence of the mRNA nucleic acid sequence disclosed in GenBank Acc. No. NM_005693, or a fragment thereof In Table 10, the coding sequence extends from position 36 to position 1379.

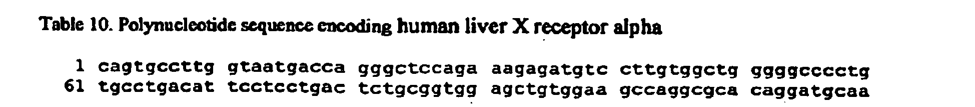

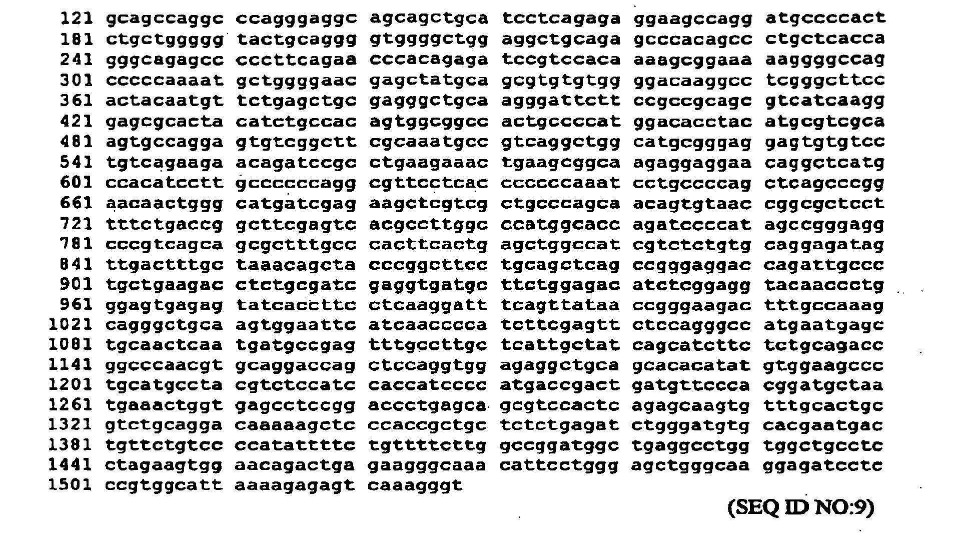

In some embodiments, a nucleic acid encoding a polypeptide having the amino acid sequence of a human liver X receptor beta shown in Table 5 includes a coding sequence of the mRNA nucleic acid sequence disclosed in GenBank Acc. No. NM_007121, or a fragment thereof. In Table 11, the coding sequence extends from position 259 to position 1644.

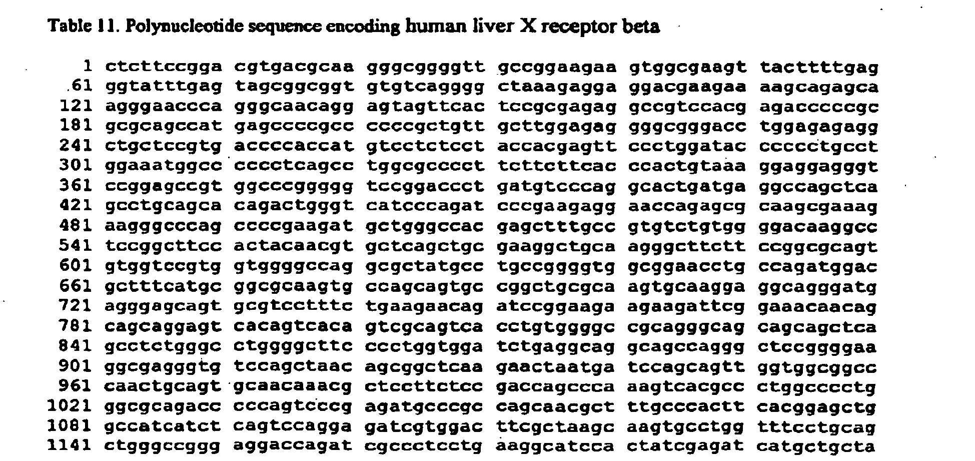

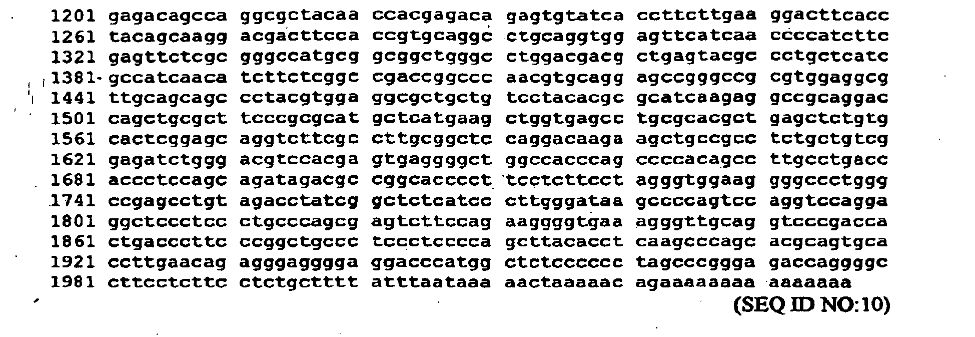

In some embodiments, a nucleic acid encoding a polypeptide having the amino acid sequence of a murine liver X receptor alpha shown in Table 6 includes a coding sequence of the mRNA nucleic acid sequence disclosed in GenBank Acc. No.NM_013839, or a fragment thereof In Table 12, the coding sequence extends from position 190 to position 1527.

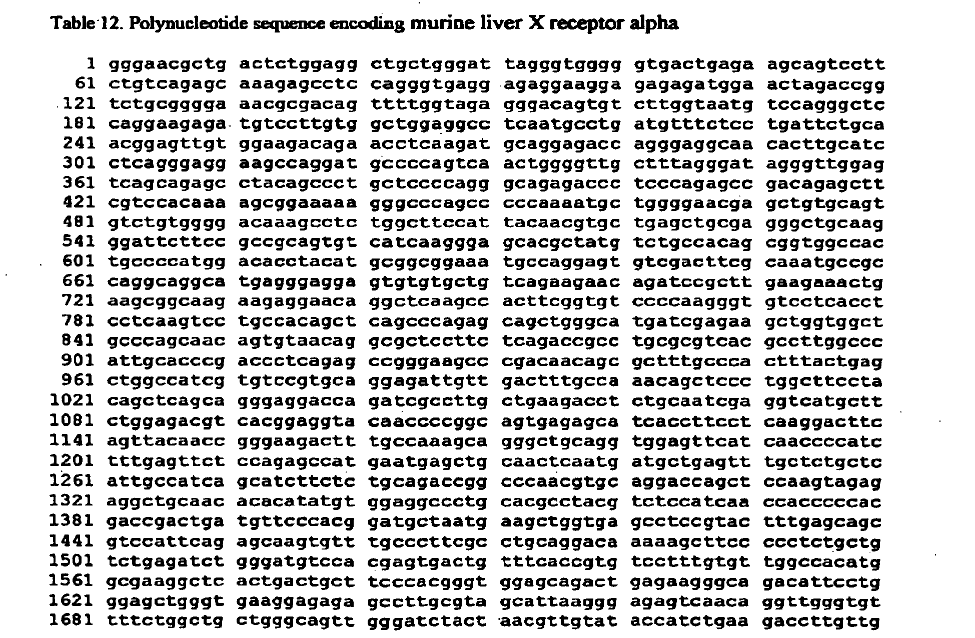

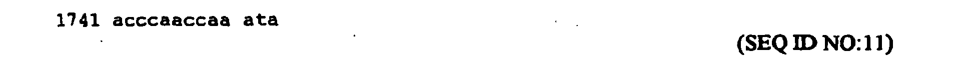

In some embodiments, a nucleic acid encoding a polypeptide having the amino acid sequence of a murine liver X receptor beta shown in Table 7 includes a coding sequence of the mRNA nucleic acid sequence disclosed in GenBank Acc. No. NM_009473, or a fragment thereof In Table 13, the coding sequence extends from position 271 to position 1611.

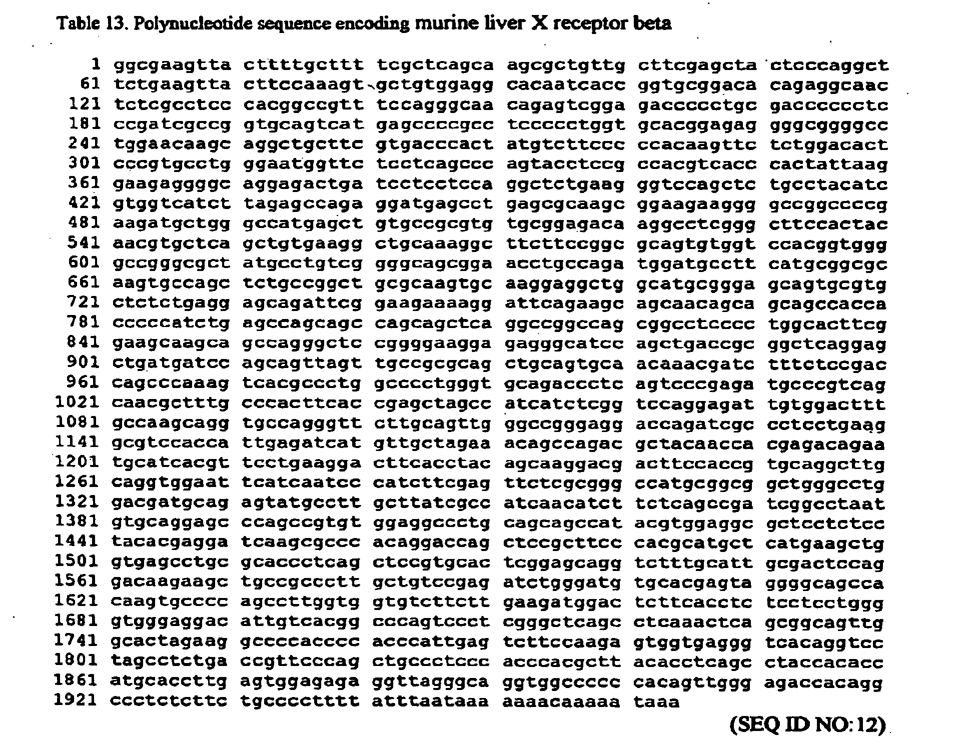

Additionally, the invention includes polynucleotides that are mutant or variant nucleic acids of the sequences shown in Tables 8-13, or a fragment thereof, any of whose bases may be changed from the disclosed sequence while still encoding a polypeptide that maintains its SIRT1 protein-like activities and physiological functions. An SIRT1 mutant or variant polynucleotide encodes a mutant or variant SIRT1 or LXR polypeptide that may have from 1 amino acid residue up to 1% of the residues changed, or up to 2%, or up to 5%, or up to 8%, or up to 10%, or up to 15%, or up to 20%, or somewhat higher percent, of the residues change from a wild type or reference sequence. By "nucleic acid" or "polynucleotide" is meant a DNA, an RNA, a DNA or RNA including one or more modified nucleotides or modified pentose phosphate backbone structures, a polypeptide-nucleic acid, and similar constructs that preserve the coding properties of the sequence of bases included in the construct.

Also disclosed are nucleic acid fragments. A nucleic acid fragment may encode a fragment of an SIRT1 or LXR polypeptide. In addition SIRT1 or LXR nucleic fragments may be used as hybridization probes to identify SIRT1 or LXR protein-encoding nucleic acids (e.g., SIRT1 or LXR mRNA) and fragments for use as polymerase chain reaction (PCR) primers for the amplification or mutation of SIRT1 or LXR nucleic acid molecules. As used herein, the term "nucleic acid molecule" is intended to include DNA molecules (e.g., cDNA or genomic DNA), RNA molecules (e.g., mRNA), analogs of the DNA or RNA generated using nucleotide analogs, and derivatives, fragments and homologs thereof. The nucleic acid molecule can be single-stranded or double-stranded, but preferably is double-stranded DNA.

### Nucleic Acids

As used herein, a "nucleic acid" or "polynucleotide", and similar terms and phrases, relate to polymers composed of naturally occurring nucleotides as well as to polymers composed of synthetic or modified nucleotides. Thus, as used herein, a polynucleotide that is a RNA, or a polynucleotide that is a DNA, or a polynucleotide that contains both deoxyribonucleotides and ribonucleotides, may include naturally occurring moieties such as the naturally occurring bases and ribose or deoxyribose rings, or they may be composed of synthetic or modified moieties such as those described below. A polynucleotide employed in the invention may be single stranded or it may be a base paired double stranded structure, or even a triple stranded base paired structure.

Nucleic acids and polynucleotides may be 20 or more nucleotides in length, or 30 or more nucleotides in length, or 50 or more nucleotides in length, or 100 or more, or 1000 or more, or tens of thousands or more, or hundreds of thousands or more, in length. As used herein, "oligonucleotides" and similar terms based on this relate to short polymers composed of naturally occurring nucleotides as well as to polymers composed of synthetic or modified nucleotides, as described in the immediately preceding paragraph. Oligonucleotides may be 10 or more nucleotides in length, or 15, or 16, or 17, or 18, or 19, or 20 or more nucleotides in length, or 21, or 22, or 23, or 24 or more nucleotides in length, or 25, or 26, or 27, or 28 or 29, or 30 or more nucleotides in length, 35 or more, 40 or more, 45 or more, up to about 50, nucleotides in length. Oligonucleotides may be chemically synthesized and may be used as siRNAs, PCR primers, or probes.

It is understood that, because of the overlap in size ranges provided in the preceding paragraph, the terms "polynucleotide" and "oligonucleotide" may be used synonymously herein.

As used herein "nucleotide sequence", "oligonucleotide sequence" or "polynucleotide sequence", and similar terms, relate interchangeably both to the sequence of bases that an oligonucleotide or polynucleotide has, as well as to the oligonucleotide or polynucleotide structure possessing the sequence. A nucleotide sequence or a polynucleotide sequence furthermore relates to any natural or synthetic polynucleotide or oligonucleotide in which the sequence of bases is defined by description or recitation of a particular sequence of letters designating bases as conventionally employed in the field.

A "nucleoside" is conventionally understood by workers of skill in fields such as biochemistry, molecular biology, genomics, and similar fields related to the field of the invention as comprising a monosaccharide linked in glycosidic linkage to a purine or pyrimidine base; and a "nucleotide" comprises a nucleoside with at least one phosphate group appended, typically at a 3' or a 5' position (for pentoses) of the saccharide, but may be at other positions of the saccharide. Nucleotide residues occupy sequential positions in an oligonucleotide or a polynucleotide. A modification or derivative of a nucleotide may occur at any sequential position in an oligonucleotide or a polynucleotide. Modifications or derivatives can occur in the phosphate group, the monosaccharide or the base.

By way of nonlimiting examples, the following descriptions provide certain modified or derivatized nucleotides. The monosaccharide may be modified by being, for example, a pentose or a hexose other than a ribose or a deoxyribose. The monosaccharide may also be modified by substituting hydryoxyl groups with hydro or amino groups, by alkylating or esterifying additional hydroxyl groups, and so on. Substituents at the 2' position, such as 2'-O-methyl, 2'-O-ethyl, 2'-O-propyl, 2'-O-allyl, 2'-O-aminoalkyl or 2'-deoxy-2'-fluoro group provide enhanced hybridization properties to an oligonucleotide.

The bases in oligonucleotides and polynucleotides may be "unmodified" or "natural" bases include the purine bases adenine (A) and guanine (G), and the pyrimidine bases thymine (T), cytosine (C) and uracil (U). In addition they may be bases with modifications or substitutions. Nonlimiting examples of modified bases include other synthetic and natural bases such as hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine, 5-hydroxymethyl cytosine, xanthine, hypoxanthine, 2-aminoadenine, 6-methyl and other alkyl derivatives of adenine and guanine, and 2-propyl and other alkyl derivatives of adenine and guanine

The linkages between nucleotides is commonly the 3'-5' phosphate linkage, which may be a natural phosphodiester linkage, a phosphothioester linkage, and still other synthetic linkages. Oligonucleotides containing phosphorothioate backbones have enhanced nuclease stability. Nonlimiting examples of modified backbones include, phosphorothioates, chiral phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and boranophosphates

Use of any modified nucleotide is equivalent to use of a naturally occurring nucleotide having the same base-pairing properties, as understood by a worker of skill in the art.

An "isolated" nucleic acid molecule is one that is separated from other nucleic acid molecules that are present in the natural source of the nucleic acid. Examples of isolated nucleic acid molecules include, but are not limited to, recombinant DNA molecules contained in a vector, recombinant DNA molecules maintained in a heterologous host cell, partially or substantially purified nucleic acid molecules, and synthetic DNA or RNA molecules.

A nucleic acid molecule disclosed herein, e.g., a nucleic acid molecule having the nucleotide sequence of Table 8-13, or a complement thereof, can be isolated using standard molecular biology techniques and the sequence information provided herein. Using all or a portion of the nucleic acid sequence of Table 8-13 as a hybridization probe, SIRT1 or LXR nucleic acid sequences can be isolated using standard hybridization and cloning techniques (*e.g.,* as described in Sambrook et al., eds., MOLECULAR CLONING: A LABORATORY MANUAL 2nd Ed., Cold Spring Harbor Laboratory Press, Cold Spring Harbor, NY, 1989; and Ausubel, et al., eds., CURRENT PROTOCOLS IN MOLECULAR BIOLOGY, John Wiley & Sons, New York, NY, 1993.)

A nucleic acid disclosed in the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to SIRT1 or LXR nucleotide sequences can be prepared by standard synthetic techniques, e.g., using an automated DNA synthesizer.

### Synthesis of Polynucleotides

Oligonucleotides and polynucleotides can be prepared by standard synthetic techniques, e.g., using an automated DNA synthesizer. Methods for synthesizing oligonucleotides include well-known chemical processes, including, but not limited to, sequential addition of nucleotide phosphoramidites onto surface-derivatized particles, as described by T. Brown and Dorcas J. S. Brown in Oligonucleotides and Analogues A Practical Approach, F. Eckstein, editor, Oxford University Press, Oxford, pp. 1-24 (1991), and incorporated herein by reference.

An example of a synthetic procedure uses Expedite RNA phosphoramidites and thymidine phosphoramidite (Proligo, Germany). Synthetic oligonucleotides are deprotected and gel-purified (Elbashir et al. (2001) Genes & Dev. 15, 188-200), followed by Sep-Pak C18 cartridge (Waters, Milford, Mass., USA) purification (Tuschl et al. (1993) Biochemistry, 32:11658-11668). Complementary ssRNAs are incubated in an annealing buffer (100 mM potassium acetate, 30 mM HEPES-KOH at pH 7.4, 2 mM magnesium acetate) for 1 min at 90° C followed by 1 h at 37° C to hybridize to the corresponding ds-polynucleotides.

Other methods of oligonucleotide synthesis include, but are not limited to solid-phase oligonucleotide synthesis according to the phosphotriester and phosphodiester methods (Narang, et al., (1979) Meth. Enzymol. 68:90), and to the H-phosphonate method (Garegg, P. J., et al., (1985) "Formation of internucleotidic bonds via phosphonate intermediate", Chem. Scripta 25, 280-282; and Froehler, B. C., et al., (1986a) "Synthesis of DNA via deoxynucleoside H-phosphonate intermediates", Nucleic Acid Res., 14, 5399-5407, among others) and synthesis on a support (Beaucage, et al. (1981) Tetrahedron Letters 22:1859-1862) as well as phosphoramidate techniques (Caruthers, M. H., et al., "Methods in Enzymology," Vol. 154, pp. 287-314 (1988), U. S. Patent 5,153,319, 5,132,418, 4,500,707, 4,458,066, 4,973,679, 4,668,777, and 4,415,732, and others described in "Synthesis and Applications of DNA and RNA," S. A. Narang, editor, Academic Press, New York, 1987, and the references contained therein, and nonphosphoramidite techniques. Solid phase synthesis helps isolate the oligonucleotide from impurities and excess reagents. Once cleaved from the solid support the oligonucleotide may be further isolated by known techniques.

### Vectors

The present disclosure provides various vectors that contain one or more polynucleotides. Advantageously any vector disclosed includes a promoter, an enhancer, or both, operably linked to the nucleotide sequence.

Methods for preparing the vectors disclosed are widely known in the fields of molecular biology, cell biology, oncology and related fields of medicine, and other fields related to the present invention. Methods useful for preparing the vectors are described, by way on nonlimiting example, in Molecular Cloning: A Laboratory Manual (3rd Edition) (Sambrook, J et al. (2001) Cold Spring Harbor Laboratory Press, Cold Spring Harbor, NY), and Short protocols in molecular biology (5th Ed.) (Ausubel FM et al. (2002) John Wiley & Sons, New York City).

### Antibodies

The term "antibody" as used herein refers to immunoglobulin molecules and immunologically active portions of immunoglobulin (Ig) molecules, i.e., molecules that contain an antigen binding site that specifically binds (immunoreacts with) an antigen. Such antibodies include, but are not limited to, polyclonal, monoclonal, chimeric, single chain, F_{ab}, F_{ab'} and F(_{ab'})₂ fragments, and an F_{ab} expression library. In general, antibody molecules obtained from humans relates to any of the classes IgG, IgM, IgA, IgE and IgD, which differ from one another by the nature of the heavy chain present in the molecule. Certain classes have subclasses as well, such as IgG₁, IgG₂. and others. Furthermore, in humans, the light chain may be a kappa chain or a lambda chain. Reference herein to antibodies includes a reference to all such classes, subclasses and types of human antibody species. Any antibody disclosed herein binds "immunospecifically" to its cognate antigen. By immunospecific binding is meant that an antibody raised by challenging a host with a particular immunogen binds to a molecule such as an antigen that includes the immunogenic moiety with a high affinity, and binds with only a weak affinity or not at all to non-immunogen-containing molecules. As used in this definition, high affinity means having a dissociation constant less than about 1 x 10⁻⁶ M, and weak affinity means having a dissociation constant higher than about 1 x 10⁻⁶ M.

An isolated protein, or a complex containing such a protein, intended to serve as an antigen, or a portion or fragment thereof, can be used as an immunogen to generate antibodies that immunospecifically bind the antigen, using standard techniques for polyclonal and monoclonal antibody preparation. The full-length protein can be used or, alternatively, the invention provides antigenic peptide fragments of the antigen for use as immunogens. An antigenic peptide fragment comprises at least 6 amino acid residues of the amino acid sequence of the full length protein, such as an amino acid sequence shown in Tables 2-7, and encompasses an epitope thereof such that an antibody raised against the peptide forms a specific immune complex with the full length protein or with any fragment that contains the epitope. Preferably, the antigenic peptide comprises at least 10 amino acid residues, or at least 15 amino acid residues, or at least 20 amino acid residues, or at least 30 amino acid residues. Preferred epitopes encompassed by the antigenic peptide are regions of the protein that are located on its surface; commonly these are hydrophilic regions.

In certain embodiments of the invention, at least one epitope encompassed by the antigenic peptide is a region of the SIRT1 or LXR protein that is located on the surface of the protein, *e.g*., a hydrophilic region. A hydrophobicity analysis of the human SIRTI or LXR protein sequence will indicate which regions of a growth promoting polypeptide are particularly hydrophilic and, therefore, are likely to encode surface residues useful for targeting antibody production. As a means for targeting antibody production, hydropathy plots showing regions of hydrophilicity and hydrophobicity may be generated by any method well known in the art, including, for example, the Kyte Doolittle or the Hopp Woods methods, either with or without Fourier transformation. See, *e.g*., Hopp and Woods, 1981, Proc. Nat. Acad Sci. USA 78: 3824-3828; Kyte and Doolittle 1982, J. Mol. Biol. 157: 105-142. Antibodies that are specific for one or more domains within an antigenic protein, or derivatives, fragments, analogs or homologs thereof, are also provided herein.

A protein of the invention, or a derivative, fragment, analog, homolog or ortholog thereof, may be utilized as an immunogen in the generation of antibodies that immunospecifically bind these protein components.

Various procedures known within the art may be used for the production of polyclonal or monoclonal antibodies directed against a protein; or against derivatives, fragments, analogs homologs or orthologs thereof (see, for example, Antibodies: A Laboratory Manual, Harlow E, and Lane D, 1988, Cold Spring Harbor Laboratory Press, Cold Spring Harbor, NY, incorporated herein by reference). Some of these antibodies are discussed below.

### Polyclonal Antibodies

For the production of polyclonal antibodies, various suitable host animals (e.g., rabbit, goat, mouse or other mammal) may be immunized by one or more injections with the native protein, a synthetic variant thereof, or a derivative of the foregoing. An appropriate immunogenic preparation can contain, for example, the naturally occurring immunogenic protein, a chemically synthesized polypeptide representing the immunogenic protein, or a recombinantly expressed immunogenic protein. Furthermore, the protein may be conjugated to a second protein known to be immunogenic in the mammal being immunized. Examples of such immunogenic proteins include but are not limited to keyhole limpet hemocyanin, serum albumin, bovine thyroglobulin, and soybean trypsin inhibitor. The preparation can further include an adjuvant.

The polyclonal antibody molecules directed against the immunogenic protein can be isolated from the mammal (e.g., from the blood) and further purified by well known techniques, such as affinity chromatography using protein A or protein G, or on a column of the antigen. Purification of immunoglobulins is discussed, for example, by D. Wilkinson (The Scientist, published by The Scientist, Inc., Philadelphia PA, Vol. 14, No. 8 (April 17, 2000), pp. 25-28).

### Monoclonal Antibodies

Monoclonal antibodies can be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes can be immunized in vitro.

The immunizing agent will typically include the protein antigen, a fragment thereof or a fusion protein thereof. The lymphocytes are then fused with an immortalized cell line using a suitable fusing agent, such as polyethylene glycol, to form a hybridoma cell [Coding, Monoclonal Antibodies: Principles and Practice, Academic Press, (1986) pp. 59-103].

The culture medium in which the hybridoma cells are cultured can then be assayed for the presence of monoclonal antibodies directed against the antigen. After the desired hybridoma cells are identified, the clones can be subcloned by limiting dilution procedures and grown by standard methods (Goding,1986). Suitable culture media for this purpose include, for example, Dulbecco's Modified Eagle's Medium and RPMI-1640 medium. Alternatively, the hybridoma cells can be grown in vivo as ascites in a mammal.

The monoclonal antibodies secreted by the subclones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

### Recombinant Vectors and Host Cells

Described herein are methods of promoting ABCA1-mediated cholesterol efflux from a mammalian cell that include the introduction into the cell a nucleic acid that contains a sequence encoding a protein deacetylase. The mammalian cell may be a human cell. Variously the protein deacetylase may be a eukaryotic Sir2 protein, or a mammalian SIRT1 protein. Techniques for transacting mammalian cells with intended nucleic acid sequences are widely known to workers of skill in the field of the invention. Certain nonlimiting examples of such methods are described in detail in the following

One aspect disclosed pertains to vectors, preferably expression vectors, containing a nucleic acid encoding a protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g*., bacterial vectors having a bacterial origin of replication and episomal mammalian vectors). Other vectors (*e.g*., nonepisomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g*., replication defective retroviruses, adenoviruses and adeno-associated viruses), which serve equivalent functions.

The recombinant expression vectors disclosed comprise a nucleic acid in a form suitable for expression of the nucleic acid in a host cell, which means that the recombinant expression vectors include one or more regulatory sequences, selected on the basis of the host cells to be used for expression, that is operatively linked to the nucleic acid sequence to be expressed. Within a recombinant expression vector, "operably linked" is intended to mean that the nucleotide sequence of interest is linked to the regulatory sequence(s) in a manner that allows for expression of the nucleotide sequence *(e.g.,* in an *in vitro* transcription/translation system or in a host cell when the vector is introduced into the host cell). The term "regulatory sequence" is intended to includes promoters, enhancers and other expression control elements (*e.g*., polyadenylation signals). Such regulatory sequences are described, for example, in Goeddel; GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e.g*., tissue-specific regulatory sequences). It will be appreciated by those skilled in the art that the design of the expression vector can depend on such factors as the choice of the host cell to be transformed, the level of expression of protein desired, etc. The expression vectors can be introduced into host cells to thereby produce proteins or peptides, including fusion proteins or peptides, encoded by nucleic acids as described herein (*e.g*., SIRT1 or LXR proteins, mutant forms of the SIRT1 or LXR protein, fusion proteins, etc.).

The recombinant expression vectors can be designed for expression of the SIRT1 or LXR protein in prokaryotic or eukaryotic cells. For example, the SIRT1 or LXR protein can be expressed in bacterial cells such as *E. coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro,* for example using T7 promoter regulatory sequences and T7 polymerase.

Expression of proteins in prokaryotes is most often carried out in E. coli with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (1) to increase expression of recombinant protein; (2) to increase the solubility of the recombinant protein; and (3) to aid in the purification of the recombinant protein by acting as a ligand in affinity purification. Typical fusion expression vectors include pGEX (Pharmacia Biotech Inc; Smith and Johnson (1988) Gene 67:31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion E. coli expression vectors include pTrc (Amrann et al, (1988) Gene 69:301-315) and pET 11d (Studier et al., GENE EXPRESSION TECHNOLOGY: METHOD IN ENZYMOLOGY 185, Academic Press, San Diego, Calif. (1990) 60-89).

In another embodiment, the proteins disclosed in the invention are expressed using a yeast expression vector Examples of vectors for expression in yeast S. cerevisiae include pYepSec1 (Baldari, et al., (1987) EMBO J 6:229-234), pMFa (Kurjan and Herskowitz, (1982) Cell 30:933-943), pJRY88 (Schultz et al., (1987) Gene 54:113-123), pYES2 (Invitrogen Corporation, San Diego, Calif), and picZ (Invitrogen Corp, San Diego, Calif.).

Alternatively, the proteins disclosed in the complexes of the invention can be expressed in insect cells using baculovirus expression vectors. Baculovirus vectors available for expression of proteins in cultured insect cells (e.g., SF9 cells) include the pAc series (Smith et aL (1983) Mol Cell Biol 3:2156-2165) and the pVL series (Lucklow and Summers (1989) Virology 170:31-39).

In yet another embodiment, a nucleic acid is expressed in mammalian cells using a mammalian expression vector Examples of mammalian expression vectors include pCDM8 (Seed (1987) Nature 329:840) and pMT2PC (Kaufman et al. (1987) EMBO J6:187-195). When used in mammalian cells, the expression vector's control functions are often provided by viral regulatory elements For example, commonly used promoters are derived from polyoma, Adenovirus 2, cytomegalovirus and Simian Virus 40. For other suitable expression systems for both prokaryotic and eukaryotic cells. See, *e.g*., Chapters 16 and 17 of Sambrook et al., MOLECULAR CLONING. A LABORATORY MANUAL. 2nd ed., Cold Spring Harbor Laboratory, Cold Spring Harbor Laboratory Press. Cold Spring Harbor, N.Y., 1989.

In another embodiment, the recombinant mammalian expression vector is capable of directing expression of the nucleic acid preferentially in a particular cell type (*e.g*., tissuespecific regulatory elements are used to express the nucleic acid). Tissue-specific regulatory elements are known in the art. Non-limiting examples of suitable tissue-specific promoters include the albumin promoter (liver-specific; Pinkert et al. (1987) Genes Dev 1:268-277), lymphoid-specific promoters (Calame and Eaton (1988) Adv Immunol 43:235-275), in particular promoters ofT cell receptors (Winoto and Baltimore (1989) EMBO J 8:729-733) and immunoglobulins (Banerji et al. (1983) Cell 33:729-740; Queen and Baltimore (1983) Cell 33:741-748), neuron-specific promoters (*e.g.,* the neurofilament promoter; Byrne and Ruddle (1989) PNAS 86:5473-5477), pancreas-specific promoters (Edlund et al. (1985) Science 230:912-916), and mammary gland-specific promoters (*e.g*., milk whey promoter, U.S. Pat. No. 4,873,316 and European Application Publication No. 264,166). Developmentally-regulated promoters are also encompassed, *e.g*., the murine hox promoters (Kessel and Gruss (1990) Science 249:374-379) and the α-fetoprotein promoter (Campes and Tilghman (1989) Genes Dev 3:537-546).

Another aspect pertains to host cells into which a recombinant expression vector has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, the SIRT1 or LXR protein can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g*., DNA) into a host cell, including calcium phosphate or calcium chloride co-precipitation, DEAE-dextran-mediated transfection, lipofection, or electroporation. Suitable methods for transforming or transfecting host cells can be found in Sambrook, *et al.* (2001), Ausubel et al. (2002), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g*., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding the growth promoter or can be introduced on a separate vector. Cells stably transfected with the introduced nucleic acid can be identified by drug selection (*e.g*., cells that have incorporated the selectable marker gene will survive, while the other cells die).

A host cell, such as a prokaryotic or eukaryotic host cell in culture, can be used to produce (i.e., express) the SIRT1 or LXR protein. Accordingly, the disclosure further provides methods for producing the SIRT1 or LXR protein using the host cells of the invention. In one embodiment, the method comprises culturing the host cell (into which a recombinant expression vector encoding the SIRT1 or LXR protein has been introduced) in a suitable medium such that the SIRT1 or LXR protein is produced. In another embodiment, the method further comprises isolating the SIRT1 or LXR protein from the medium or the host cell.

Transfection of a vertebrate cell can further be accomplished using recombinant vectors which include, but are not limited, to adenovirus, adeno-associated virus, and retrovirus vectors, in addition to other particles that introduce DNA into cells, such as liposomes. Techniques such as those described above can be utilized for the introduction of any SIRT1 or LXR polypeptide encoding nucleotide sequences into vertebrate cells. For example, for transfection of mammalian cells, a number of viral-based expression systems may be utilized. In cases where an adenovirus is used as an expression vector, the SIRT1 or LXR nucleotide sequence of interest may be ligated to an adenovirus transcription/translation control complex, *e.g*., the late promoter and tripartite leader sequence. This chimeric gene may then be inserted in the adenovirus genome by in vitro or in vivo recombination. Insertion in a non-essential region of the viral genome (e.g., region E1 or E3) will result in a recombinant virus that is viable and capable of expressing an SIRT1 or LXR product in infected hosts (e.g., See Logan & Shenk, 1984, Proc. Natl. Acad. Sci. USA 81:3655-3659). In cases where only a portion of an SIRT1 or LXR coding sequence is inserted, exogenous translational control signals, including, perhaps, the ATG initiation codon, must be provided. These exogenous translational control signals and initiation codons can be of a variety of origins, both natural and synthetic. The efficiency of expression can be enhanced by the inclusion of appropriate transcription enhancer elements, transcription terminators, etc. (See Bitter et al., 1987, Methods in Enzymol. 153:516-544).

### Therapeutic Treatment

Certain pathologies and medical conditions are believed to respond favorably to the expression of a heterologous SIRT1 or LXR or a heterologous LXRα or LXRβ in the cells of a subject. Accordingly, the present disclosure discloses a method of treating a pathology, a disease or a medical condition in a subject, wherein the pathology responds to a SIRT1 or LXR, LXRα or LXRβ polypeptides. The method includes administering a nucleic acid encoding an SIRT1 or LXR, LXRα or LXRβ polypeptide to the subject in an amount effective to attenuate or ameliorate the pathology. Attenuating a pathology signifies that a trend of worsening symptomology is abated to a slower or more gentle trend of worsening. Ameliorating a pathology signifies an actual improvement in the patient, such that the signs and indications of the pathology diminish, and the patient improves toward better health. In important implementations of this method the pathology is chosen from among myocardial infarction, cerebrovascular stroke, a kidney disease, a neurological disease including Alzheimer's disease, and the like. In advantageous embodiments of the method of treating a pathology the subject is a human.

In various embodiments of the methods of treatment described herein, a nucleic acid encoding an SIRTI or LXR, LXRα or LXRβ polypeptide, a variant thereof, or a fragment thereof, may be administered to a subject in any of a variety of compositions that ensure efficient delivery of the nucleic acid sequence into cells, including delivery into the cells of a subject.

Treatment of a subject with an *SIRT1 or LXR* nucleic acid sequence can be accomplished by administering a suitable nucleic acid, plasmid, vector, viral vector, liposomal or similar composition that is effective to introduce the *SIRT1 or LXR* nucleic acid sequence into a vertebrate cell. Transfection of nucleic acids may be assisted with the use of cationic amphiphiles (US Patent 6,503,945 and references disclosed therein). Ex vivo retroviral gene therapy is described, for example, in Hacein-Bey-Abina et al. (2003, Science302:415-419). Methods for therapeutic introduction of a transgene into a subject are discussed in "Gene Transfer Methods: Introducing DNA Into Living Cells and Organisms" P.A. Norton and L.F. Steel, Eaton Publishing, 2000. Approaches to the therapeutic introduction of transgenes into cells and organism are provided in "Gene Therapy Protocols" Paul D. Robbins (Ed.), Humana Press (1997).

### Pharmaceutical Compositions Comprising Polynucleotides

Pharmaceutical compositions for therapeutic applications include one or more polynucleotides and a carrier. The pharmaceutical composition comprising the one or more polynucleotide is useful for treating a disease or disorder associated with the expression or activity of a Target Gene. Carriers include, but are not limited to, saline, buffered saline, dextrose, water, glycerol, ethanol, and combinations thereof. For drugs administered orally, pharmaceutically acceptable carriers include, but are not limited to, pharmaceutically acceptable excipients such as inert diluents, disintegrating agents, binding agents, lubricating agents, sweetening agents, flavoring agents, coloring agents and preservatives.

A maximum dosage of 5 mg polynucleotide per kilogram body weight of recipient per day is sufficient to inhibit or completely suppress expression of the target gene. In general, a suitable dose of polynucleotide will be in the range of 0.01 to 5.0 milligrams per kilogram body weight of the recipient per day, preferably in the range of 0.1 to 200 micrograms per kilogram body weight (mcg/kg) per day, more preferably in the range of 0.1 to 100 mcg/kg per day, even more preferably in the range of 1.0 to 50 mcg/kg per day, and most preferably in the range of 1.0 to 25 mcg/kg per day. The pharmaceutical composition may be administered once daily, or the polynucleotide may be administered as two, three, four, five, six or more sub-doses at appropriate intervals throughout the day. In that case, the polynucleotide contained in each sub-dose must be correspondingly smaller in order to achieve the total daily dosage. The dosage unit can also be compounded as a sustained release formulation for delivery over several days, e.g., using a conventional formulation which provides sustained release of the polynucleotide over a several day period. Sustained release formulations are well known in the art. In this embodiment, the dosage unit contains a corresponding multiple of the daily dose.

The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a composition can include a single treatment or a series of treatments. Estimates of effective dosages and in vivo half-lives for the individual polynucleotides encompassed by the invention can be made using conventional methodologies or on the basis of in vivo testing using an appropriate animal model, and can be adjusted during treatment according to established criteria for determining appropriate dose-response characteristics.

The pharmaceutical compositions encompassed by the invention may be administered by any means known in the art including, but not limited to oral or parenteral routes, including intravenous, intramuscular, intraperitoneal, subcutaneous, transdermal, airway (aerosol), rectal, vaginal and topical (including buccal and sublingual) administration. In certain embodiments, the pharmaceutical compositions are administered by intravenous or intraparenteral infusion or injection, and in additional common embodiments the pharmaceutical composition comprising polynucleotides may be delivered directly in situ to a tumor, a cancer or a precancerous growth using laparoscopic and similar microsurgical procedures.

For intramuscular, intraperitoneal, subcutaneous and intravenous use, the pharmaceutical compositions of the invention will generally be provided in sterile aqueous solutions or suspensions, buffered to an appropriate pH and isotonicity. Suitable aqueous vehicles include Ringer's solution and isotonic sodium chloride. In a preferred embodiment, the carrier consists exclusively of an aqueous buffer. In this context, "exclusively" means no auxiliary agents or encapsulating substances are present which might affect or mediate uptake of polynucleotide in the cells that express the target gene. Such substances include, for example, micellar structures, such as liposomes or capsids, as described below. Surprisingly, the present inventors have discovered that compositions containing only naked polynucleotide and a physiologically acceptable solvent are taken up by cells, where the polynucleotide effectively inhibits expression of the target gene. Although microinjection, lipofection, viruses, viroids, capsids, capsoids, or other auxiliary agents are required to introduce polynucleotide into cell cultures, surprisingly these methods and agents are not necessary for uptake of polynucleotide in vivo. Aqueous suspensions according to the invention may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

The pharmaceutical compositions useful according to the invention also include encapsulated formulations to protect the polynucleotide against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811; PCT publication WO 91/06309; and European patent publication EP-A-43075.

In certain embodiments, the encapsulated formulation comprises a viral coat protein. In this embodiment, the polynucleotide may be bound to, associated with, or enclosed by at least one viral coat protein. The viral coat protein may be derived from or associated with a virus, such as a polyoma virus, or it may be partially or entirely artificial. For example, the coat protein may be a Virus Protein 1 and/or Virus Protein 2 of the polyoma virus, or a derivative thereof.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred.

The data obtained from cell culture assays and animal studies can be used in formulating a range of dosages for use in humans. The dosage of compositions of the invention lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays and animal models to achieve a circulating plasma concentration range of the compound that includes the IC50 (i.e., the concentration of the test compound which achieves a half-maximal inhibition of symptoms) as determined in cell culture. Such information can be used to more accurately determine useful doses in humans.

In addition to their administration individually or as a plurality, as discussed above, the polynucleotides useful according to the invention can be administered in combination with other known agents effective in treatment of diseases. In any event, the administering physician can adjust the amount and timing of polynucleotide administration on the basis of results observed using standard measures of efficacy known in the art or described herein.

### Pharmaceutical Compositions

The polynucleotides disclosed in the invention are designated "active compounds" or "therapeutics" herein. These therapeutics can be incorporated into pharmaceutical compositions suitable for administration to a subject.

Described herein are methods for increasing the ratio of cholesterol bound to high density lipoprotein (HDL) to total cholesterol in the plasma of a mammal by administering an agent that stimulates SIRTI activity, as well as se of an agent that stimulates SIRT1 activity in the manufacture of a medicament for increasing the ratio of cholesterol bound to high density lipoprotein (HDL) to total cholesterol in the plasma of a mammal by administering the agent to the mammal. Such agents are designated "active compounds" or "therapeutics" herein. An example of such an agent is T0901317. These therapeutics can be incorporated into pharmaceutical compositions suitable for administration to a subject.

Described herein are also, methods for increasing the ratio of cholesterol bound to high density lipoprotein (HDL) to total cholesterol in the plasma of a mammal by administering an agent that promotes formation of a complex comprising a mammalian SIRT1 protein and a mammalian LXR protein. In addition we disclose the use of an agent that promotes formation of a complex comprising a mammalian SIRT1 protein and a mammalian LXR protein in the manufacture of a medicament for increasing the ratio of cholesterol bound to high density lipoprotein (HDL) to total cholesterol in the plasma of a mammal by administering the agent to the mammal. Such agents are designated "active compounds" or "therapeutics" herein. Examples of such agents include either 22(R)-hydroxycholesterol, or 9-cis retinoic acid, or combinations of both 22(R)-hydroxycholesterol and 9-cis retinoic acid. These therapeutics can be incorporated into pharmaceutical compositions suitable for administration to a subject.

As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in textbooks such as Remington's Pharmaceutical Science, Gennaro AR (Ed.) 20^{th} edition (2000) Williams & Wilkins PA, USA, and Wilson and Gisvold's Textbook of Organic Medicinal and Pharmaceutical Chemistry, by Delgado and Remers, Lippincott-Raven. Preferred examples of components that may be used in such carriers or diluents include, but are not limited to, water, saline, phosphate salts, carboxylate salts, amino acid solutions, Ringer's solutions, dextrose (a synonym for glucose) solution, and 5% human serum albumin. By way of nonlimiting example, dextrose may used as 5% or 10% aqueous solutions. Liposomes and non-aqueous vehicles such as fixed oils may also be used. The use of such media and agents for pharmaceutically active substances is well known in the art. Supplementary active compounds can also be incorporated into the compositions.

A pharmaceutical composition of the invention is formulated to be compatible with its intended route of administration. Examples of routes of administration include parenteral, *e.g*., intravenous, intradermal, subcutaneous, oral, nasal, inhalation, transdermal (topical), transmucosal, and rectal administration. Solutions or suspensions used for parenteral, intravenous, intradermal, or subcutaneous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose.

For administration by inhalation, the compounds are delivered in the form of an aerosol spray from pressured container or dispenser which contains a suitable propellant, *e.g*., a gas such as carbon dioxide, or a nebulizer.

In one embodiment, the active compounds are prepared with carriers that will protect the compound against rapid elimination from the body, such as a controlled release formulation, including implants and microencapsulated delivery systems. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773;919), copolymers of L-glutamic acid and γ ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release pharmaceutical active agents over shorter time periods. Advantageous polymers are biodegradable, or biocompatible. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811. Sustained-release preparations having advantageous forms, such as microspheres, can be prepared from materials such as those described above.

The polynucleotides disclosed can be inserted into vectors and used as gene therapy vectors. Gene therapy vectors can be delivered to a subject by any of a number of routes, *e.g.*, as described in U.S. Patent Nos. 5,703,055. Delivery can thus also include, *e.g.*, intravenous injection, local administration (see U.S. Pat. No. 5,328,470) or stereotactic injection (see *e.g.*, Chen et al. (1994) Proc. Natl. Acad Sci. USA 91:3054-3057). The pharmaceutical preparation of the gene therapy vector can include the gene therapy vector in an acceptable diluent, or can comprise a slow release matrix in which the gene delivery vehicle is embedded Alternatively, where the complete gene delivery vector can be produced intact from recombinant cells, *e.g.*, retroviral vectors, the pharmaceutical preparation can include one or more cells that produce the gene delivery system.

The pharmaceutical compositions can be included in a kit, *e.g.*, in a container, pack, or dispenser together with instructions for administration.

Also within the invention is the use of a therapeutic in the manufacture of a pharmaceutical composition or medicament for treating a respiratory viral infection in a subject.

In several embodiments the polynucleotides disclosed in the invention are delivered by liposome-mediated transfection, for example by using commercially available reagents or techniques, e.g., Oligofectamine™, LipofectAmine™ reagent, LipofectAmine 2000™ (Invitrogen), as well as by electroporation, and similar techniques. Additionally polynucleotides are delivered to animal models, such as rodents or non-human primates, through inhalation and instillation into the respiratory tract. Additional routes for use with animal models include intravenous (IV), subcutaneous (SC), and related routes of administration. The pharmaceutical compositions include additional components that protect the stability of polynucleotides, prolong their lifetime, potentiate their function, or target them to specific tissues/cells. These include a variety of biodegradable polymers, cationic polymers (such as polyethyleneimine), cationic copolypeptides such as histidine-lysine (HK) polypeptides see, for example, PCT publications WO 01/47496 to Mixson et al., WO 02/096941 to Biomerieux, and WO 99/42091 to Massachusetts Institute of Technology), PEGylated cationic polypeptides, and ligand-incorporated polymers, etc. positively charged polypeptides, PolyTran polymers (natural polysaccharides, also known as scleroglucan; Pillsbury Co., Minneapolis, Minn), surfactants (Infasurf; Forest Laboratories, Inc.; ONY Inc.), and cationic polymers (such as polyethyleneimine). Infasurf® (calfactant) is a natural lung surfactant isolated from calf lung for use in intratracheal instillation; it contains phospholipids, neutral lipids, and hydrophobic surfactant-associated proteins B and C. The polymers can either be uni-dimensional or multi-dimensional, and also could be microparticles or nanoparticles with diameters less than 20 microns, between 20 and 100 microns, or above 100 micron. The said polymers could carry ligand molecules specific for receptors or molecules of special tissues or cells, thus be used for targeted delivery of polynucleotides. The polynucleotides are also delivered by cationic liposome based carriers, such as DOTAP, DOTAP/Cholesterol (Qbiogene, Inc.) and other types of lipid aqueous solutions. In addition, low percentage (5-10%) glucose aqueous solution, and Infasurf are effective carriers for airway delivery of polynucleotides.

### Gene Therapy

Currently gene therapy is in a high state of development for use in therapeutic and prophylactic (e. g. immunizing) settings. Novel delivery methods are available, and several clinical trials of various gene therapeutic modalities are under way. U. S. Patent Application Publication 20060115462 entitled "Direct DNA delivery to bone cells" discloses a method that enables in vivo delivery of a polynucleotide such as a naked polynucleotide or a gene expression vector to cells of a mammalian bone limb.

U. S. Patent Application Publication 20060115456 entitled "Replication-competent adenoviral vectors" discloses improved replication-competent adenoviral vectors, including adenovirus types 2, 4, 5, and 7, for use in delivery of nucleic acids and polynucleotides for therapeutic and prophylactic applications.

U. S. Patent Application Publication 20060104950 entitled "Methods ofTranducing genes into T cells" discloses transduction of a gene into activated T cells using a paramyxovirus vector.

U. S. Patent Application Publication 20060110361 entitled "Methods of making viral particles having a modified cell binding activity and uses thereof" discloses a method for packaging viral particles such that one or more peptides on the surface of the article are derived from the packaging cell. The application states that such a system is of use, for example, in gene therapy treatments.

U. S. Patent Application Publication 20060110364 entitled "Vector-mediated delivery of polynucleotides encoding soluble VEGF receptors" discloses vector compositions for expression of a soluble form of VEGFR3 and methods for their use in the treatment of cancer.

U. S. Patent Application Publication 20050239204 entitled "Multifunctional molecular complexes for gene transfer to cells" discloses a non-viral multifunctional molecular complex for the transfer of a nucleic acid composition to a target cell.

VandenDriessche et al. showed that hemophilia A could be effectively treated by an in vivo gene therapy that introduces the human Factor VIII gene into Factor VIII-deficient mice by means of retroviral vectors.⁷⁹ Gene transfer occurred at least in liver, spleen and lungs. The safety of a nonviral somatic cell gene therapy system in patients with severe hemophilia A has been tested by Roth et al.⁸⁰.

Because of its stable, post-mitotic state skeletal muscle is an important target for genetic manipulation by use of both integrating and non-integrating viral and non-viral vectors. Muscle-directed gene therapy to prevent autoimmune diabetes in nonobese diabetic (NOD) mice was reported by Goudy et al.⁸¹ They developed recombinant adeno-associated viral vectors containing murine cDNAs for immunomodulatory cytokines IL4 or IL10. Skeletal muscle transduction of female NOD mice with IL10, but not ILA, completely abrogated diabetes.

According to Wells⁸², adeno-associated viral vectors and naked plasmid DNA are currently the vectors of choice for gene transfer into muscle. Major breakthroughs in the field of vector delivery systems, particularly those using the vascular route have been generated within last two years or so, such that gene therapy of muscular dystrophies and the use of muscle as a platform for the production of secreted proteins has become a clinical possibility.

Oncolytic therapy is a novel anticancer treatment based on attenuated lytic viruses such as adenovirus (Ad).⁸³ These viruses induce destruction of host cells by lysis and are thus distinct from classical gene therapy viruses. Oncolytic Ads are genetically engineered so as to replicate only in cancer cells. Three oncolytic Ads have completed Phase I and II clinical trials in cancer patients. These viruses are being engineered further to provide them with additional therapeutic genes.⁸³

Takahashi et al. have started a clinical study of multidrug resistance (MDR1) gene therapy. The two patients treated to date are in complete remission and suffered no apparent adverse effects from the MDR1 gene transfer.⁸⁴

Broedl et al. describe that, in the face of currently ineffective treatments in various hyperlipidemias, somatic gene therapy is considered to be a potential approach to therapy. In many cases preclinical proof-of-principle studies have already been performed, and in the case homozygous familial hypercholesterolaemia a clinical trial has been conducted

Antisense oligodeoxynucleotides directed against clusterin have been used in a Phase I clinical trial for treatment of prostate cancer.⁸⁶ The treatment provided up to 90% suppression of clusterin in prostate cancer. Miyake et al. state that phase II studies were scheduled to begin in early 2005.⁸⁶.

Satoh et al. review the status of prostate cancer gene therapy, including virus-mediated transduction of the herpes simplex virus thymidine kinase gene followed by a course of the prodrug ganciclovir.⁸⁷ They report that a gene therapy clinical trial for human prostate cancer demonstrated safety, clinical efficacy, and biological effects of antitumor activity. According to Satoh et al; after numerous favorable preclinical studies several clinical studies have been approved for gene therapy of prostate cancer using immunomodulatory cytokines such as interleukin-2, interleukin-12, B7-1 (CD80), B7-2 (CD86) and granulocyte-macrophage colony-stimulating factor.⁸⁷

### Assay Methods for Identifying Potential Active Agents

In various aspects the invention discloses assay methods intended to determine whether a candidate chemical compound or agent modulates an activity of interest in the present invention. Such methods include the following.

Described herein is a method for assessing whether a candidate substance modulates an LXR-dependent process, including
a) transfecting a cell with a vector harboring a reporter gene operably driven by an LXRE;
b) contacting the cell with the candidate; and
c) determining whether the candidate modulates the expression of the reporter gene in comparison with a cell not contacted with the candidate;
whereby a difference in extent of ocrurrence of the LXR-dependent process detected between the presence and absence of the candidate indicates that the candidate modulates the LXR-dependent process.

The invention additionally discloses a method for assessing whether a candidate substance modulates an SIRT1-dependent effect of an LXR, including
a) transfecting a cell with a vector harboring an SIRT1gene;
b) further transfecting the cell with a vector harboring a reporter gene operably driven by an LXR promoter;
c) contacting the cell with the candidate; and
d) determining whether the candidate modulates the expression of the reporter gene in comparison with a cell not contacted with the candidate;
whereby a difference in the extent of the SIRT1-dependent effect of the LXR detected between the presence and absence of the candidate indicates that the candidate modulates the SIRT1-dependent effect of an LXR.

The invention further discloses a method for assessing whether a candidate substance modulates the formation of a specific binding pair comprising specific binding pair members SIRT1 and an LXR, including
a) transfecting a cell with a vector harboring a sequence encoding an epitope-tagged first member of the specific binding pair;
b) further transfecting the cell with a vector harboring a sequence encoding the second member of the specific binding pair;
c) contacting the cell with the candidate substance;
d) lysing the cells, contacting the cell lysates with an antibody specific for the epitope tag, and recovering immunoprecipitates comprising a complex of the SIRT1 and the LXR with an antibody-specific adsorbent;
e) carrying out a Western blot procedure using antibodies specific for SIRT1 and an LXR;
whereby a difference in complex formation detected in the presence of the candidate compared with the absence of the candidate indicates that the candidate modulates the interaction of the SIRT1 with the LXR

In these assay methods, transfection with vectors may be carried out by techniques such as those that have been described above, incorporated herein by reference. Any equivalent technique known to workers of skill in the field of the invention, not specifically described herein, may likewise by used to accomplish transfections.

Various of the assay methods further include transfection with a vector harboring a reporter gene operably driven by an LXRE. The reporter gene will be activated by a cellular component that specifically binds the LXRE to express the reporter gene. The reporter gene product is detectable in the experimental system devised to conduct the assay. In general, a reporter gene encodes a protein, factor, or enzyme activity that upon expression is readily detectable by chemical or physical detection methods. Nonlimiting examples of reporter genes include genes encoding an enzyme such as horse radish peroxidase or firefly luciferase, a chromophoric protein or a fluorescent protein such as green fluorescent protein, and the like. Any equivalent reporter gene known to workers of skill in the field of the invention, not specifically described herein, may likewise by used to transfect the cell in order to carry out the assay for identifying potential active agents.

Various additional embodiments of the assay methods include transfecting a cell with a vector harboring a sequence encoding an epitope-tagged first member of the specific binding pair made up of SIRT1 and an LXR. Epitopes that are usable for this purpose include any epitope bound by a specific antibody. A nonlimiting example of an epitope is the FLAG epitope; others are widely known to workers of skill in the field of the invention. Any equivalent epitope, not specifically described herein, may by used to as a chimera with a member of the specific binding pair in order to carry out the assay for identifying potential active agents.

Assays for potential active agents are frequently carried out using high throughput screens in highly replicated parallel assay apparatuses. Multiwell plates may be employed in these systems such that a large number of assays are carried out simultaneously. The candidate compounds employed in assays for detecting a potential active agent are commonly obtained as the result of chemical synthesis of combinatorial libraries of chemical compounds, in which various moieties and/or substituents in a family of related compounds are systematically varied. Analogous chemical libraries are commonly already available in stocks of compounds accessible to a person or an entity. Further analogous chemical libraries include chemicals obtained from disparate natural sources, as well as derivatives of such natural compounds that have been further modified using the methods of combinatorial chemistry. In general, libraries of chemical compounds are widely known to workers of skill in the field of the invention. Any equivalent method for preparing a candidate compound, or for preparing a library of candidate compounds, known to workers of skill in the field of the invention, may be used in carrying out an assay to identify a potential active agent.

### EXAMPLES

### Methods

### Plasmids

The plasmids pBabe-SIRT1 and pCMV-FLAG-HsSIRT1 have been described ^{15,16}.

Animals and plasma lipoprotein analysis

SIRTI^{+/+}, SIRT1^{+/-}, and SIRT1^{-/-} mice in mixed 129/sv-CD1 background were housed in a temperature-controlled (22 °C) facility with a daylight cycle from 7 a.m. to 7 p.m. and were given access to food (with 0.02% cholesterol) and water ad libitum.

Cells, retroviral infection, and transfection

The SIRT1^{+/+}, SIRT1^{+/-}, and SIRT1^{-/-} mouse embryonic fibroblasts (MEFs) were isolated from E14.5 embryos as previously described ⁷³, and were cultured in Dulbecco's modified Eagle's medium (DMEM) with 10% FBS and antibiotics. Immortalized MEFs (MEFIs) were generated by transfecting primary MEFs with a pRS-SV40T plasmid. HEK293T and Phoenix cells (ATCC, Rockville, MD) were cultured in DMEM with 10% FBS and antibiotics. THP-1 cells (ATCC, Rockville, MD) were cultured in RPMI 1640 with 10% FBS and 0.5 mM β-mecaptoethanol.

All transfections were performed with lipofectamine 2000 plus reagent according to the manufacturer's instructions (Invitrogen, Carlsbad, Ca). For retroviral infection, Phoenix cells were transfected with either pBabe or pBabe-SIRT1. Medium containing retroviruses was collected 48 h later, filtered, treated with polybrene (1 mg/ml), and transferred on MEFs. Infected cells were then cultured with normal medium containing 1 µg/ml puromycin.

### Example 1. Plasma Cholesterol in Wild Type and SIRT1 Knockout Mice.

Ten wildtype, 13 *SIRT1*^{+/-}*,* and 13 littermate *SIRT1*^{-/-} male mice in each group were analyzed. Animals were fasted for four hours from the beginning of the daylight cycle, then blood was collected and plasma was obtained by K₃-EDTA treatment. Plasma total cholesterol, HDL, and LDL levels were measured by the enzymatic, colorimetric assay kits (Wako Diagnostics, Richmond, Va.). 100 µl of pooled plasma from 4 *SIRT1*^{-/-} and 4 littermate *SIRT1*^{+/+} males were size fractionated using two fast-performance liquid chromatography (FPLC) columns (Superose 6B columns, Amersham-Pharmacia Biotech, Piscataway, NJ). A representative profile is shown from three independent experiments. Fractions from FPLC were then analyzed for cholesterol contents with the enzymatic, colorimetric assay kit from Wako. The experiment was repeated three times with total of 12 *SIRT1*^{*-*/}*⁻* and 12 littermate *SIRT1*^{+/+} males. Results were compared with student's t-test.

Fig. 1a shows that *SIRT1*^{-/-} mice had slightly lower total plasma cholesterol levels, compared with wildtype age- and gender-matched mice. Upon inspection of Fig. 1, panels b (direct enzymatic assay) and d (size exclusion chromatographic/enzymatic assay), it is seen that this is primarily due to an approximately 40% reduction in the cholesterol carried in HDL (*, *p*<0.001), the principal cholesterol-rich lipoprotein (80-90% of total) in murine plasma. The plasma LDL cholesterol levels were similar in *SIRT1*^{-/-} and control mice (Fig. 1c and d). As a result, the total cholesterol/HDL ratio *of SIRT1*^{-/-} mice was abnormally high (2.57± 0.98 vs.1.36 ± 0.19 for wildtype controls, p<0.001), whereas the HDL/LDL ratio was abnormally low (1.92 ± 0.94 vs. 3.56 ±1.24, p<0.001). Because of the pleitropic effects in the SIRT1^{-/-} mice, SIRT1^{+/-} heterozygotes, which are phenotypically similar to wildtype, were also examined. HDL levels were also found to be significantly reduced in SIRT1^{+/-}heterozygotes (Fig 1b, (*, *p*<0.001)).

Total cholesterol (TC), free cholesterol (FC), cholesterol ester (CE), and phospholipids (PL) contents were measured in HDL fractions (fraction # 25-38) from the 12 pairs of animals, shown in Fig. 1, panel e. The relative reductions in SIRT1-/- mice of the levels of total cholesterol, free cholesterol, cholesterol ester and phospholipids in plasma HDL were similar, about 25-30% (Fig. 1e; **, p<0.01). The size distribution of HDL was normal (Fig. 1d) in *SIRT1*^{-/-} mice, suggesting that the reduction in HDL was a consequence of fewer particles rather than of particles with altered composition or structure.

### Example 2. Cholesterol in Tissues of Wild Type and SIRT1 Knockout Mice.

To determine the total cholesterol levels in mouse tissues, *SIRT1*^{*-*/-} mice and control littermates were fasted for four hours from the beginning of the daylight cycle before sacrificing. Tissues were then harvested and weighted. Total cholesterol from liver and testis were extracted and measured by GC as described previously ^{70.71}. Total lipids including triglycerides were also dissolved into a solution containing 60% butanol, 13% methanol, and 27% Triton X-100, and measured with the enzymatic, colorimetric assay kits from Wako.

In *SIRT*^{-/-} mice, abnormally low plasma HDL (Example 1) was associated with an increase in the accumulation of cholesterol within two tissues for which HDL is important as a source of exogenous cholesterol: the testis, which uses HDL cholesterol for sterol stores and steroidogenesis ²⁵ and is the organ that has highest relative levels of SIRT1 protein ^{26.27}, and the liver, which plays a central role in cholesterol and HDL homeostasis ²⁸ (Fig. 1f; n=8; ***, *p*<0.01).

The decrease in plasma HDL cholesterol and increase in accumulation of tissue cholesterol in the liver and testis in *SIRT1*^{-/-} mice could occur because of increases in lipoprotein cholesterol uptake or decreases in cellular cholesterol efflux (export) to lipoproteins. Increase in tissue cholesterol could also be due to increases in local cholesterol synthesis. However, we observed in the liver and testis essentially normal levels of mRNA for the highly sensitive, cholesterol-regulated gene encoding the rate controlling enzyme in cholesterol biosynthesis, HMG-CoA reductase (data not shown). Also, there were no apparent increases in these tissues in the protein levels of the major HDL receptor SR-BI (data not shown) that mediates cellular uptake of HDL cholesterol ²⁹, nor increases in the rates of uptake of lipid from HDL in *SIRT1*^{-/-} mouse embryonic fibroblasts (MEFs; data not shown). Furthermore, there were substantial decreases, rather than increases, in the mRNA levels in testis and liver of two other major lipoprotein receptors, the LDL receptor (LDLR), and the VLDL/chylomicron receptor LRP (data not shown). Because neither increased *de novo* cholesterol synthesis nor receptor-mediated import appears to account for the increases in tissue and decreases in plasma cholesterol in *SIRT1*-/- mice, it seemed likely that reductions in cellular cholesterol efflux might account for these observations.

### Example 3. Efflux of Cholesterol from Wild Type and SIRT1^{-/-} Cells.

Reverse cholesterol transport is the process whereby excess cholesterol in peripheral tissues is transporter to the liver for elimination from the body ^{30.31}. The first step of this process is the efflux of cholesterol from cells to lipoproteins, particularly HDL. Several cell surface cholesterol transport proteins can mediate cholesterol efflux, including SR-BI ³²⁻³⁵, ABCG1^{36,37}, and the best characterized of these, the ATP-binding cassette (ABC) transporter called ABCA1 that transfers unesterified cholesterol and phospholipids to lipid-poor apolipoproteins (mainly apoA-I) to form HDL particles ^{30.38-44}. To test the effects of SIRT1 on this process, apoA-I -mediated cholesterol efflux was measured in two distinct cultured cell systems in which cells were labeled with [³H]cholesterol and the afflux of labeled cholesterol to apoA-I in the extracellular medium was monitored.

For MEFs, cholesterol efflux assay was performed as described ⁷² with modifications. Primary *SIRT1*^{-/-} and littermates control *SIRT1*^{+/+} or *SIRT1*^{+/-} MEFs were plated in 12-well plates at 50% confluence and cultured for overnight. Cells were then transferred into RPMI 1640/0.2% bovine serum albumin (BSA) for 24 h and cholesterol loaded by incubation with 50 µg/ml of LDL and 1 µCi/ml of [³H]cholesterol (NEN Life Science Products, Boston, Ma.) for additional 24 h. Cells were washed with PBS, equilibrated for 4 h in RPMI 1640/0.2% BSA, and then incubated in RPMI 1640/0.2% BSA with or without 15 µg/ml apoAI for 24h. The medium was then collected, and cells were lysed with 0.2 N NaOH. The radioactivity recovered in the medium and cell lysates were measured, and the apoAI-mediated cholesterol efflux was calculated as the percentage of the radioactivity recovered in the medium over the total radioactivity subtracting of the nonspecific efflux in apoAI-free medium. The cholesterol efflux assays were performed in duplicates with 7 pairs of *SIRT1*^{-/-} and control *SIRT1*^{+/+} or *SIRT1*^{+/-} MEFs from 3 litters.

Using primary MEFs, wildtype control cells and *SIRT1*^{-/-} cells loaded with [³H]cholesterol were incubated with RPMI 1640 medium with or without 15 µg/ml apoAI. [³H]cholesterol levels in the cells and culture medium were measured 24 h later and apoAI-mediated cholesterol efflux was calculated. Fig 1g (left panel) shows that efflux from primary control *SIRT1*^{+/+} MEFs (filled bar) was almost three-fold higher than from *SIRT1*^{-/-}MEFs (open bar) (n=7; ****, *p*<0.001).

For the human monocytic cell line THP-1 cells, cells were treated with 10 mM nicotinamide in RPMI1640/10% FBS for 48 h, then cholesterol loaded with 50 µg/ml of LDL and 1 µCi/ml of [³H]cholesterol in RPMI1640/1% FBS/0.2% BSA medium for 24 h. Cells were washed with PBS and incubated in RPMI 1640/0.2% BSA with or without 15 µg/ml apoAI for 24 h. The medium and cells were then separated, and cells were lysed with 0.2N NaOH. The radioactivity recovered in the medium and cell lysates were measured and the apoAI-mediated cholesterol afflux was calculated as described above.

Treatment of TBP-1 with the SIRT1 inhibitor nicotinamide (Nico) ⁴⁵ also suppressed [³H]chotesterol efflux by roughly three-fold (Fig. 1g, right panel). The experiment was performed in triplicate and repeated twice (*p*<0.01). Thus loss of SIRTI in MEFs and inhibition of SIRT1 activity in monocytes reduces apoAI-mediated cholesterol efflux. These results support the possibility that reductions in cholesterol efflux might lead to reduced plasma HDL cholesterol and increased tissue cholesterol in *SIRT1*^{-/-} mice.

### Example 4. Levels of ABCA1 mRNA in Wild Type and SIRT1 Knockout Tissues.

To examine whether lower cholesterol efflux could produce reduced plasma HDL cholesterol and increased tissue cholesterol in *SIRT1*^{*-*/}*⁻* mice, the levels of ABCA1 mRNA in liver, testis, and ovary were compared.

Total RNA was isolated from mouse livers, testes, thioglycolate-stimulated peritoneal macrophages, and ovaries by a Qiagen RNeasy mini-kit (Qiagen Inc., Calencia, Ca.). For real-time PCR analysis, cDNA was synthesized by SuperScript III reverse transcriptase (Invitrogen, Carlsbad, Ca) with random primers. The resulting cDNA was then subjected to PCR analysis with gene-specific primers in the presence of Cybergreen (Qiagen Inc., Calencia, Ca.). Relative abundance of mRNA was obtained by normalization to cyclophilin levels. For northern hybridization, RNA (10 µg/lane) was separated on formaldehyde-agarose gels (1.0%), transferred to GeneScreen Plus membranes (NEN Life Science Products, Boston, Ma.), and hybridized according to standard procedures. The mRNA levels of ABCA1 were quantified by phosphoimager and normalized against the corresponding actin levels.

ABCA1 mRNA expression was reduced by 40-50% in *SIRT1*^{-/-} mice in the three tissues tested, compared with wild type (Fig. 2a). Consistent with the idea that this reduction might have contributed to the reduced levels of plasma HDL, in *ABCA1*^{-/-} heterozygous mice, the HDL cholesterol is reduced to ∼60% of ABCA1^{+/+} controls ⁴⁶, similar to the reduction in *SIRT1*^{*-*/}*⁻* mice.

### Example 5. Activity of the ABCA1 Promoter.

Transcription of ABCA1 is induced by increasing cellular cholesterol, and the LXR nuclear receptor transcriptional factors mediate this response ⁴⁷⁻⁴⁹. LXR/ retinoid X receptor (RXR) heterodimers are activated by oxysterols ⁵⁰ and play key roles in the regulation of whole body cholesterol homeostasis, lipid biosynthesis, inflammatory response, carbohydrate metabolism, and obesity ⁵¹. To determine if the effects of SIRT1 on expression of ABCA1 are mediated by LXRs, reporter constructs were generated in which expression of the firefly luciferase gene was driven by either the mouse ABCA1 promoter or a control mutant promoter in which an eight-nucleotide deletion removed the LXR response element (LXRE). Previous studies have shown that a combination of 22(R)-hydroxycholesterol (22(R)-HC, a LXR agonist) and 9-cis retinoic acid (9-cisRA, a RXR agonist) dramatically stimulates the activity of the LXRE in the ABCA1 promoter ⁴⁷.

The luciferase reporter plasmid pGL3-ABCA1 was created by inserting the - 847 to +244 of mouse ABCA1 promoter region into KpnI-XhoI sites of pGL3 vector (Promega, Madison, Wi). pGL3-ABCA1-LXRE, the LXRE mutant version of pGL3-ABCA1, was created by deleting the LXRE from wildtype promoter of mouse ABCA1.

For the luciferase assay, MEFIs or HEK293T cells plated in 24-well plates were transfected with 100 ng of pGL3-ABCA1 or pGL3-ABCA1-LXRE, and 10 ng of pRL-TK (*Renilla* luciferase; Promega). The cells transfected with wildtype (WT) or LXRE mutant (-LXRE) mouse ABCA1 promoter driven luciferase reporter vectors were treated with 1 µM Trichostatin A (TSA), an inhibitor of class I and II deacetylases, or 10 mM nicotinamide (Nico), or both 10 mM nicotinamide + 1 µM TSA. Cells transfected with the LXRE mutant vector were incubated in DMEM with 10% FBS in the absence (solid bars) or presence (open bars) of 10 µM 22(R)-HC and 1 µM 9-cisRA (Fig. 2b). Luciferase activity was then measured by using the Dual-Luciferase Reporter Assay System (Promega) 6 or 24 h later. The final GL (firefly luciferase) activity was normalized with co-expressed RL (*Renilla* luciferase) activity. The experiments were performed in duplicate and were repeated 3 times.

The wildtype and mutant reporter constructs were expressed in HEK293T cells (Fig. 2b, WT (left panel) or -LXRE (right panel)). Luciferase expression from the wildtype was stimulated 8-fold by treatment with 22(R)-HC+9-cisRA (Fig. 2b, left panel). Moreover, this induced expression was inhibited 50% by the inhibitor nicotinamide which inhibits class m deacetylases such as SIRT1 (a), but was not inhibited by TSA. Constructs missing the LXRE were unresponsive to the LXR agonist and showed a low basal activity (Fig. 2b, right panel). Together, these findings suggest that SIRT1, but not other classes of deacetylases, modulates LXR activity at the ABCA1 promoter, interestingly as a positive regulator.

In addition, it is seen that the system used in these experiments affords a method for assessing the modulation of LXR-dependent processes by a candidate substance.

### Example 6. Effect of SIRT1 Activity on the ABCA1 Promoter.

To directly determine the influence of SIRT1 on the ABCA1 promoter, these promoter reporter constructs were introduced into immortalized *SIRT1*^{+/+} and *SIRT1*^{-/-} MEFs that were or were not infected with the pBabe-mT1 vector to express SIRT1 protein (Fig. 2c). *SIRT1*^{+/+} *and SIRT1*^{-/-} MEFs were generated and infected with pBabe-SIRT1 (+) or a pBabe control vector (-) as described in Example 3.

The insert in Fig. 2c, right panel, shows the immuno-blotting results of the expression levels of SIRT1 in these four cell lines; tubulin was used as loading control. Expression of SIRT1 from this vector was slightly lower than that of endogenous SIRT1 in *SIRT1*^{+/+} MEFs.

MEFs were then transfected with wildtype (WT) or LXRE mutant (-LXRE) mouse ABCA1 promoter driven luciferase reporter vectors in the absence (filled bar) or presence (open bar) of 10 µM 22(R)-HC and 1 µM 9-cisRA. Luciferase activities were measured after 24 h. All cells with the wildtype promoter exhibited marked stimulation of luciferase expression when treated with the LXR/RXR activators (Fig.2 c, left panel), whereas the low basal activity in cells with the control mutant (-LXRE) reporter was not stimulated by these activators (Fig. 2c, right panel). Most importantly, LXRE-dependent activity of the wildtype promoter construct was 40% lower (p<0.02) in cells that were SIRT1 deficient *(SIRT1*^{-/-}MEFs) than in cells that were *SIRT1*^{+/+}*,* or were reconstituted with SIRS1 (pBabe-T1). These findings support the model that SIRT1 stimulates the ABCA1 promoter via LXR/RXR acting at the LXRE.

The experimental system in this Example affords a method for assessing the modulatory effect of a candidate substance on the SIRT1-dependent effect of LXRs.

### Example 7. Interaction of SIRT1 with the LXRE of ABCA1 Promoter.

To determine if SIRT1 binds directly to the LXRE in the ABCA1 promoter, we used the chromatin immunoprecipitation (ChIP) assay and probed with different primers for the murine ABCA1 promoter.

ChIP analysis was performed as described ⁷⁶ with modifications. Briefly, cells were crosslinked in 1% paraformaldehyde in cultured media for 15 min at room temperature and crosslinking was terminated with 0.125M glycine. Cells were then harvested in HEPES buffer (50 mM HEPES-NaOH, pH 7.9, 140 mM NaCl, 1 mM EDTA, 10 % Glycerol, 0.5% NP40, 0.25% Triton X-100, and Complete™ protease inhibitor mixture (Roche, Indianapolis, In)), and nucleus were purified. Purified nucleus were resuspended in TE buffer (10 mM Tris-HCl, pH 8.0, 1mM EDTA, and Complete™ protease inhibitor mixture), and sonicated on ice to an average length of 1 kb. Sonicated supernatants were diluted into IP buffer (10 mM Tris-HCl, pH 8.0, 1 mM EDTA, 10 % Glycerol, 1% Triton X-100, 0.1% Na-Deoxycholate, and Complete™ protease inhibitor mixture), precleaned twice with protein A beads. Precleaned chromatin samples were then incubated with anti-SIRT1 rabbit polyclonal antibodies (Upstate, Charlottesville, Va) overnight at 4°C, and immune complexes were recovered by adding protein A beads for 2h. Recovered immunoprecipitates were then washed with IP buffer twice, IP buffer with 500 mM NaCl twice, and RIPA buffer (10 mM Tris-HCl, pH 8.0, 1 mM EDTA, 250 mM NaCl, 0.5% NP40, 0.5% Na-deoxycholate, and Complete™ protease inhibitor mixture) twice. Chromatins in immunoprecipitates were then eluted with 10×TE/1% SDS at 65°C for 15 min. Eluted chromatins were incubated at 65°C for at least 6 h to reverse crosslinking, treated with proteinase K and RNaseA, and extracted with phenol/chloroform and chloroform. Final DNA fragments were recovered by LiCl/glycogen/ethanol precipitation and pellets were resuspended in water and assayed by PCR.
For the LXRE of mouse ABCA1 promoter, PCR reaction was performed using primers
5'-GCTTTCTGCTGAGTGACTGAACTAC-3' (SEQ ID NO:13) :and
5'-GAATTACTGCTTTTTGCCGCG-3' (SEQ ID NO:14).
As the negative control of above ChIP assay, a fragment 6.2 kb upstream of LXRE on ABCA1 promoter was also amplified with primers
5'-GCAGCCCAACTCTTCAGAAC-3' (SEQ ID NO:15) and
5'TCCCCTTTGTCTTTGTGGAC-3' (SEQ ID NO:16).
For the LXRE of human promoter, PCR reaction was performed using primers 5'-GCTTTCTGCTGAGTGACTGAACTAC-3' (SEQ ID NO:17) and
5'-TGCGTCTCTTTCTCCTACCC-3' (SEQ ID NO:18).
A fragment 7.5 kb upstream of LXRE was amplified with primers
5'-AGGCAGGTGGATCATTTGAG-3' (SEQ ID NO:19) and
5'-CCAAACATCTGGGCTTCTGT-3' (SEQ ID NO:20)
as a negative control. Experiment was performed at least three times, and representative data are shown.

Fig.2d top shows results of probing with LXRE- or upstream-specific PCR probes before (Input) or after (αSIRT1) immunoprecipitation with antibodies to SIRT1. It is seen that in the experimental treatment (αSIRT1) antibody treatment precipitated fragments of chromatin containing the LXRE from *SIRT1*^{+/+} MEFs (left lane, upper image showing a dark gray band), but not *SIRT1*^{-/-} MEF controls (right lane, upper image showing solid black), and did not precipitate fragments containing DNA 6.2 kb upstream of the LXRE from either cell line (lower image, both lanes, showing solid black). Similar results were found in human HEK293T (Fig 2d, bottom) and THP-1 (data not shown) cells transfected with a vector to express FLAG epitope tagged human SIRT 1 or a negative control vector, and precipitated with anti-FLAG antibody.. Thus, SIRT1 binds to the ABCA1 promoter in close proximity to the LXRE.

### Example 8. Interaction of SIRT1 with LXRα and LXRβ

SIRT1 does not directly bind to DNA ²⁶, so its presence on the ABCA1 promoter requires binding to proteins associated with DNA, such as the LXRs. There are two members of the LXR transcriptional factor family, LXRα and LXRβ ^{50,52}. The expression of LXRα is tissue-specific, whereas that of LXRβ is ubiquitous. To determine if there were association between SIRT1 and LXRs by co-immunoprecipitation, murine LXRa and LXRβ cDNAs were cloned into HA-tagged expression vectors. The SIRT1 expression vector pBabe-SIRT1 was then transfected into HEK293T cells with or without the vectors for the HA-tagged LXRs.

In this Example, the possible interaction between SIRT1 and LXRα or LXRβ was examined. Plasmids expressing NH₂-terminal HA-tagged *LXR*α (HA-LXRα) and *LXR*β (HA- LXRβ) were created by cloning the full-length mouse *LXR*α or *LXR*β cDNA downstream of (in-frame with) a HA epitope in a modified pcDNA3-NHA vector. HEK293T cells over-expressing mouse SIRT1 (MmSIRT1) and HA-LXRα, or MmSIRTI and HA-LXRβ, were harvested in NP40 buffer (10 mM Tris-yCl, pH 8.0, 150 mM NaCl, 0.5% NP40, and Complete™ protease inhibitor mixture) and lysed by passing through a 18-gauge syringe needle 10 times. After rocking at 4 °C for 1 h, lysates were subjected to centrifugation at 16,000 x g for 15 min. The resulting supernatant was then diluted into NP40 buffer with 1% BSA and immunoprecipitation was initiated by adding 3 µg of anti-HA monoclonal antibodies (Santa Cruz Biotechnology). The mixture was then incubated with rocking at 4 °C for 2 h to overnight. Immune complexes were recovered by adding protein A beads for additional 1 h and immunoprecipitates were washed five times with 1 ml NP40 buffer. The total lysates and corresponding immuno-precipitation samples were then separated by SDS-PAGE and immuno-blotted with anti-SIRT1 polyclonal antibodies and anti-myc or anti-HA antibodies.

The monoclonal mouse anti-LXRα antibodies were from R&D system and the polyclonal goat anti-LXRα/β antibodies were from Santa Cruz Biotechnology. Human HEK293T cells expressing indicated proteins were lysed and immuno-precipitated (IP) with anti-HA monoclonal antibodies as described above (Fig. 2 e). The total lysates (lanes 1-4) and anti-HA immuno-precipitation samples (lanes 5-8) were then separated by SDS-PAGE and probed with anti-SIRT1 polyclonal antibodies (top images) and with anti-HA-LXRα antibodies (top panel) or anti-HA-LXRβ antibodies (bottom panel). As shown in Fig. 2e, transfected murine SIRT1 protein (mT1) was cross-precipitated by anti-HA antibodies only when the tagged LXRα or LXRβ was co-expressed in the cells (lanes 8, top and bottom panels). HA-LXR also co-precipitated the endogenous human SIRT1 protein (hT1), which exhibited a slightly lower mobility in the gels. Thus, direct or indirect binding of SIRT1 to LXR is probably responsible for the association of SIRT1 with the LXRE of the ABCA1 promoter in intact cells.

Furthermore, the immunoprecipitation experiment described in this Example affords a sensitive assay for ability of a test substance to modulate the interaction of SIRT1 with either LXRα or LXRβ.

### Example 9. LXR target mRNAs in livers of SIRT1^{-/}⁻ mice.

The interaction between LXRs and SIRT1 raises the possibility that loss of function of SIRT1 could impair LXR-mediated lipid homeostasis in response to dietary cholesterol. To test this hypothesis, the mRNA levels of an array of LXR target genes, including ABCA1, were analyzed in the livers *of SIRT1*^{*-*/}*⁻* mice (Fig. 3a), using total RNA from livers of wildtype control animals (filled bars) and *SIRT1*^{*-*/}*⁻* animals (open bars). The analyses were done by real-time PCR. Even though the levels of LXR mRNA were normal in *SIRT1*^{*-*/*-*} liver, many LXR targets, such as SREBP1, ABCA1, ABCG1, ABCG5, and LDLR, were decreased compared to the *SIRT1*^{*+*/}*⁺* controls (n=3, *, P<0.05). Levels of mRNA of control genes known not to be LXR targets ^{53,54} were not significantly altered in *SIRT*^{*-*/*-*}mice.

### Example 10. Relative levels of metabolites in livers of mice treated with the LXR agonist T0901317.

To gain further insight into the effect of SIRT1 on LXR function in *vivo,* wildtype and *SIRT1*^{*-*/}*⁻* animals were analyzed for their response to 8-day oral administration of the LXR agonist T0901317 (N-(2,2,2-trifluoro-ethyl)-N-[4-(2,2,2-tfifluoro-1-hydroxy-1-trifluoromethyl-ethyl)-phenyl]-benzenesulfonamide)⁵³.

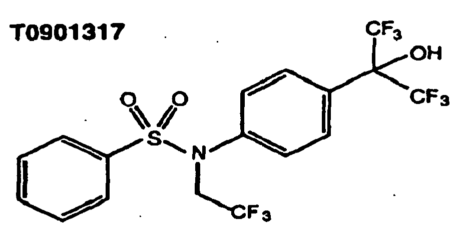

To detect ABCA1 proteins in mouse tissues, SIRT1^{+/+} or SIRT1^{-/-}livers were homogenized in RIPA buffer. Liver extracts were then mixed with SDS sample buffer and denatured at 37 °C for 20 min to prevent the aggregation of ABCA1 at high temperature. Samples were then resolved in 7.5% SDS-PAGE and blotted with anti-ABCA1 polyclonal antibodies (R80 antibodies, gift from Drs. M. Fitzgerald and M. Freeman).

2-6 month old SIRT1-/- and control males were fed with T0901317 (10mg/kg) by oral gavage, plasma were collected before and after feeding for plasma lipid analyses. Tissues were collected after feeding for further analyses. Total RNA from liver of wildtype animals without (black filled bar) or fed with 10mg/kg T0901317 (black striped bar) and *SIRT*^{*-*/*-*}mice without (open bar) or with T0901317 (gray striped bar) were analyzed by real-time PCR. T0901317 feeding of wildtype animals significantly induced the expression of several LXR targets: SREBP-1 c, LPL, and ABCA1 at the RNA level (Fig 3b) (n=4, **, p<0.05). However, the induction of these targets in *SIRT1*^{*-*/}*⁻* mice was blunted. Total protein lysates were obtained from livers of indicated mice and analyzed for the levels of ABCA1 protein by immunoblotting (Fig 3c). (As previously observed, ABCA1 proteins comprise multiple bands by western blotting ⁷⁸.)

Plasma results in Fig. 3, panels d, e, and f represent the same mice before and after 8-days administration of T0901317. Liver results in Fig. 3, panel d compare littermates without or with T0901317, as in Fig. 3, panels b (Example 9) and c (this Example) above. Triglycerides from plasma (Fig. 3 d, left panel) and liver (Fig. 3 d, right panel) of wildtype mice without (black filled bar) or with T0901317 (left striped bar) and *SIRT1*^{*-*/}*⁻* animals without (open bar) or with T0901317 (right striped bar) were analyzed. A reduced response in *SIRT1*^{*-*/}*⁻* animals to LXR agonist T0901317 induction ⁵³ of plasma and hepatic triglyceride accumulation was observed (Fig. 3d; n=5, ***, *p*<0.001). Lower plasma cholesterol levels in treated mice were also seen (Fig 3e; n=5, ****, *p*<0.02) especially in HDL size particles as determined by cholesterol assays of FPLC lipoprotein fractions (Fig. 3f; n=5)). Because T0901317 results in elevation of plasma and hepatic triglycerides through induction of SREBP-1c⁵³, the low levels of SREBP1-c mRNA in *SIRT1*^{*-*/}*⁻* mice (Fig. 3a) may explain the reduction of triglycerides induced by T0901317. Thus, not only was the effect of SIRT1 on the ABCA1 promoter in reporter assays dependent on LXR, the functions ofLXR and its agonist in vivo was also dependent on SIRT1.

### Example 11. Effect of SIRT1 on LXR Protein Expression.

SIRT1 directly interacts with and deacetylates many target proteins, such as histones 12, p53 15-17, FOXO transcriptional factors 18, 19, and PGC-1α 23, 24. Deacetylation of these targets by SIRT1 either represses (p53 and FOXO at some promoters) or activates (PGC-1α and FOXO at other promoters) their transcriptional activities. To investigate how SIRT1 positively regulates LXR, the levels of endogenous LXR protein in the livers of SIRT1+/+ and SIRT1-/- mice were first determined. Surprisingly, LXR protein levels were dramatically increased in SIRT1-/- animals (Fig. 4a), even though the ability ofLXR to activate targets was reduced compared to wildtype (Example 9; Fig. 3a).

HEK293T cells expressing HA-LXRβ driven by an exogenous CMV promoter were transfected either with pSuper vector, or with a pSuper-SIRT1 RNAi construct. Cells were harvested 3 days after transfection and the levels of HA-LXRβ and SIRT1 were analyzed by western blotting using antibodies to HA and SIRT1. It was found that both RNAi-mediated knockdown of SIRT1 expression (Fig. 4b, lanes 3 and 4) and inhibition of SIRT1 activity by nicotinamide - but not inhibition of other deacetylases by TSA - (Fig. 4c) resulted in elevated levels of HA-LXRβ protein. Since there was no detectable reduction of LXR mRNA in SIRT1-/- mice (Fig. 3a), SIRT1 regulates LXR protein levels post-transoriptionally.

HEK293T cells expressing HA-LXRβ were treated with either control medium (Fig. 3c, lane 1), or medium with 1µM TSA (lane 2), 10 mM nicotinamide (lane 3), 25 µM MG132 (carbobenzyloxy-Leu-Leu-Leu-al, a proteasome inhibitor) (lane 4), or 10 mM nicotinamide plus 25 µM MG132 (lane 5) for 6 h. Cells were then harvested and the levels of HA-LXRβ and SIRT1 were analyzed by western blotting using antibodies to HA (upper images) and SIRT1 (middle images). Inhibition of SIRT1 activity by nicotinamide (lane 3) - but not inhibition of other deacetylases by TSA (lane 2) - resulted in elevated levels of HA-LXRβ protein (upper images). Since there was no detectable reduction of LXR's mRNA in **SIRT1-/-** mice (see Example 9, Fig. 3a), **SIRT1** regulates LXR protein levels post-transcriptionally. It is believed that inhibition of SIRT1 activity by nicotinamide increases the protein levels of LXR by interfering with proteasome-mediated LXR degradation.

The basis for the finding that LXR activity is inversely correlated with protein levels was next examined. Since acetylation of lysine residues can block ubiquitination and SIRTI is a deacetylase, loss of SIRT1 activity might stabilize LXR proteins by increasing the steady state level of LXR acetylation, thus inhibiting the ubiquitin/proteasome degradation pathway. If this were true, inhibition of proteasome activity by MG132 should increase the steady state levels of LXR when SIRT1 is active, but not when SIRT1 is inhibited by nicotinamide. This in fact was observed in HEK293T cells expressing HA-tagged LXRβ (Fig. 4c, lane 4 vs. lane 5)).

HEK293T cells were co-transfected with constructs expressing an empty pBabe vector, HA-LXRβ, pBabe-SIRT1, and pBapbe-hSIRTIHY (an inactive deacetylase SIRT1 mutant (see Vaziri et al., 2001)). 40 h later, cells were incubated with 25µM MG132 for 1 h and harvested. HA-LXRβ was then immunoprecipitated using antibodies to HA followed by western blotting using antibodies to ubiquitin, HA and SIRT1. The results show that expression of an active SIRT1 transgene (hSIRT1) but not the inactive mutant hSIRT1HY increased the amount of ubiquitin-conjugated LXR in transfected HEK293T cells (Fig. 4d, lane 3 vs. lane 4).

### Example 12. Acetylation of LXR in vivo

To detect the acetylation of LXR and deacetylation of LXR by SIRT1 in vivo, HEK293T cells co-transfected with HA-LXRβ, and with either pBabe, pBabe-HsSIRT1, or pBabe-HsSIRT1H355Y, were cultured in medium containing sodium [3H]acetate as described ⁷⁷ with modification. Briefly, 40 h after transfection, 2 mCi/ml sodium [3H]acetate, the proteasomal inhibitor MG132 (25 µM), 22(R)-HC (10 µM), and 9-cis-RA (1 µM) were added to the culture for 1 h. Cells were then washed twice with cold phosphate-buffered saline and lysed in NP40 buffer. The lysate was centrifuged at 16,000 × g for 15 min at 4 °C. HA-LXRβ was then immunoprecipitated with anti-HA antibody and separated by SDS-PAGE. Gels containing [3H]acetate-labeled HA-LXRβ were first stained with Coomassie Brilliant Blue and then enhanced by impregnating with a commercial fluorography enhancing solution (Amplify, Amersham Biosciences) for 30 min. Dried gels were subjected to autoradiography at -70 °C for 3-7 days. The same samples were also immuno-blotted with anti-SIRT1 antibodies.

The results are shown in Fig. 4e. HA-LXRβ was acetylated under normal culture conditions (lane 1). Furthermore, activation of LXRβ by treatment with 22(R)-HC + 9-cisRA stimulated deacetylation (and/or possibly inhibited acetylation; lane 2). It appears likely that SIRT1 is responsible, at least in part, for the deacetylation of LXR, because, as shown in Fig. 4f, the levels of [3H] acetate-labeled HA-LXR were substantially lower in cells expressing hSIRT1 (pBabe-hSIRT1; lane 2), than in control cells transfected with an empty vector (pBabe; lane 1) or one encoding an enzymatically inactive SIRT1 (pBabe-hSIRT1HY; lane 3). These findings suggest that deacetylation of LXRβ by SIRT1 both facilitates its turnover and increases its activity.

### Example 13. Effect of inhibition of SIRT1 or proteasome-mediated LXR degradation on the transcription activity of LXR on the ABCA1 promoter.

HEK293T cells transfected with wildtype (WT; Fig. 4g, left panel) or LXRE mutant (-LXRE; Fig. 4g, right panel) mouse ABCA1 promoter driven luciferase reporter vectors were treated with either 10 mM nicotinamide (Nico), 25 µM MG132, or both 10 mM nicotinamide + 25 µM MG132, in the absence (solid bars) or presence (open bars) of 10 µM 22(R)-HC and 1 µM 9-cisRA for 6 h. Luciferase activities were measured and normalized as in Example 5.

It was found that nicotinamide blunted the induction of the luciferase reporter driven by the ABCA1 promoter significantly (Fig. 4g, left panel). Moreover, the proteasome inhibitor MG132 inhibited the reporter to the same extent as nicotinamide, and the combination of nicotinamide and MG132 did not result in a further inhibition. The control reporter missing the LXRE displayed basal activity that was not altered by nicotinamide or MG132 (Fig. 4g, right panel). These findings suggest that the deacetylation of LXRβ by SIRT1 and its subsequent ubiquitination and degradation by the proteasome help activate the ABCA1 promoter, as discussed below.

These Examples show that SIRT1 positively regulates LXR transcription factors, and thus plays an important role in the regulation of cholesterol homeostasis. LXR and SIRT1 activate transcription of gene encoding the ABCA1 transporter, which mediates reverse cholesterol transport and HDL synthesis. These findings may be directly relevant to human medicine, as genetic and epidemiological studies indicate plasma HDL levels are inversely associated with risk of cardiovascular diseases³⁰ and possibly Alzheimer's disease^{5,6}. HDL mediated reverse cholesterol transport may protect against atherosclerosis by clearing excess cholesterol from arterial cells^{55,56} and protect against Alzheimer's disease by decreasing cholesterol-rich raft formation in the brain^{57,58}. By activating LXR, SIRT1 may decrease the risk of ageing associated atherosclerosis and possibly Alzheimer's disease.

The physical and functional interactions between SIRT1 and LXRs suggest that **SIRT1** exerts at least some of its effects in vivo by modulating LXRs. In fact, SIRT1-deficient mice share many metabolic defects with LXR deficient mice^{53,54,63}, such as decreased HDL cholesterol (Fig. 1) and triglyceride levels (Fig. 3d). Also, PEPCK, the rate-limiting enzyme in gluconeogenesis that can be repressed by a liver LXR agonist⁶⁴, is induced in SIRT1-/- mice ¹⁹. LXR dependent gene expression and energy homeostasis have recently been shown to be important for the innate immune response against microbes and mice lacking LXRs are highly susceptible to infection with bacteria⁶⁵. Therefore, it is possible that the observed susceptibility of SIRT1 knockout animals to lung infection²⁶ and eye infection (unpublished observation) is due to a blunted LXR activity in these animals.

The present findings suggest that SIRT1 promotes LXR activity by deacetylating it and causing its turnover (Fig. 5). Regulation ofLXR by SIRT1 places this sirtuin at the center of cholesterol homeostasis pathways

### REFERENCES:

1. Grundy, S. M. Obesity, metabolic syndrome, and coronary atherosclerosis. Circulation 105, 2696-2698 (2002).
2. Kivipelto, M. et al. Midlife vascular risk factors and Alzheimer's disease in later life: longitudinal, population based study. Bmj 322, 1447-51 (2001).
3. Kivipelto, M. et al. Apolipoprotein E epsilon4 allele, elevated midlife total cholesterol level, and high midlife systolic blood pressure are independent risk factors for late-life Alzheimer disease. Ann Intern Med 137, 149-55 (2002).
4. Wolozin, B. Cholesterol and the biology of Alzheimer's disease. Neuron 41, 7-10 (2004).
5. Merched, A., Xia, Y., Visvikis, S., Serot, J. M. & Siest, G. Decreased high-density lipoprotein cholesterol and serum apolipoprotein AI concentrations are highly correlated with the severity of Alzheimer's disease. Neurobiol Aging 21, 27-30 (2000).
6. Bonarek, M. et al. Relationships between cholesterol, apolipoprotein E polymorphism and dementia: a cross-sectional analysis from the PAQUID study. Neuroepidemiology 19, 141-8 (2000).
7. Blander, G. & Guarente, L. The Sir2 family of protein deacetylases. Annu Rev Biochem 73, 417-35 (2004).
8. Wood, J. G. et al. Sirtuin activators mimic caloric restriction and delay ageing in metazoans. Nature 430, 686-9 (2004).
9. Rogina, B. & Helfand, S. L. Sir2 mediates longevity in the fly through a pathway related to calorie restriction. Proc Natl Acad Sci U S A 101, 15998-6003 (2004).
10. Lin, S. J., Defossez, P. A. & Guarente, L. Requirement of NAD and SIR2 for lifespan extension by calorie restriction in Saccharomyces cerevisiae. Science 289, 2126-8 (2000).
11. Lin, S. J. et al. Calorie restriction extends Saccharomyces cerevisiae lifespan by increasing respiration. Nature 418, 344-8 (2002).
12. Imai, S., Armstrong, C. M., Kaeberlein, M. & Guarente, L. Transcriptional silencing and longevity protein Sir2 is an NAD-dependent histone deacetylase. Nature 403, 795-800 (2000).
13. Landry, J. et al. The silencing protein SIR2 and its homologs are NAD-dependent protein deacetylases. Proc Natl Acad Sci U S A 97, 5807-11 (2000).
14. Smith, J. S. et al. A phylogenetically conserved NAD+-dependent protein deacetylase activity in the Sir2 protein family. Proc Natl Acad Sci U S A 97, 6658-63 (2000).
15. Luo, J. et al. Negative control of p53 by Sir2alpha promotes cell survival under stress. Cell 107, 137-48 (2001).
16. Vaziri, H. et al. hSIR2(SIRT1) functions as an NAD-dependent p53 deacetylase. Cell 107, 149-59 (2001).
17. Langley, E. et al. Human SIR2 deacetylates p53 and antagonizes PML/p53-induced cellular senescence. Embo J 21, 2383-96 (2002).
18. Brunet, A. et al. Stress-dependent regulation of FOXO transcription factors by the SIRT1 deacetylase. Science 303, 2011-5 (2004).
19. Motta, M. C. et al. Mammalian SIRT1 represses forkhead transcription factors. Cell 116, 551-63 (2004).
20. Araki, T., Sasaki, Y. & Milbrandt, J. Increased nuclear NAD biosynthesis and SIRT1 activation prevent axonal degeneration. Science 305, 1010-3 (2004).
21. Giannakou, M. E. & Partridge, L. The interaction between FOXO and SIRT1: tipping the balance towards survival. Trends Cell Biol 14, 408-12 (2004).
22. Picard, F. et al. Sirt1 promotes fat mobilization in white adipocytes by repressing PPAR-gamma. Nature 429, 771-6 (2004).
23. Rodgers, J. T. et al. Nutrient control of glucose homeostasis through a complex of PGC-1alpha and S1RT1. Nature 434, 113-8 (2005).
24. Nemoto, S., Fergusson, M. M. & Finkel, T. SIRT1 functionally interacts with the metabolic regulator and transcriptional coactivator PGC-1 {alpha}. J Biol Chem 280, 16456-60 (2005).
25. Rigotti, A., Miettinen, H. E. & Krieger, M. The role of the high-density lipoprotein receptor SR-BI in the lipid metabolism of endocrine and other tissues. Endocr Rev 24, 357-87 (2003).
26. McBurney, M W. et al. The mammalian SIR2alpha protein has a role in embryogenesis and gametogenesis. Mol Cell Biol 23, 38-54 (2003).
27. Cheng, H. L. et al. Developmental defects and p53 hyperacetylation in Sir2 homolog (SIRT1)-deficient mice. Proc Natl Acad Sci U S A (2003).
28. Brewer, H. B., Jr. & Santamarina-Fojo, S. New insights into the role of the adenosine triphosphate-binding cassette transporters in high-density lipoprotein metabolism and reverse cholesterol transport. Am J Cardiol 91, 3E-11E (2003).
29. Krieger, M. Charting the fate of the "good cholesterol": identification and characterization of the HDL receptor SR-BI. Annu Rev Biochem 68, 523-588 (1999).
30. Oram, J. F. & Lawn, R. M. ABCA1: the gatekeeper for eliminating excess tissue cholesterol. J Lipid Research 42, 1173-1179 (2001).
31. Lewis, G. F. & Rader, D. J. New insights into the regulation of HDL metabolism and reverse cholesterol transport. Circ Res 96, 1221-32 (2005).
32. Ji, Y. et al. Scavenger receptor BI promotes high density lipoprotein-mediated cellular cholesterol efflux. J Biol Chem 272, 20982-5 (1997).
33. Liu, T., Krieger, M., Kan, H. Y. & Zannis, V. I. The effects of mutations in helices 4 and 6 of ApoA-I on scavenger receptor class B type I (SR-BI)-mediated cholesterol efflux suggest that formation of a productive complex between reconstituted high density lipoprotein and SR-BI is required for efficient lipid transport. J Biol Chem 277, 21576-84 (2002).
34. Gu, X., Kozarsky, K. & Krieger, M. Scavenger receptor class B, type I-mediated [3H]cholesterol efflux to high and low density lipoproteins is dependent on lipoprotein binding to the receptor. J Biol Chem 275, 29993-30001 (2000).
35. Zhang, Y. et al. Hepatic expression of scavenger receptor class B type I (SR-BI) is a positive regulator of macrophage reverse cholesterol transport in vivo. J Clin Invest 115, 2870-4 (2005).
36. Nakamura, K. et al. Expression and regulation of multiple murine ATP-binding cassette transporter G1 mRNAs/isoforms that stimulate cellular cholesterol efflux to high density lipoprotein. J Biol Chem 279, 45980-9 (2004).
37. Kennedy, M. A. et al. ABCG1 has a critical role in meditating cholesterol efflux to HDL and preventing cellular lipid accumulation. Cell Metab 1, 121-31 (2005).
38. Brooks-Wilson, A. et al. Mutations in ABC1 in Tangier disease and familial high-density lipoprotein deficiency. Nat Genet 22, 336-45 (1999).
39. Bodzioch, M. et al. The gene encoding ATP-binding cassette transporter 1 is mutated in Tangier disease. Nat Genet 22, 347-51 (1999).
40. Rust, S. et al. Tangier disease is caused by mutations in the gene encoding ATP-binding cassette transporter 1. Nat Genet 22, 352-5 (1999).
41. Lawn, R. M. et al. The Tangier disease gene product ABC1 controls the cellular apolipoprotein-mediated lipid removal pathway. J Clin Invest 104, R25-31 (1999).
42. Joyce, C., Freeman, L., Brewer, H. B., Jr. & Santamarina-Fojo, S. Study of ABCA1 function in transgenic mice. Arterioscler Thromb Vasc Biol 23, 965-71 (2003).
43. Oram, J. F., Wolfbauer, G., Vaughan, A. M., Tang, C. & Albers, J. J. Phospholipid transfer protein interacts with and stabilizes ATP-binding cassette transporter A1 and enhances cholesterol efflux from cells. J Biol Chem 278, 52379-85 (2003).
44. Smith, J. D. et al. ABCA1 mediates concurrent cholesterol and phospholipid efflux to apolipoprotein A-I. J Lipid Res 45, 635-44 (2004).
45. Bitterman, K. J., Anderson, R. M., Cohen, H. Y., Latorre-Esteves, M. & Sinclair, D. A. Inhibition of silencing and accelerated aging by nicotinamide, a putative negative regulator of yeast sir2 and human SIRT1. J Biol Chem 277, 45099-45107 (2002).
46. McNeish, J. et al. High density lipoprotein deficiency and foam cell accumulation in mice with targeted disruption of ATP-binding cassette transporter-1. Proc Natl Acad Sci U S A 97, 4245-50 (2000).
47. Costet, P., Luo, Y., Wang, N. & Tall, A. R. Sterol-dependent transactivation of the ABC1 promoter by the liver X receptor/retinoid X receptor. J Biol Chem 275, 28240-28245 (2000).
48. Schwartz, K., Lawn, R. M. & Wade, D. P. ABAC1 gene expression and apoAI-mediated cholesterol efflux are regulated by LXR. Biochem. Biophys. Res. Commun. 274, 794-802 (2000).
49. Venkateswaran, A. et al. Control of cellular cholesterol efflux by the nuclear oxysterol receptor LXRalpha. Proc Natl Acad Sci U S A 97, 12097-12102 (2000).
50. Chawla, A., Repa, J. J., Evans, R. M. & Mangelsdorf, D. J. Nuclear receptors and lipid physiology: opening the X-files. Science 294, 1866-1870 (2001).
51. Steffensen, K. R. & Gustafsson, J. A. Putative metabolic effects of the liver X receptor (LXR). Diabetes 53, S36-S42 (2004).
52. Peet, D. J., Janowski, B. A. & Mangelsdorf, D. J. The LXRs: a new class of oxysterol receptors. Curr Opin Genet Dev 8, 571-5 (1998).
53. Schultz, J. R. et al. Role of LXRs in control of lipogenesis. Genes Dev 14, 2831-2838 (2000).
54. Kalaany, N. Y. et al. LXRs regulate the balance between fat storage and oxidation. Cell Metab 1, 231-44 (2005).
55. von Eckardstein, A., Nofer, J. R. & Assmann, G. High density lipoproteins and arteriosclerosis. Role of cholesterol efflux and reverse cholesterol transport. Arterioscler Thromb Vasc Biol 21, 13-27 (2001).
56. Linsel-Nitschke, P. & Tall, A. R. HDL as a target in the treatment of atherosclerotic cardiovascular disease. Nat Rev Drug Discov 4, 193-205 (2005).
57. Simons, M. et al. Cholesterol depletion inhibits the generation of beta-amyloid in hippocampal neurons. Proc Natl Acad Sci U S A 95, 6460-4 (1998).
58. Simons, K. & Ehehalt, R. Cholesterol, lipid rafts, and disease. J Clin Invest 110, 597-603 (2002).
59. Wang, C. et al. Direct acetylation of the estrogen receptor alpha hinge region by p300 regulates transactivation and hormone sensitivity. J Biol Chem 276, 18375-83 (2001).
60. Fu, M. et al. Androgen receptor acetylation governs trans activation and MEKK1-induced apoptosis without affecting in vitro sumoylation and trans-repression function. Mol Cell Biol 22, 3373-88 (2002).
61. Fu, M. et al. Acetylation of androgen receptor enhances coactivator binding and promotes prostate cancer cell growth. Mol Cell Biol 23, 8563-75 (2003).
62. Fu, M., Wang, C., Zhang, X. & G., P. R. Acetylation of nuclear receptors in cell growth and apoptosis. Biochemical Pharmacology 68, 1199-1208 (2004).
63. Schuster, G. U. et al. Accumulation of foam cells in liver X receptor-deficient mice. Circulation 106, 1147-1153 (2002).
64. Cao, G. et al. Antidiabetic action of a liver X receptor agonist mediated by inhibition of hepatic gluconeogenesis. J Biol Chem 278, 1131-1136 (2003).
65. Joseph, S. B. et al. LXR-dependent gene expression is important for macrophage survival and the innate immune response. Cell 119, 299-309 (2004).
66. Lipford, J. R. & Deshaies, R. J. Diverse roles for ubiquitin-dependent proteolysis in transcriptional activation. Nature cell Biol. 5, 845-850 (2003).
67. Lipford, J. R., Smith, G. T., Chi, Y. & Deshaies, R. J. A putative stimulatory role for activator turnover in gene expression. Nature 438, 113-116 (2005).
68. Pagans, S. et al. SIRT1 regulates HIV transcription via Tat deacetylation. PLoS Biol 3, e41 (2005).
69. Kitamura, Y. I. et al. FoxO1 protects against pancreatic beta cell failure through NeuroD and MafA induction. Cell Metab 2, 153-63 (2005).
70. Lutjohann, D. et al. Cholesterol homeostasis in human brain: evidence for an age-dependent flux of 24S-hydroxycholesterol from the brain into the circulation. Proc Natl Acad Sci U S A 93, 9799-9804 (1996).
71. Lutjohann, D. et al. Profile of cholesterol-related sterols in aged amyloid precursor protein transgenic mouse brain. J Lipid Research 43, 1078-1085 (2002).
72. Chawla, A. et al. APPAR gamma-LXR-ABCA1 pathway in macrophages is involved in cholesterol efflux and atherogenesis. Mol Cell 7, 161-71 (2001).
73. Hogan, B., Beddington, R., Costantini, F. & Lacy, E. Manipulating the Mouse Embryo, a laboratory manual. Second Edition, 260-261 (1994).
74. Vinals, M., Xu, S., Vasile, E. & Krieger, M. Identification of the N-linked glycosylation sites on the high density lipoprotein (HDL) receptor SR-BI and assessment of their effects on HDL binding and selective lipid uptake. J Biol Chem 278, 5325-32 (2003).
75. Parathath, S. et al. Glycine 420 near the C-terminal transmembrane domain of SR-BI is critical for proper delivery and metabolism of high density lipoprotein cholesteryl ester. J Biol Chem 279, 24976-85 (2004).
76. Takahashi, Y., Rayman, J. B. & Dynlacht, B. D. Analysis of promoter binding by the E2F and pRB families in vivo: distinct E2F proteins mediate activation and repression. Genes Dev 14, 804-16 (2000).
77. Blander, G. et al. DNA damage-induced translocation of the Werner helicase is regulated by acetylation. J Biol Chem 277, 50934-40 (2002).
78. Denis, M. et al. Characterization of oligomeric human ATP binding cassette transporter A1. Potential implications for determining the structure of nascent high density lipoprotein particles. J Biol Chem 279, 41529-36 (2004).
79. VandenDriessche, T.; Vanslembrouck, V.; Goovaerts, I.; Zwinnen, H.; Vanderhaeghen, M.-L.; Collen, D.; Chuah, M. K. L. : Long-term expression of human coagulation factor VIII and correction of hemophilia A after in vivo retroviral gene transfer in factor VIII-deficient mice. Proc. Nat. Acad. Sci. 96: 10379-10384 (1999).
80. Roth, D. A.; Tawa, N. E., Jr.; O'Brien, J. M.; Treco, D. A.; Selden, R F. : Nonviral transfer of the gene encoding coagulation factor VIII in patients with severe hemophilia A. New Eng. J. Med. 344: 1735-1742 (2001).
81. Goudy, K.; Song, S.; Wasserfall, C.; Zhang, Y. C.; Kapturczak, M.; Muir, A.; Powers, M.; Scott-Jorgensen, M.; Campbell-Thompson, M.; Crawford, J. M.; Ellis, T. M.; Flotte, T. R.; Atkinson, M. A. : Adeno-associated virus vector-mediated IL-10 gene delivery prevents type 1 diabetes in NOD mice. Proc. Nat. Acad. Sci. 98: 13913-13918 (2001).
82. Wells DJ. Curr Opin Drug Discov Devel. Viral and non-viral methods for gene transfer into skeletal muscle. 9(2):163-8 (2006).
83. Post DE, Shim H, Toussaint-Smith E, Van Meir EG. Cancer scene investigation: how a cold virus became a tumor killer. Future Oncol. 1(2):247-58 (2005).
84. Takahashi S, Ito Y, Hatake K, Sugimoto Y. Gene therapy for breast cancer. --Review of clinical gene therapy trials for breast cancer and MDR1 gene therapy trial in Cancer Institute Hospital.Breast Cancer. 13(1):8-15 (2006).
85. Broedl UC, Rader DJ. Gene therapy for lipoprotein disorders Expert Opin Biol Ther. 5(8):1029-38 (2005).
86. Miyake H, Hara I, Gleave ME. Antisense oligodeoxynucleotide therapy targeting clusterin gene for prostate cancer: Vancouver experience from discovery to clinic. Int J Urol. Sep. 12(9):785-94 (2005).
87. Satoh T, Irie A, Egawa S, Baba S In situ gene therapy for prostate cancer. Curr Gene Ther. 5(1):111-9 (2005).

## Claims

1. An isolated complex comprising a mammalian SIRTI protein and a mammalian LXR protein.

2. The complex described in claim 1 further comprising an LXR response element.

3. A method of forming a complex comprising a mammalian SIRTI protein and a mammalian LXR protein, the method comprising combining a first composition comprising a mammalian SIRTI protein, a second composition comprising a mammalian LXR protein and a third composition comprising a fragment of a cellular nucleic acid that includes a LXR response element.

4. A method of identifying an agent that modulates formation of a complex comprising a mammalian SIRTI protein and a mammalian LXR protein, the method comprising a) combining a first composition comprising a mammalian SIRTI protein, a second composition comprising a mammalian LXR protein and a third composition comprising a fragment of a cellular nucleic acid that includes a LXR response element to provide a complex composition; b) further either i) contacting the first composition, the second composition, or the third composition with a fourth composition comprising the agent prior to the combining step, or ii) contacting the complex composition with the fourth composition comprising the agent after the combining step; thereby providing a test composition, and c) determining whether formation of the complex is modulated by the agent.

5. The method described in claim 4 wherein the agent increases formation of the complex.

6. The method described in claim 4 wherein the determining further comprises d) combining the first composition that includes a mammalian SIRTI protein, the second composition that includes a mammalian LXR protein, and the third composition comprising a fragment of a cellular nucleic acid that includes a LXR response element (LXRE) to provide a control composition not including the agent, and e) determining whether formation of the complex in the test composition is modulated in comparison with formation of the complex in the control composition.

7. A method for assessing whether a candidate substance modulates an SIRTI - dependent effect of an LXR, comprising
a) transfecting a cell with a vector harboring an SIRTI gene;
b) further transfecting the cell with a vector harboring a reporter gene operably driven by an LXRE promoter;
c) contacting the cell with the candidate; and
d) determining whether the candidate modulates the expression of the reporter gene in comparison with a cell not contacted with the candidate; whereby a difference in the extent of the SIRTI -dependent effect of the LXR detected between the presence and absence of the candidate indicates that the candidate modulates the SIRTI - dependent effect of an LXR.

8. A method for assessing whether a candidate substance modulates the formation of a specific binding pair comprising specific binding pair members SIRTI and an LXR, comprising a) transfecting a cell with a vector harboring a sequence encoding an epitope-tagged first member of the specific binding pair;
b) further transfecting the cell with a vector harboring a sequence encoding the second member of the specific binding pair;
c) contacting the cell with the candidate substance;
d) lysing the cells, contacting the cell lysates with an antibody specific for the epitope tag, and recovering immunoprecipitates comprising a complex of the SIRTI and the LXR with an antibody-specific adsorbent;
e) carrying out a Western blot procedure using antibodies specific for SIRTI and an LXR; whereby a difference in complex formation detected in the presence of the candidate compared with the absence of the candidate indicates that the candidate modulates the interaction of the SIRTI with the LXR.

## Patentansprüche

1. Isolierter Komplex, umfassend ein SIRTI-Protein von einem Säuger und ein LXR-Protein von einem Säuger.

2. Komplex nach Anspruch 1, der ferner ein LXR Response Element umfasst.

3. Verfahren zur Bildung eines Komplexes, umfassend ein Säuger-SIRT1-Protein und ein Säuger-LXR-Protein, wobei das Verfahren Folgendes umfasst: Kombinieren einer ersten Zusammensetzung, die ein Säuger-SIRT1-Protein umfasst, einer zweiten Zusammensetzung, die ein Säuger-LXR-Protein umfasst und einer dritten Zusammensetzung, die ein Fragment einer zellulären Nukleinsäure umfasst, das ein LXR Response Element einschließt.

4. Verfahren zur Identifikation eines Mittels, das die Bildung eines Komplexes moduliert, umfassend ein Säuger-SIRTI-Protein und ein Säuger-LXR-Protein, wobei das Verfahren Folgendes umfasst: a) Kombinieren einer ersten Zusammensetzung, umfassend ein Säuger-SIRT1-Protein, einer zweiten Zusammensetzung, umfassend ein Säuger-LXR-Protein und einer dritten Zusammensetzung, umfassend ein Fragment einer zellulären Nukleinsäure, das ein LXR Response Element zur Bereitstellung einer Komplex-Zusammensetzung einschließt; b) ferner entweder i) das Inkontaktbringen der ersten Zusammensetzung, der zweiten Zusammensetzung oder der dritten Zusammensetzung mit der vierten Zusammensetzung, umfassend das Mittel vor dem Kombinationsschritt, oder ii) das Inkontaktbringen der Komplex-Zusammensetzung mit der vierten Zusammensetzung, umfassend das Mittel nach dem Kombinationsschritt; wodurch eine Testzusammensetzung bereitgestellt wird, und c) Bestimmen, ob die Bildung des Komplexes durch das Mittel moduliert wird.

5. Verfahren nach Anspruch 4, worin das Mittel die Bildung des Komplexes steigert.

6. Verfahren nach Anspruch 4, worin die Bestimmung ferner Folgendes umfasst: d) Kombinieren der ersten Zusammensetzung, die ein Säuger-SIRT1-Protein einschließt, der zweiten Zusammensetzung, die ein Säuger-LXR-Protein einschließt und der dritten Zusammensetzung, die ein Fragment einer zellulären Nukleinsäure aufweist, das ein LXR Response Element (LXRE) einschließt, zur Bereitstellung einer Kontrollzusammensetzung, die das Mittel nicht einschließt, und e) Bestimmen, ob die Bildung des Komplexes in der Testzusammensetzung im Vergleich zur Bildung des Komplexes in der Kontrollzusammensetzung moduliert wird.

7. Verfahren zur Beurteilung, ob eine Kandidatensubstanz eine SIRTI-abhängige Wirkung eines LXR moduliert, umfassend:
a) Transfizieren einer Zelle mit einem Vektor, der ein SIRT1-Gen beherbergt;
b) ferner Transfizieren der Zelle mit einem Vektor, der ein Reporter-Gen beherbergt, das durch einen LXRE-Promotor operativ angetrieben wird;
c) Inkontaktbringen der Zelle mit dem Kandidaten; und
d) Bestimmen, ob der Kandidat die Expression des Reportergens moduliert, im Vergleich zu einer Zelle, die nicht mit dem Kandidaten in Kontakt gebracht wird, wobei ein Unterschied im Ausmaß der SIRT1-abhängigen Wirkung des zwischen der An- und Abwesenheit des Kandidaten nachgewiesenen LXR anzeigt, dass der Kandidat die SIRT1-abhängige Wirkung eines LXR moduliert.

8. Verfahren zur Beurteilung, ob eine Kandidatensubstanz die Bildung eines die spezifischen Bindungspaarglieder SIRT1 und LXR umfassenden spezifischen Bindungspaars moduliert, umfassend a) Transfizieren einer Zelle mit einem Vektor, der eine Sequenz beherbergt, die für ein Epitop-getaggtes erstes Glied des spezifischen Bindungspaars kodiert;
b) ferner Transfizieren der Zelle mit einem Vektor, der eine Sequenz beherbergt, die für das zweite Glied des spezifischen Bindungspaars kodiert;
c) Inkontaktbringen der Zelle mit der Kandidatensubstanz;
d) Lysieren der Zellen, Inkontaktbringen der Zelllysate mit einem für das Epitop-Tag-spezifischen Antikörper und Rückgewinnung von Immunpräzipitaten, umfassend einen Komplex aus SIR1 und LXR mit einem Antikörper-spezifischen Adsorbens;
e) Durchführen eines Western Blot-Verfahrens unter Verwendung von für SIRT1 und ein LXR spezifischen Antikörpern, wobei ein in Anwesenheit des Kandidaten im Vergleich zur Abwesenheit des Kandidaten nachgewiesener Unterschied in der Komplexbildung anzeigt, dass der Kandidat die Interaktion von SIR1 mit LXR moduliert.

## Revendications

1. Complexe isolé comprenant une protéine SIRT1 mammifère et une protéine LXR mammifère.

2. Complexe décrit dans la revendication 1, comprenant en outre un élément de réponse à LXR.

3. Procédé pour la formation d'un complexe comprenant une protéine SIRT1 mammifère et une protéine LXR mammifère, le procédé comprenant la combinaison d'une première composition comprenant une protéine SIRT1 mammifère, d'une deuxième composition comprenant une protéine LXR mammifère et d'une troisième composition comprenant un fragment d'un acide nucléique cellulaire qui inclut un élément de réponse à LXR.

4. Procédé pour l'identification d'un agent qui module la formation d'un complexe comprenant une protéine SIRT1 mammifère et une protéine LXR mammifère, le procédé comprenant a) la combinaison d'une première composition comprenant une protéine SIRT1 mammifère, d'une deuxième composition comprenant une protéine LXR mammifère et d'une troisième composition comprenant un fragment d'un acide nucléique cellulaire qui inclut un élément de réponse à LXR pour donner une composition de complexe; b) en outre soit i) la mise en contact de la première composition, de la deuxième composition ou de la troisième composition avec une quatrième composition comprenant l'agent avant l'étape de combinaison, soit ii) la mise en contact de la composition de complexe avec la quatrième composition comprenant l'agent après l'étape de combinaison; donnant ainsi une composition d'essai, et c) la détermination si la formation du complexe est modulée par l'agent.

5. Procédé décrit dans la revendication 4, dans lequel l'agent augmente la formation du complexe.

6. Procédé décrit dans la revendication 4, dans lequel la détermination comprend en outre d) la combinaison de la première composition qui inclut une protéine SIRT1 mammifère, de la deuxième composition qui inclut une protéine LXR mammifère et de la troisième composition comprenant un fragment d'un acide nucléique cellulaire qui inclut un élément de réponse à LXR (LXRE) pour donner une composition témoin n'incluant pas l'agent; et e) la détermination si la formation du complexe dans la composition d'essai est modulée en comparaison à la formation du complexe dans la composition témoin.

7. Procédé pour évaluer si une substance candidate module un effet dépendant de SIRT1 d'une protéine LXR, comprenant:
a) la transfection d'une cellule avec un vecteur hébergeant un gène de SIRT1;
b) en outre la transfection de la cellule avec un vecteur hébergeant un gène rapporteur conduit de manière fonctionnelle par un promoteur de LXRE;
c) la mise en contact de la cellule avec le candidat; et
d) la détermination si le candidat module l'expression du gène rapporteur en comparaison à une cellule qui n'a pas été mise en contact avec le candidat; en conséquence de quoi une différence dans l'ampleur de l'effet dépendant de SIRT1 de la protéine LXR détectée entre la présence et l'absence du candidat indique que le candidat module l'effet dépendant de SIRT1 d'une protéine LXR.

8. Procédé pour évaluer si une substance candidate module la formation d'une paire de liaison spécifique comprenant les membres de la paire de liaison spécifique SIRT1 et LXR, comprenant:
a) la transfection d'une cellule avec un vecteur hébergeant une séquence codant pour un premier membre marqué par un épitope de la paire de liaison spécifique;
b) en outre la transfection de la cellule avec un vecteur hébergeant une séquence codant pour le second membre de la paire de liaison spécifique;
c) la mise en contact de la cellule avec la substance candidate;
d) la lyse des cellules, la mise en contact des lysats cellulaires avec un anticorps spécifique pour le marqueur épitope et la récupération des immuno-précipités comprenant un complexe de SIRT1 et de LXR avec un adsorbant spécifique à l'anticorps;
e) la réalisation d'une procédure de transfert de type Western en utilisant des anticorps spécifiques pour SIRT1 et LXR; en conséquence de quoi une différence dans la formation du complexe détectée en présence du candidat comparée à l'absence du candidat indique que le candidat module l'interaction de SIRT1 avec LXR.


### SEQUENCE LISTING

<110> Massachusetts Institute of Technology
   GUARENTE, Leonard
   KRIEGER, Monty
   LI, Xiaoling
<120> Cholesterol-Regulating Complex of SIRT1 and LXR and Methods of Use
<130> 407-02-PCT
<150> US 60/811,154
   <151> 2006-06-06
<160> 20
<170> PatentIn version 3.3
<210> 1
   <211> 747
   <212> PRT
   <213> Homo sapiens
<400> 1
   Met Ala Asp Glu Ala Ala Leu Ala Leu Gln Pro Gly Gly Ser Pro Ser
   1               5                   10                  15
   Ala Ala Gly Ala Asp Arg Glu Ala Ala Ser Ser Pro Ala Gly Glu Pro
               20                  25                  30
   Leu Arg Lys Arg Pro Arg Arg Asp Gly Pro Gly Leu Glu Arg Ser Pro
           35                  40                  45
   Gly Glu Pro Gly Gly Ala Ala Pro Glu Arg Glu Val Pro Ala Ala Ala
       50                  55                  60
   Arg Gly Cys Pro Gly Ala Ala Ala Ala Ala Leu Trp Arg Glu Ala Glu
   65                  70                  75                  80
   Ala Glu Ala Ala Ala Ala Gly Gly Glu Gln Glu Ala Gln Ala Thr Ala
                   85                  90                  95
   Ala Ala Gly Glu Gly Asp Asn Gly Pro Gly Leu Gln Gly Pro Ser Arg
               100                 105                 110
   Glu Pro Pro Leu Ala Asp Asn Leu Tyr Asp Glu Asp Asp Asp Asp Glu
           115                 120                 125
   Gly Glu Glu Glu Glu Glu Ala Ala Ala Ala Ala Ile Gly Tyr Arg Asp
       130                 135                 140
   Asn Leu Leu Phe Gly Asp Glu Ile Ile Thr Asn Gly Phe His Ser Cys
   145                 150                 155                 160
   Glu Ser Asp Glu Glu Asp Arg Ala Ser His Ala Ser Ser Ser Asp Trp
                   165                 170                 175
   Thr Pro Arg Pro Arg Ile Gly Pro Tyr Thr Phe Val Gln Gln His Leu
               180                 185                 190
   Met Ile Gly Thr Asp Pro Arg Thr Ile Leu Lys Asp Leu Leu Pro Glu
           195                 200                 205
   Thr Ile Pro Pro Pro Glu Leu Asp Asp Met Thr Leu Trp Gln Ile Val
       210                 215                 220
   Ile Asn Ile Leu Ser Glu Pro Pro Lys Arg Lys Lys Arg Lys Asp Ile
   225                 230                 235                 240
   Asn Thr Ile Glu Asp Ala Val Lys Leu Leu Gln Glu Cys Lys Lys Ile
                   245                 250                 255
   Ile Val Leu Thr Gly Ala Gly Val Ser Val Ser Cys Gly Ile Pro Asp
               260                 265                 270
   Phe Arg Ser Arg Asp Gly Ile Tyr Ala Arg Leu Ala Val Asp Phe Pro
           275                 280                 285
   Asp Leu Pro Asp Pro Gln Ala Met Phe Asp Ile Glu Tyr Phe Arg Lys
       290                 295                 300
   Asp Pro Arg Pro Phe Phe Lys Phe Ala Lys Glu Ile Tyr Pro Gly Gln
   305                 310                 315                 320
   Phe Gln Pro Ser Leu Cys His Lys Phe Ile Ala Leu Ser Asp Lys Glu
                   325                 330                 335
   Gly Lys Leu Leu Arg Asn Tyr Thr Gln Asn Ile Asp Thr Leu Glu Gln
               340                 345                 350
   Val Ala Gly Ile Gln Arg Ile Ile Gln Cys His Gly Ser Phe Ala Thr
           355                 360                 365
   Ala Ser Cys Leu Ile Cys Lys Tyr Lys Val Asp Cys Glu Ala Val Arg
       370                 375                 380
   Gly Asp Ile Phe Asn Gln Val Val Pro Arg Cys Pro Arg Cys Pro Ala
   385 390 395 400
   Asp Glu Pro Leu Ala Ile Met Lys Pro Glu Ile Val Phe Phe Gly Glu
   405 410 415
   Asn Leu Pro Glu Gln Phe His Arg Ala Met Lys Tyr Asp Lys Asp Glu
   420 425 430
   Val Asp Leu Leu Ile Val Ile Gly Ser Ser Leu Lys Val Arg Pro Val
   435 440 445
   Ala Leu Ile Pro Ser Ser Ile Pro His Glu Val Pro Gln Ile Leu Ile
   450 455 460
   Asn Arg Glu Pro Leu Pro His Leu His Phe Asp Val Glu Leu Leu Gly
   465 470 475 480
   Asp Cys Asp Val Ile Ile Asn Glu Leu Cys His Arg Leu Gly Gly Glu
   485 490 495
   Tyr Ala Lys Leu Cys Cys Asn Pro Val Lys Leu Ser Glu Ile Thr Glu
   500 505 510
   Lys Pro Pro Arg Thr Gln Lys Glu Leu Ala Tyr Leu Ser Glu Leu Pro
   515 520 525
   Pro Thr Pro Leu His Val Ser Glu Asp Ser Ser Ser Pro Glu Arg Thr
   530 535 540
   Ser Pro Pro Asp Ser Ser Val Ile Val Thr Leu Leu Asp Gln Ala Ala
   545 550 555 560
   Lys Ser Asn Asp Asp Leu Asp Val Ser Glu Ser Lys Gly Cys Met Glu
   565 570 575
   Glu Lys Pro Gln Glu Val Gln Thr Ser Arg Asn Val Glu Ser Ile Ala
   580 585 590
   Glu Gln Met Glu Asn Pro Asp Leu Lys Asn Val Gly Ser Ser Thr Gly
   595 600 605
   Glu Lys Asn Glu Arg Thr Ser Val Ala Gly Thr Val Arg Lys Cys Trp
   610 615 620
   Pro Asn Arg Val Ala Lys Glu Gln Ile Ser Arg Arg Leu Asp Gly Asn
   625 630 635 640
   Gln Tyr Leu Phe Leu Pro Pro Asn Arg Tyr Ile Phe His Gly Ala Glu
   645 650 655
   Val Tyr Ser Asp Ser Glu Asp Asp Val Leu Ser Ser Ser Ser Cys Gly
   660 665 670
   Ser Asn Ser Asp Ser Gly Thr Cys Gln Ser Pro Ser Leu Glu Glu Pro
   675 680 685
   Met Glu Asp Glu Ser Glu Ile Glu Glu Phe Tyr Asn Gly Leu Glu Asp
   690 695 700
   Glu Pro Asp Val Pro Glu Arg Ala Gly Gly Ala Gly Phe Gly Thr Asp
   705 710 715 720
   Gly Asp Asp Gln Glu Ala Ile Asn Glu Ala Ile Ser Val Lys Gln Glu
   725 730 735
   Val Thr Asp Met Asn Tyr Pro Ser Asn Lys Ser
   740 745
<210> 2
   <211> 737
   <212> PRT
   <213> Mus musculus
<400> 2
   Met Ala Asp Glu Val Ala Leu Ala Leu Gln Ala Ala Gly Ser Pro Ser
   1               5                   10                  15
   Ala Ala Ala Ala Met Glu Ala Ala Ser Gln Pro Ala Asp Glu Pro Leu
               20                  25                  30
   Arg Lys Arg Pro Arg Arg Asp Gly Pro Gly Leu Gly Arg Ser Pro Gly
           35                  40                  45
   Glu Pro Ser Ala Ala Val Ala Pro Ala Ala Ala Gly Cys Glu Ala Ala
       50                  55                  60
   Ser Ala Ala Ala Pro Ala Ala Leu Trp Arg Glu Ala Ala Gly Ala Ala
   65                  70                  75                  80
   Ala Ser Ala Glu Arg Glu Ala Pro Ala Thr Ala Val Ala Gly Asp Gly
                   85                  90                  95
   Asp Asn Gly Ser Gly Leu Arg Arg Glu Pro Arg Ala Ala Asp Asp Phe
   100 105 110
   Asp Asp Asp Glu Gly Glu Glu Glu Asp Glu Ala Ala Ala Ala Ala Ala
   115 120 125
   Ala Ala Ala Ile Gly Tyr Arg Asp Asn Leu Leu Leu Thr Asp Gly Leu
   130 135 140
   Leu Thr Asn Gly Phe His Ser Cys Glu Ser Asp Asp Asp Asp Arg Thr
   145 150 155 160
   Ser His Ala Ser Ser Ser Asp Trp Thr Pro Arg Pro Arg Ile Gly Pro
   165 170 175
   Tyr Thr Phe Val Gln Gln His Leu Met Ile Gly Thr Asp Pro Arg Thr
   180 185 190
   Ile Leu Lys Asp Leu Leu Pro Glu Thr Ile Pro Pro Pro Glu Leu Asp
   195 200 205
   Asp Met Thr Leu Trp Gln Ile Val Ile Asn Ile Leu Ser Glu Pro Pro
   210 215 220
   Lys Arg Lys Lys Arg Lys Asp Ile Asn Thr Ile Glu Asp Ala Val Lys
   225 230 235 240
   Leu Leu Gln Glu Cys Lys Lys Ile Ile Val Leu Thr Gly Ala Gly Val
   245 250 255
   Ser Val Ser Cys Gly Ile Pro Asp Phe Arg Ser Arg Asp Gly Ile Tyr
   260 265 270
   Ala Arg Leu Ala Val Asp Phe Pro Asp Leu Pro Asp Pro Gln Ala Met
   275 280 285
   Phe Asp Ile Glu Tyr Phe Arg Lys Asp Pro Arg Pro Phe Phe Lys Phe
   290 295 300
   Ala Lys Glu Ile Tyr Pro Gly Gln Phe Gln Pro Ser Leu Cys His Lys
   305 310 315 320
   Phe Ile Ala Leu Ser Asp Lys Glu Gly Lys Leu Leu Arg Asn Tyr Thr
   325 330 335
   Gln Asn Ile Asp Thr Leu Glu Gln Val Ala Gly Ile Gln Arg Ile Leu
   340 345 350
   Gln Cys His Gly Ser Phe Ala Thr Ala Ser Cys Leu Ile Cys Lys Tyr
   355 360 365
   Lys Val Asp Cys Glu Ala Val Arg Gly Asp Ile Phe Asn Gln Val Val
   370 375 380
   Pro Arg Cys Pro Arg Cys Pro Ala Asp Glu Pro Leu Ala Ile Met Lys
   385 390 395 400
   Pro Glu Ile Val Phe Phe Gly Glu Asn Leu Pro Glu Gln Phe His Arg
   405 410 415
   Ala Met Lys Tyr Asp Lys Asp Glu Val Asp Leu Leu Ile Val Ile Gly
   420 425 430
   Ser Ser Leu Lys Val Arg Pro Val Ala Leu Ile Pro Ser Ser Ile Pro
   435 440 445
   His Glu Val Pro Gln Ile Leu Ile Asn Arg Glu Pro Leu Pro His Leu
   450 455 460
   His Phe Asp Val Glu Leu Leu Gly Asp Cys Asp Val Ile Ile Asn Glu
   465 470 475 480
   Leu Cys His Arg Leu Gly Gly Glu Tyr Ala Lys Leu Cys Cys Asn Pro
   485 490 495
   Val Lys Leu Ser Glu Ile Thr Glu Lys Pro Pro Arg Pro Gln Lys Glu
   500 505 510
   Leu Val His Leu Ser Glu Leu Pro Pro Thr Pro Leu His Ile Ser Glu
   515 520 525
   Asp Ser Ser Ser Pro Glu Arg Thr Val Pro Gln Asp Ser Ser Val Ile
   530 535 540
   Ala Thr Leu Val Asp Gln Ala Thr Asn Asn Asn Val Asn Asp Leu Glu
   545 550 555 560
   Val Ser Glu Ser Ser Cys Val Glu Glu Lys Pro Gln Glu Val Gln Thr
   565 570 575
   Ser Arg Asn Val Glu Asn Ile Asn Val Glu Asn Pro Asp Phe Lys Ala
   580 585 590
   Val Gly Ser Ser Thr Ala Asp Lys Asn Glu Arg Thr Ser Val Ala Glu
   595 600 605
   Thr Val Arg Lys Cys Trp Pro Asn Arg Leu Ala Lys Glu Gln Ile Ser
   610 615 620
   Lys Arg Leu Glu Gly Asn Gln Tyr Leu Phe Val Pro Pro Asn Arg Tyr
   625 630 635 640
   Ile Phe His Gly Ala Glu Val Tyr Ser Asp Ser Glu Asp Asp Val Leu
   645 650 655
   Ser Ser Ser Ser Cys Gly Ser Asn Ser Asp Ser Gly Thr Cys Gln Ser
   660 665 670
   Pro Ser Leu Glu Glu Pro Leu Glu Asp Glu Ser Glu Ile Glu Glu Phe
   675 680 685
   Tyr Asn Gly Leu Glu Asp Asp Thr Glu Arg Pro Glu Cys Ala Gly Gly
   690 695 700
   Ser Gly Phe Gly Ala Asp Gly Gly Asp Gln Glu Val Val Asn Glu Ala
   705 710 715 720
   Ile Ala Thr Arg Gln Glu Leu Thr Asp Val Asn Tyr Pro Ser Asp Lys
   725 730 735
   Ser
<210> 3
   <211> 447
   <212> PRT
   <213> Homo sapiens
<400> 3
   Met Ser Leu Trp Leu Gly Ala Pro Val Pro Asp Ile Pro Pro Asp Ser
   1 5 10 15
   Ala Val Glu Leu Trp Lys Pro Gly Ala Gln Asp Ala Ser Ser Gln Ala
   20 25 30
   Gln Gly Gly Ser Ser Cys Ile Leu Arg Glu Glu Ala Arg Met Pro His
   35 40 45
   Ser Ala Gly Gly Thr Ala Gly Val Gly Leu Glu Ala Ala Glu Pro Thr
   50 55 60
   Ala Leu Leu Thr Arg Ala Glu Pro Pro Ser Glu Pro Thr Glu Ile Arg
   65 70 75 80
   Pro Gln Lys Arg Lys Lys Gly Pro Ala Pro Lys Met Leu Gly Asn Glu
   85 90 95
   Leu Cys Ser Val Cys Gly Asp Lys Ala Ser Gly Phe His Tyr Asn Val
   100 105 110
   Leu Ser Cys Glu Gly Cys Lys Gly Phe Phe Arg Arg Ser Val Ile Lys
   115 120 125
   Gly Ala His Tyr Ile Cys His Ser Gly Gly His Cys Pro Met Asp Thr
   130 135 140
   Tyr Met Arg Arg Lys Cys Gln Glu Cys Arg Leu Arg Lys Cys Arg Gln
   145 150 155 160
   Ala Gly Met Arg Glu Glu Cys Val Leu Ser Glu Glu Gln Ile Arg Leu
   165 170 175
   Lys Lys Leu Lys Arg Gln Glu Glu Glu Gln Ala His Ala Thr Ser Leu
   180 185 190
   Pro Pro Arg Arg Ser Ser Pro Pro Gln Ile Leu Pro Gln Leu Ser Pro
   195 200 205
   Glu Gln Leu Gly Met Ile Glu Lys Leu Val Ala Ala Gln Gln Gln Cys
   210 215 220
   Asn Arg Arg Ser Phe Ser Asp Arg Leu Arg Val Thr Pro Trp Pro Met
   225 230 235 240
   Ala Pro Asp Pro His Ser Arg Glu Ala Arg Gln Gln Arg Phe Ala His
   245 250 255
   Phe Thr Glu Leu Ala Ile Val Ser Val Gln Glu Ile Val Asp Phe Ala
   260 265 270
   Lys Gln Leu Pro Gly Phe Leu Gln Leu Ser Arg Glu Asp Gln Ile Ala
   275 280 285
   Leu Leu Lys Thr Ser Ala Ile Glu Val Met Leu Leu Glu Thr Ser Arg
   290 295 300
   Arg Tyr Asn Pro Gly Ser Glu Ser Ile Thr Phe Leu Lys Asp Phe Ser
   305 310 315 320
   Tyr Asn Arg Glu Asp Phe Ala Lys Ala Gly Leu Gln Val Glu Phe Ile
   325 330 335
   Asn Pro Ile Phe Glu Phe Ser Arg Ala Met Asn Glu Leu Gln Leu Asn
   340 345 350
   Asp Ala Glu Phe Ala Leu Leu Ile Ala Ile Ser Ile Phe Ser Ala Asp
   355 360 365
   Arg Pro Asn Val Gln Asp Gln Leu Gln Val Glu Arg Leu Gln His Thr
   370 375 380
   Tyr Val Glu Ala Leu His Ala Tyr Val Ser Ile His His Pro His Asp
   385 390 395 400
   Arg Leu Met Phe Pro Arg Met Leu Met Lys Leu Val Ser Leu Arg Thr
   405 410 415
   Leu Ser Ser Val His Ser Glu Gln Val Phe Ala Leu Arg Leu Gln Asp
   420 425 430
   Lys Lys Leu Pro Pro Leu Leu Ser Glu Ile Trp Asp Val His Glu
   435 440 445
<210> 4
   <211> 461
   <212> PRT
   <213> Homo sapiens
<400> 4
   Met Ser Ser Pro Thr Thr Ser Ser Leu Asp Thr Pro Leu Pro Gly Asn
   1 5 10 15
   Gly Pro Pro Gln Pro Gly Ala Pro Ser Ser Ser Pro Thr Val Lys Glu
   20 25 30
   Glu Gly Pro Glu Pro Trp Pro Gly Gly Pro Asp Pro Asp Val Pro Gly
   35 40 45
   Thr Asp Glu Ala Ser Ser Ala Cys Ser Thr Asp Trp Val Ile Pro Asp
   50 55 60
   Pro Glu Glu Glu Pro Glu Arg Lys Arg Lys Lys Gly Pro Ala Pro Lys
   65 70 75 80
   Met Leu Gly His Glu Leu Cys Arg Val Cys Gly Asp Lys Ala Ser Gly
   85 90 95
   Phe His Tyr Asn Val Leu Ser Cys Glu Gly Cys Lys Gly Phe Phe Arg
   100 105 110
   Arg Ser Val Val Arg Gly Gly Ala Arg Arg Tyr Ala Cys Arg Gly Gly
   115 120 125
   Gly Thr Cys Gln Met Asp Ala Phe Met Arg Arg Lys Cys Gln Gln Cys
   130 135 140
   Arg Leu Arg Lys Cys Lys Glu Ala Gly Met Arg Glu Gln Cys Val Leu
   145 150 155 160
   Ser Glu Glu Gln Ile Arg Lys Lys Lys Ile Arg Lys Gln Gln Gln Gln
   165 170 175
   Glu Ser Gln Ser Gln Ser Gln Ser Pro Val Gly Pro Gln Gly Ser Ser
   180 185 190
   Ser Ser Ala Ser Gly Pro Gly Ala Ser Pro Gly Gly Ser Glu Ala Gly
   195 200 205
   Ser Gln Gly Ser Gly Glu Gly Glu Gly Val Gln Leu Thr Ala Ala Gln
   210 215 220
   Glu Leu Met Ile Gln Gln Leu Val Ala Ala Gln Leu Gln Cys Asn Lys
   225 230 235 240
   Arg Ser Phe Ser Asp Gln Pro Lys Val Thr Pro Trp Pro Leu Gly Ala
   245 250 255
   Asp Pro Gln Ser Arg Asp Ala Arg Gln Gln Arg Phe Ala His Phe Thr
   260 265 270
   Glu Leu Ala Ile Ile Ser Val Gln Glu Ile Val Asp Phe Ala Lys Gln
   275 280 285
   Val Pro Gly Phe Leu Gln Leu Gly Arg Glu Asp Gln Ile Ala Leu Leu
   290 295 300
   Lys Ala Ser Thr Ile Glu Ile Met Leu Leu Glu Thr Ala Arg Arg Tyr
   305 310 315 320
   Asn His Glu Thr Glu Cys Ile Thr Phe Leu Lys Asp Phe Thr Tyr Ser
   325 330 335
   Lys Asp Asp Phe His Arg Ala Gly Leu Gln Val Glu Phe Ile Asn Pro
   340 345 350
   Ile Phe Glu Phe Ser Arg Ala Met Arg Arg Leu Gly Leu Asp Asp Ala
   355 360 365
   Glu Tyr Ala Leu Leu Ile Ala Ile Asn Ile Phe Ser Ala Asp Arg Pro
   370 375 380
   Asn Val Gln Glu Pro Gly Arg Val Glu Ala Leu Gln Gln Pro Tyr Val
   385 390 395 400
   Glu Ala Leu Leu Ser Tyr Thr Arg Ile Lys Arg Pro Gln Asp Gln Leu
   405 410 415
   Arg Phe Pro Arg Met Leu Met Lys Leu Val Ser Leu Arg Thr Leu Ser
   420 425 430
   Ser Val His Ser Glu Gln Val Phe Ala Leu Arg Leu Gln Asp Lys Lys
   435 440 445
   Leu Pro Pro Leu Leu Ser Glu Ile Trp Asp Val His Glu
   450 455 460
<210> 5
   <211> 445
   <212> PRT
   <213> Mus musculus
<400> 5
   Met Ser Leu Trp Leu Glu Ala Ser Met Pro Asp Val Ser Pro Asp Ser
   1 5 10 15
   Ala Thr Glu Leu Trp Lys Thr Glu Pro Gln Asp Ala Gly Asp Gln Gly
   20 25 30
   Gly Asn Thr Cys Ile Leu Arg Glu Glu Ala Arg Met Pro Gln Ser Thr
   35 40 45
   Gly Val Ala Leu Gly Ile Gly Leu Glu Ser Ala Glu Pro Thr Ala Leu
   50 55 60
   Leu Pro Arg Ala Glu Thr Leu Pro Glu Pro Thr Glu Leu Arg Pro Gln
   65 70 75 80
   Lys Arg Lys Lys Gly Pro Ala Pro Lys Met Leu Gly Asn Glu Leu Cys
   85 90 95
   Ser Val Cys Gly Asp Lys Ala Ser Gly Phe His Tyr Asn Val Leu Ser
   100 105 110
   Cys Glu Gly Cys Lys Gly Phe Phe Arg Arg Ser Val Ile Lys Gly Ala
   115 120 125
   Arg Tyr Val Cys His Ser Gly Gly His Cys Pro Met Asp Thr Tyr Met
   130 135 140
   Arg Arg Lys Cys Gln Glu Cys Arg Leu Arg Lys Cys Arg Gln Ala Gly
   145 150 155 160
   Met Arg Glu Glu Cys Val Leu Ser Glu Glu Gln Ile Arg Leu Lys Lys
   165 170 175
   Leu Lys Arg Gln Glu Glu Glu Gln Ala Gln Ala Thr Ser Val Ser Pro
   180 185 190
   Arg Val Ser Ser Pro Pro Gln Val Leu Pro Gln Leu Ser Pro Glu Gln
   195 200 205
   Leu Gly Met Ile Glu Lys Leu Val Ala Ala Gln Gln Gln Cys Asn Arg
   210 215 220
   Arg Ser Phe Ser Asp Arg Leu Arg Val Thr Pro Trp Pro Ile Ala Pro
   225 230 235 240
   Asp Pro Gln Ser Arg Glu Ala Arg Gln Gln Arg Phe Ala His Phe Thr
   245 250 255
   Glu Leu Ala Ile Val Ser Val Gln Glu Ile Val Asp Phe Ala Lys Gln
   260 265 270
   Leu Pro Gly Phe Leu Gln Leu Ser Arg Glu Asp Gln Ile Ala Leu Leu
   275 280 285
   Lys Thr Ser Ala Ile Glu Val Met Leu Leu Glu Thr Ser Arg Arg Tyr
   290 295 300
   Asn Pro Gly Ser Glu Ser Ile Thr Phe Leu Lys Asp Phe Ser Tyr Asn
   305 310 315 320
   Arg Glu Asp Phe Ala Lys Ala Gly Leu Gln Val Glu Phe Ile Asn Pro
   325 330 335
   Ile Phe Glu Phe Ser Arg Ala Met Asn Glu Leu Gln Leu Asn Asp Ala
   340 345 350
   Glu Phe Ala Leu Leu Ile Ala Ile Ser Ile Phe Ser Ala Asp Arg Pro
   355 360 365
   Asn Val Gln Asp Gln Leu Gln Val Glu Arg Leu Gln His Thr Tyr Val
   370 375 380
   Glu Ala Leu His Ala Tyr Val Ser Ile Asn His Pro His Asp Arg Leu
   385 390 395 400
   Met Phe Pro Arg Met Leu Met Lys Leu Val Ser Leu Arg Thr Leu Ser
   405 410 415
   Ser Val His Ser Glu Gln Val Phe Ala Leu Arg Leu Gln Asp Lys Lys
   420 425 430
   Leu Pro Pro Leu Leu Ser Glu Ile Trp Asp Val His Glu
   435 440 445
<210> 6
   <211> 446
   <212> PRT
   <213> Mus musculus
<400> 6
   Met Ser Ser Pro Thr Ser Ser Leu Asp Thr Pro Val Pro Gly Asn Gly
   1 5 10 15
   Ser Pro Gln Pro Ser Thr Ser Ala Thr Ser Pro Thr Ile Lys Glu Glu
   20 25 30
   Gly Gln Glu Thr Asp Pro Pro Pro Gly Ser Glu Gly Ser Ser Ser Ala
   35 40 45
   Tyr Ile Val Val Ile Leu Glu Pro Glu Asp Glu Pro Glu Arg Lys Arg
   50 55 60
   Lys Lys Gly Pro Ala Pro Lys Met Leu Gly His Glu Leu Cys Arg Val
   65 70 75 80
   Cys Gly Asp Lys Ala Ser Gly Phe His Tyr Asn Val Leu Ser Cys Glu
   85 90 95
   Gly Cys Lys Gly Phe Phe Arg Arg Ser Val Val His Gly Gly Ala Gly
   100 105 110
   Arg Tyr Ala Cys Arg Gly Ser Gly Thr Cys Gln Met Asp Ala Phe Met
   115 120 125
   Arg Arg Lys Cys Gln Leu Cys Arg Leu Arg Lys Cys Lys Glu Ala Gly
   130 135 140
   Met Arg Glu Gln Cys Val Leu Ser Glu Glu Gln Ile Arg Lys Lys Arg
   145 150 155 160
   Ile Gln Lys Gln Gln Gln Gln Gln Pro Pro Pro Pro Ser Glu Pro Ala
   165 170 175
   Ala Ser Ser Ser Gly Arg Pro Ala Ala Ser Pro Gly Thr Ser Glu Ala
   180 185 190
   Ser Ser Gln Gly Ser Gly Glu Gly Glu Gly Ile Gln Leu Thr Ala Ala
   195 200 205
   Gln Glu Leu Met Ile Gln Gln Leu Val Ala Ala Gln Leu Gln Cys Asn
   210 215 220
   Lys Arg Ser Phe Ser Asp Gln Pro Lys Val Thr Pro Trp Pro Leu Gly
   225 230 235 240
   Ala Asp Pro Gln Ser Arg Asp Ala Arg Gln Gln Arg Phe Ala His Phe
   245 250 255
   Thr Glu Leu Ala Ile Ile Ser Val Gln Glu Ile Val Asp Phe Ala Lys
   260 265 270
   Gln Val Pro Gly Phe Leu Gln Leu Gly Arg Glu Asp Gln Ile Ala Leu
   275 280 285
   Leu Lys Ala Ser Thr Ile Glu Ile Met Leu Leu Glu Thr Ala Arg Arg
   290 295 300
   Tyr Asn His Glu Thr Glu Cys Ile Thr Phe Leu Lys Asp Phe Thr Tyr
   305 310 315 320
   Ser Lys Asp Asp Phe His Arg Ala Gly Leu Gln Val Glu Phe Ile Asn
   325 330 335
   Pro Ile Phe Glu Phe Ser Arg Ala Met Arg Arg Leu Gly Leu Asp Asp
   340 345 350
   Ala Glu Tyr Ala Leu Leu Ile Ala Ile Asn Ile Phe Ser Ala Asp Arg
   355 360 365
   Pro Asn Val Gln Glu Pro Ser Arg Val Glu Ala Leu Gln Gln Pro Tyr
   370 375 380
   Val Glu Ala Leu Leu Ser Tyr Thr Arg Ile Lys Arg Pro Gln Asp Gln
   385 390 395 400
   Leu Arg Phe Pro Arg Met Leu Met Lys Leu Val Ser Leu Arg Thr Leu
   405 410 415
   Ser Ser Val His Ser Glu Gln Val Phe Ala Leu Arg Leu Gln Asp Lys
   420 425 430
   Lys Leu Pro Pro Leu Leu Ser Glu Ile Trp Asp Val His Glu
   435 440 445
<210> 7
   <211> 4107
   <212> DNA
   <213> Homo sapiens
<400> 7
   gtcgagcggg agcagaggag gcgagggagg agggccagag aggcagttgg aagatggcgg      60
   acgaggcggc cctcgccctt cagcccggcg gctccccctc ggcggcgggg gccgacaggg     120
   aggccgcgtc gtcccccgcc ggggagccgc tccgcaagag gccgcggaga gatggtcccg     180
   gcctcgagcg gagcccgggc gagcccggtg gggcggcccc agagcgtgag gtgccggcgg     240
   cggccagggg ctgcccgggt gcggcggcgg cggcgctgtg gcgggaggcg gaggcagagg     300
   cggcggcggc aggcggggag caagaggccc aggcgactgc ggcggctggg gaaggagaca     360
   atgggccggg cctgcagggc ccatctcggg agccaccgct ggccgacaac ttgtacgacg     420
   aagacgacga cgacgagggc gaggaggagg aagaggcggc ggcggcggcg attgggtacc     480
   gagataacct tctgttcggt gatgaaatta tcactaatgg ttttcattcc tgtgaaagtg     540
   atgaggagga tagagcctca catgcaagct ctagtgactg gactccaagg ccacggatag     600
   gtccatatac ttttgttcag caacatctta tgattggcac agatcctcga acaattctta     660
   aagatttatt gccggaaaca atacctccac ctgagttgga tgatatgaca ctgtggcaga 720
   ttgttattaa tatcctttca gaaccaccaa aaaggaaaaa aagaaaagat attaatacaa 780
   ttgaagatgc tgtgaaatta ctgcaagagt gcaaaaaaat tatagttcta actggagctg 840
   gggtgtctgt ttcatgtgga atacctgact tcaggtcaag ggatggtatt tatgctcgcc 900
   ttgctgtaga cttcccagat cttccagatc ctcaagcgat gtttgatatt gaatatttca 960
   gaaaagatcc aagaccattc ttcaagtttg caaaggaaat atatcctgga caattccagc 1020
   catctctctg tcacaaattc atagccttgt cagataagga aggaaaacta cttcgcaact 1080
   atacccagaa catagacacg ctggaacagg ttgcgggaat ccaaaggata attcagtgtc 1140
   atggttcctt tgcaacagca tcttgcctga tttgtaaata caaagttgac tgtgaagctg 1200
   tacgaggaga tatttttaat caggtagttc ctcgatgtcc taggtgccca gctgatgaac 1260
   cgcttgctat catgaaacca gagattgtgt tttttggtga aaatttacca gaacagtttc 1320
   atagagccat gaagtatgac aaagatgaag ttgacctcct cattgttatt gggtcttccc 1380
   tcaaagtaag accagtagca ctaattccaa gttccatacc ccatgaagtg cctcagatat 1440
   taattaatag agaacctttg cctcatctgc attttgatgt agagcttctt ggagactgtg 1500
   atgtcataat taatgaattg tgtcataggt taggtggtga atatgccaaa ctttgctgta 1560
   accctgtaaa gctttcagaa attactgaaa aacctccacg aacacaaaaa gaattggctt 1620
   atttgtcaga gttgccaccc acacctcttc atgtttcaga agactcaagt tcaccagaaa 1680
   gaacttcacc accagattct tcagtgattg tcacactttt agaccaagca gctaagagta 1740
   atgatgattt agatgtgtct gaatcaaaag gttgtatgga agaaaaacca caggaagtac 1800
   aaacttctag gaatgttgaa agtattgctg aacagatgga aaatccggat ttgaagaatg 1860
   ttggttctag tactggggag aaaaatgaaa gaacttcagt ggctggaaca gtgagaaaat 1920
   gctggcctaa tagagtggca aaggagcaga ttagtaggcg gcttgatggt aatcagtatc 1980
   tgtttttgcc accaaatcgt tacattttcc atggcgctga ggtatattca gactctgaag 2040
   atgacgtctt atcctctagt tcttgtggca gtaacagtga tagtgggaca tgccagagtc 2100
   caagtttaga agaacccatg gaggatgaaa gtgaaattga agaattctac aatggcttag 2160
   aagatgagcc tgatgttcca gagagagctg gaggagctgg atttgggact gatggagatg 2220
   atcaagaggc aattaatgaa gctatatctg tgaaacagga agtaacagac atgaactatc 2280
   catcaaacaa atcatagtgt aataattgtg caggtacagg aattgttcca ccagcattag 2340
   gaactttagc atgtcaaaat gaatgtttac ttgtgaactc gatagagcaa ggaaaccaga 2400
   aaggtgtaat atttataggt tggtaaaata gattgttttt catggataat ttttaacttc 2460
   attatttctg tacttgtaca aactcaacac taactttttt ttttttaaaa aaaaaaaggt 2520
   actaagtatc ttcaatcagc tgttggtcaa gactaacttt cttttaaagg ttcatttgta 2580
   tgataaattc atatgtgtat atataatttt ttttgttttg tctagtgagt ttcaacattt 2640
   ttaaagtttt caaaaagcca tcggaatgtt aaattaatgt aaagggacag ctaatctaga 2700
   ccaaagaatg gtattttcac ttttctttgt aacattgaat ggtttgaagt actcaaaatc 2760
   tgttacgcta aacttttgat tctttaacac aattattttt aaacactggc attttccaaa 2820
   actgtggcag ctaacttttt aaaatctcaa atgacatgca gtgtgagtag aaggaagtca 2880
   acaatatgtg gggagagcac tcggttgtct ttacttttaa aagtaatact tggtgctaag 2940
   aatttcagga ttattgtatt tacgttcaaa tgaagatggc ttttgtactt cctgtggaca 3000
   tgtagtaatg tctatattgg ctcataaaac taacctgaaa aacaaataaa tgctttggaa 3060
   atgtttcagt tgctttagaa acattagtgc ctgcctggat ccccttagtt ttgaaatatt 3120
   tgccattgtt gtttaaatac ctatcactgt ggtagagctt gcattgatct tttccacaag 3180
   tattaaactg ccaaaatgtg aatatgcaaa gcctttctga atctataata atggtacttc 3240
   tactggggag agtgtaatat tttggactgc tgttttccat taatgaggag agcaacaggc 3300
   ccctgattat acagttccaa agtaataaga tgttaattgt aattcagcca gaaagtacat 3360
   gtctcccatt gggaggattt ggtgttaaat accaaactgc tagccctagt attatggaga 3420
   tgaacatgat gatgtaactt gtaatagcag aatagttaat gaatgaaact agttcttata 3480
   atttatcttt atttaaaagc ttagcctgcc ttaaaactag agatcaactt tctcagctgc 3540
   aaaagcttct agtctttcaa gaagttcata ctttatgaaa ttgcacagta agcatttatt 3600
   tttcagacca tttttgaaca tcactcctaa attaataaag tattcctctg ttgctttagt 3660
   atttattaca ataaaaaggg tttgaaatat agctgttctt tatgcataaa acacccagct 3720
   aggaccatta ctgccagaga aaaaaatcgt attgaatggc catttcccta cttataagat 3780
   gtctcaatct gaatttattt ggctacacta aagaatgcag tatatttagt tttccatttg 3840
   catgatgttt gtgtgctata gatgatattt taaattgaaa agtttgtttt aaattatttt 3900
   tacagtgaag actgttttca gctcttttta tattgtacat agtcttttat gtaatttact 3960
   ggcatatgtt ttgtagactg tttaatgact ggatatcttc cttcaacttt tgaaatacaa 4020
   aaccagtgtt ttttacttgt acactgtttt aaagtctatt aaaattgtca tttgactttt 4080
   ttctgttaaa aaaaaaaaaa aaaaaaa 4107
<210> 8
   <211> 3849
   <212> DNA
   <213> Mus musculus
<400> 8
   gcggagcaga ggaggcgagg gcggagggcc agagaggcag ttggaagatg gcggacgagg 60
   tggcgctcgc ccttcaggcc gccggctccc cttccgcggc ggccgccatg gaggccgcgt 120
   cgcagccggc ggacgagccg ctccgcaaga ggccccgccg agacgggcct ggcctcgggc 180
   gcagcccggg cgagccgagc gcagcagtgg cgccggcggc cgcggggtgt gaggcggcga 240
   gcgccgcggc cccggcggcg ctgtggcggg aggcggcagg ggcggcggcg agcgcggagc 300
   gggaggcccc ggcgacggcc gtggccgggg acggagacaa tgggtccggc ctgcggcggg 360
   agccgagggc ggctgacgac ttcgacgacg acgagggcga ggaggaggac gaggcggcgg 420
   cggcagcggc ggcggcagcg atcggctacc gagacaacct cctgttgacc gatggactcc 480
   tcactaatgg ctttcattcc tgtgaaagtg atgacgatga cagaacgtca cacgccagct 540
   ctagtgactg gactccgcgg ccgcggatag gtccatatac ttttgttcag caacatctca 600
   tgattggcac cgatcctcga acaattctta aagatttatt accagaaaca attcctccac 660
   ctgagctgga tgatatgacg ctgtggcaga ttgttattaa tatcctttca gaaccaccaa 720
   agcggaaaaa aagaaaagat atcaatacaa ttgaagatgc tgtgaagtta ctgcaggagt 780
   gtaaaaagat aatagttctg actggagctg gggtttctgt ctcctgtggg attcctgact 840
   tcagatcaag agacggtatc tatgctcgcc ttgcggtgga cttcccagac ctcccagacc 900
   ctcaagccat gtttgatatt gagtatttta gaaaagaccc aagaccattc ttcaagtttg 960
   caaaggaaat atatcccgga cagttccagc cgtctctgtg tcacaaattc atagctttgt 1020
   cagataagga aggaaaacta cttcgaaatt atactcaaaa tatagatacc ttggagcagg 1080
   ttgcaggaat ccaaaggatc cttcagtgtc atggttcctt tgcaacagca tcttgcctga 1140
   tttgtaaata caaagttgat tgtgaagctg ttcgtggaga catttttaat caggtagttc 1200
   ctcggtgccc taggtgccca gctgatgagc cacttgccat catgaagcca gagattgtct 1260
   tctttggtga aaacttacca gaacagtttc atagagccat gaagtatgac aaagatgaag 1320
   ttgacctcct cattgttatt ggatcttctc tgaaagtgag accagtagca ctaattccaa 1380
   gttctatacc ccatgaagtg cctcaaatat taataaatag ggaacctttg cctcatctac 1440
   attttgatgt agagctcctt ggagactgcg atgttataat taatgagttg tgtcataggc 1500
   taggtggtga atatgccaaa ctttgttgta accctgtaaa gctttcagaa attactgaaa 1560
   aacctccacg cccacaaaag gaattggttc atttatcaga gttgccacca acacctcttc 1620
   atatttcgga agactcaagt tcacctgaaa gaactgtacc acaagactct tctgtgattg 1680
   ctacacttgt agaccaagca acaaacaaca atgttaatga tttagaagta tctgaatcaa 1740
   gttgtgtgga agaaaaacca caagaagtac agactagtag gaatgttgag aacattaatg 1800
   tggaaaatcc agattttaag gctgttggtt ccagtactgc agacaaaaat gaaagaactt 1860
   cagttgcaga aacagtgaga aaatgctggc ctaatagact tgcaaaggag cagattagta 1920
   agcggcttga gggtaatcaa tacctgtttg taccaccaaa tcgttacata ttccacggtg 1980
   ctgaggtata ctcagactct gaagatgacg tcttgtcctc tagttcctgt ggcagtaaca 2040
   gtgacagtgg cacatgccag agtccaagtt tagaagaacc cttggaagat gaaagtgaaa 2100
   ttgaagaatt ctacaatggc ttggaagatg atacggagag gcccgaatgt gctggaggat 2160
   ctggatttgg agctgatgga ggggatcaag aggttgttaa tgaagctata gctacaagac 2220
   aggaattgac agatgtaaac tatccatcag acaaatcata acactattga agctgtccgg 2280
   attcaggaat tgctccacca gcattgggaa ctttagcatg tcaaaaaaat gaatgtttac 2340
   ttgtgaactt gaacaaggaa atctgaaaga tgtattattt atagactgga aaatagattg 2400
   tcttcttgga taatttctaa agttccatca tttctgtttg tacttgtaca ttcaacactg 2460
   ttggttgact tcatcttcct ttcaaggttc atttgtatga tacattcgta tgtatgtata 2520
   attttgtttt ttgcctaatg agtttcaacc ttttaaagtt ttcaaaagcc attggaatgt 2580
   taatgtaaag ggaacagctt atctagacca aagaatggta tttcacactt ttttgtttgt 2640
   aacattgaat agtttaaagc cctcaatttc tgttctgctg aacttttatt tttaggacag 2700
   ttaacttttt aaacactggc attttccaaa acttgtggca gctaactttt taaaatcaca 2760
   gatgacttgt aatgtgagga gtcagcaccg tgtctggagc actcaaaact tgggctcagt 2820
   gtgtgaagcg tacttactgc atcgtttttg tacttgctgc agacgtggta atgtccaaac 2880
   aggcccctga gactaatctg ataaatgatt tggaaatgtg tttcagttgt tctagaaaca 2940
   atagtgcctg tctatatagg tccccttagt ttgaatattt gccattgttt aattaaatac 3000
   ctatcactgt ggtagagcct gcatagatct tcaccacaaa tactgccaag atgtgaatat 3060
   gcaaagcctt tctgaatcta ataatggtac ttctactggg gagagtgtaa tattttggac 3120
   tgctgttttt ccattaatga ggaaagcaat aggcctctta attaaagtcc caaagtcata 3180
   agataaattg tagctcaacc agaaagtaca ctgttgcctg ttgaggattt ggtgtaatgt 3240
   atcccaaggt gttagccttg tattatggag atgaatacag atccaatagt caaatgaaac 3300
   tagttcttag ttatttaaaa gcttagcttg ccttaaaact agggatcaat tttctcaact 3360
   gcagaaactt ttagcctttc aaacagttca cacctcagaa agtcagtatt tattttacag 3420
   acttctttgg aacattgccc ccaaatttaa atattcatgt gggtttagta tttattacaa 3480
   aaaaatgatt tgaaatatag ctgttcttta tgcataaaat acccagttag gaccattact 3540
   gccagaggag aaaagtatta agtagctcat ttccctacct aaaagataac tgaatttatt 3600
   tggctacact aaagaatgca gtatatttag ttttccattt gcatgatgtg tttgtgctat 3660
   agacaatatt ttaaattgaa aaatttgttt taaattattt ttacagtgaa gactgttttc 3720
   agctcttttt atattgtaca tagactttta tgtaatctgg catatgtttt gtagaccgtt 3780
   taatgactgg attatcttcc tccaactttt gaaatacaaa aacagtgttt tatactaaaa 3840
   aaaaaaaaa 3849
<210> 9
   <211> 1528
   <212> DNA
   <213> Homo sapiens
<400> 9
   cagtgccttg gtaatgacca gggctccaga aagagatgtc cttgtggctg ggggcccctg 60
   tgcctgacat tcctcctgac tctgcggtgg agctgtggaa gccaggcgca caggatgcaa 120
   gcagccaggc ccagggaggc agcagctgca tcctcagaga ggaagccagg atgccccact 180
   ctgctggggg tactgcaggg gtggggctgg aggctgcaga gcccacagcc ctgctcacca 240
   gggcagagcc cccttcagaa cccacagaga tccgtccaca aaagcggaaa aaggggccag 300
   cccccaaaat gctggggaac gagctatgca gcgtgtgtgg ggacaaggcc tcgggcttcc 360
   actacaatgt tctgagctgc gagggctgca agggattctt ccgccgcagc gtcatcaagg 420
   gagcgcacta catctgccac agtggcggcc actgccccat ggacacctac atgcgtcgca 480
   agtgccagga gtgtcggctt cgcaaatgcc gtcaggctgg catgcgggag gagtgtgtcc 540
   tgtcagaaga acagatccgc ctgaagaaac tgaagcggca agaggaggaa caggctcatg 600
   ccacatcctt gccccccagg cgttcctcac ccccccaaat cctgccccag ctcagcccgg 660
   aacaactggg catgatcgag aagctcgtcg ctgcccagca acagtgtaac cggcgctcct 720
   tttctgaccg gcttcgagtc acgccttggc ccatggcacc agatccccat agccgggagg 780
   cccgtcagca gcgctttgcc cacttcactg agctggccat cgtctctgtg caggagatag 840
   ttgactttgc taaacagcta cccggcttcc tgcagctcag ccgggaggac cagattgccc 900
   tgctgaagac ctctgcgatc gaggtgatgc ttctggagac atctcggagg tacaaccctg 960
   ggagtgagag tatcaccttc ctcaaggatt tcagttataa ccgggaagac tttgccaaag 1020
   cagggctgca agtggaattc atcaacccca tcttcgagtt ctccagggcc atgaatgagc 1080
   tgcaactcaa tgatgccgag tttgccttgc tcattgctat cagcatcttc tctgcagacc 1140
   ggcccaacgt gcaggaccag ctccaggtgg agaggctgca gcacacatat gtggaagccc 1200
   tgcatgccta cgtctccatc caccatcccc atgaccgact gatgttccca cggatgctaa 1260
   tgaaactggt gagcctccgg accctgagca gcgtccactc agagcaagtg tttgcactgc 1320
   gtctgcagga caaaaagctc ccaccgctgc tctctgagat ctgggatgtg cacgaatgac 1380
   tgttctgtcc ccatattttc tgttttcttg gccggatggc tgaggcctgg tggctgcctc 1440
   ctagaagtgg aacagactga gaagggcaaa cattcctggg agctgggcaa ggagatcctc 1500
   ccgtggcatt aaaagagagt caaagggt 1528
<210> 10
   <211> 2037
   <212> DNA
   <213> Homo sapiens
<400> 10
   ctcttccgga cgtgacgcaa gggcggggtt gccggaagaa gtggcgaagt tacttttgag      60
   ggtatttgag tagcggcggt gtgtcagggg ctaaagagga ggacgaagaa aagcagagca     120
   agggaaccca gggcaacagg agtagttcac tccgcgagag gccgtccacg agacccccgc     180
   gcgcagccat gagccccgcc ccccgctgtt gcttggagag gggcgggacc tggagagagg     240
   ctgctccgtg accccaccat gtcctctcct accacgagtt ccctggatac ccccctgcct     300
   ggaaatggcc cccctcagcc tggcgcccct tcttcttcac ccactgtaaa ggaggagggt     360
   ccggagccgt ggcccggggg tccggaccct gatgtcccag gcactgatga ggccagctca     420
   gcctgcagca cagactgggt catcccagat cccgaagagg aaccagagcg caagcgaaag     480
   aagggcccag ccccgaagat gctgggccac gagctttgcc gtgtctgtgg ggacaaggcc     540
   tccggcttcc actacaacgt gctcagctgc gaaggctgca agggcttctt ccggcgcagt     600
   gtggtccgtg gtggggccag gcgctatgcc tgccggggtg gcggaacctg ccagatggac     660
   gctttcatgc ggcgcaagtg ccagcagtgc cggctgcgca agtgcaagga ggcagggatg     720
   agggagcagt gcgtcctttc tgaagaacag atccggaaga agaagattcg gaaacaacag     780
   cagcaggagt cacagtcaca gtcgcagtca cctgtggggc cgcagggcag cagcagctca     840
   gcctctgggc ctggggcttc ccctggtgga tctgaggcag gcagccaggg ctccggggaa     900
   ggcgagggtg tccagctaac agcggctcaa gaactaatga tccagcagtt ggtggcggcc     960
   caactgcagt gcaacaaacg ctccttctcc gaccagccca aagtcacgcc ctggcccctg    1020
   ggcgcagacc cccagtcccg agatgcccgc cagcaacgct ttgcccactt cacggagctg    1080
   gccatcatct cagtccagga gatcgtggac ttcgctaagc aagtgcctgg tttcctgcag    1140
   ctgggccggg aggaccagat cgccctcctg aaggcatcca ctatcgagat catgctgcta    1200
   gagacagcca ggcgctacaa ccacgagaca gagtgtatca ccttcttgaa ggacttcacc    1260
   tacagcaagg acgacttcca ccgtgcaggc ctgcaggtgg agttcatcaa ccccatcttc    1320
   gagttctcgc gggccatgcg gcggctgggc ctggacgacg ctgagtacgc cctgctcatc    1380
   gccatcaaca tcttctcggc cgaccggccc aacgtgcagg agccgggccg cgtggaggcg 1440
   ttgcagcagc cctacgtgga ggcgctgctg tcctacacgc gcatcaagag gccgcaggac 1500
   cagctgcgct tcccgcgcat gctcatgaag ctggtgagcc tgcgcacgct gagctctgtg 1560
   cactcggagc aggtcttcgc cttgcggctc caggacaaga agctgccgcc tctgctgtcg 1620
   gagatctggg acgtccacga gtgaggggct ggccacccag ccccacagcc ttgcctgacc 1680
   accctccagc agatagacgc cggcacccct tcctcttcct agggtggaag ggccctgggc 1740
   ccgagcctgt agacctatcg gctctcatcc cttgggataa gccccagtcc aggtccagga 1800
   ggctccctcc ctgcccagcg agtcttccag aaggggtgaa agggttgcag gtcccgacca 1860
   ctgacccttc ccggctgccc tccctcccca gcttacacct caagcccagc acgcagtgca 1920
   ccttgaacag agggagggga ggacccatgg ctctcccccc tagcccggga gaccaggggc 1980
   cttcctcttc ctctgctttt atttaataaa aactaaaaac agaaaaaaaa aaaaaaa 2037
<210> 11
   <211> 1753
   <212> DNA
   <213> Mus musculus
<400> 11
   gggaacgctg actctggagg ctgctgggat tagggtgggg gtgactgaga agcagtcctt   60
   ctgtcagagc aaagagcctc cagggtgagg agaggaagga gagagatgga actagaccgg  120
   tctgcgggga aacgcgacag ttttggtaga gggacagtgt cttggtaatg tccagggctc  180
   caggaagaga tgtccttgtg gctggaggcc tcaatgcctg atgtttctcc tgattctgca  240
   acggagttgt ggaagacaga acctcaagat gcaggagacc agggaggcaa cacttgcatc  300
   ctcagggagg aagccaggat gccccagtca actggggttg ctttagggat agggttggag  360
   tcagcagagc ctacagccct gctccccagg gcagagaccc tcccagagcc gacagagctt  420
   cgtccacaaa agcggaaaaa gggcccagcc cccaaaatgc tggggaacga gctgtgcagt  480
   gtctgtgggg acaaagcctc tggcttccat tacaacgtgc tgagctgcga gggctgcaag  540
   ggattcttcc gccgcagtgt catcaaggga gcacgctatg tctgccacag cggtggccac  600
   tgccccatgg acacctacat gcggcggaaa tgccaggagt gtcgacttcg caaatgccgc  660
   caggcaggca tgagggagga gtgtgtgctg tcagaagaac agatccgctt gaagaaactg  720
   aagcggcaag aagaggaaca ggctcaagcc acttcggtgt ccccaagggt gtcctcacct  780
   cctcaagtcc tgccacagct cagcccagag cagctgggca tgatcgagaa gctggtggct  840
   gcccagcaac agtgtaacag gcgctccttc tcagaccgcc tgcgcgtcac gccttggccc  900
   attgcacccg accctcagag ccgggaagcc cgacaacagc gctttgccca ctttactgag  960
   ctggccatcg tgtccgtgca ggagattgtt gactttgcca aacagctccc tggcttccta  1020
   cagctcagca gggaggacca gatcgccttg ctgaagacct ctgcaatcga ggtcatgctt  1080
   ctggagacgt cacggaggta caaccccggc agtgagagca tcaccttcct caaggacttc  1140
   agttacaacc gggaagactt tgccaaagca gggctgcagg tggagttcat caaccccatc  1200
   tttgagttct ccagagccat gaatgagctg caactcaatg atgctgagtt tgctctgctc  1260
   attgccatca gcatcttctc tgcagaccgg cccaacgtgc aggaccagct ccaagtagag  1320
   aggctgcaac acacatatgt ggaggccctg cacgcctacg tctccatcaa ccacccccac  1380
   gaccgactga tgttcccacg gatgctaatg aagctggtga gcctccgtac tttgagcagc  1440
   gtccattcag agcaagtgtt tgcccttcgc ctgcaggaca aaaagcttcc ccctctgctg  1500
   tctgagatct gggatgtcca cgagtgactg tttcaccgtg tcctttgtgt tggccacatg  1560
   gcgaaggctc actgactgct tcccacgggt ggagcagact gagaagggca gacattcctg  1620
   ggagctgggt gaaggagaga gccttgcgta gcattaaggg agagtcaaca ggttgggtgt  1680
   tttctggctg ctgggcagtt gggatctact aacgttgtat accatctgaa gaccttgttg  1740
   acccaaccaa ata                                                      1753
<210> 12
   <211> 1964
   <212> DNA
   <213> Mus musculus
<400> 12
   ggcgaagtta cttttgcttt tcgctcagca agcgctgttg cttcgagcta ctcccaggct      60
   tctgaagtta cttccaaagt gctgtggagg cacaatcacc ggtgcggaca cagaggcaac     120
   tctcgcctcc cacggccgtt tccagggcaa cagagtcgga gaccccctgc gacccccctc     180
   ccgatcgccg gtgcagtcat gagccccgcc tccccctggt gcacggagag gggcggggcc     240
   tggaacaagc aggctgcttc gtgacccact atgtcttccc ccacaagttc tctggacact     300
   cccgtgcctg ggaatggttc tcctcagccc agtacctccg ccacgtcacc cactattaag     360
   gaagaggggc aggagactga tcctcctcca ggctctgaag ggtccagctc tgcctacatc     420
   gtggtcatct tagagccaga ggatgagcct gagcgcaagc ggaagaaggg gccggccccg     480
   aagatgctgg gccatgagct gtgccgcgtg tgcggagaca aggcctcggg cttccactac     540
   aacgtgctca gctgtgaagg ctgcaaaggc ttcttccggc gcagtgtggt ccacggtggg     600
   gccgggcgct atgcctgtcg gggcagcgga acctgccaga tggatgcctt catgcggcgc     660
   aagtgccagc tctgccggct gcgcaagtgc aaggaggctg gcatgcggga gcagtgcgtg     720
   ctctctgagg agcagattcg gaagaaaagg attcagaagc agcaacagca gcagccacca     780
   cccccatctg agccagcagc cagcagctca ggccggccag cggcctcccc tggcacttcg 840
   gaagcaagca gccagggctc cggggaagga gagggcatcc agctgaccgc ggctcaggag 900
   ctgatgatcc agcagttagt tgccgcgcag ctgcagtgca acaaacgatc tttctccgac 960
   cagcccaaag tcacgccctg gcccctgggt gcagaccctc agtcccgaga tgcccgtcag 1020
   caacgctttg cccacttcac cgagctagcc atcatctcgg tccaggagat tgtggacttt 1080
   gccaagcagg tgccagggtt cttgcagttg ggccgggagg accagatcgc cctcctgaag 1140
   gcgtccacca ttgagatcat gttgctagaa acagccagac gctacaacca cgagacagaa 1200
   tgcatcacgt tcctgaagga cttcacctac agcaaggacg acttccaccg tgcaggcttg 1260
   caggtggaat tcatcaatcc catcttcgag ttctcgcggg ccatgcggcg gctgggcctg 1320
   gacgatgcag agtatgcctt gcttatcgcc atcaacatct tctcagccga tcggcctaat 1380
   gtgcaggagc ccagccgtgt ggaggccctg cagcagccat acgtggaggc gctcctctcc 1440
   tacacgagga tcaagcgccc acaggaccag ctccgcttcc cacgcatgct catgaagctg 1500
   gtgagcctgc gcaccctcag ctccgtgcac tcggagcagg tctttgcatt gcgactccag 1560
   gacaagaagc tgccgccctt gctgtccgag atctgggatg tgcacgagta ggggcagcca 1620
   caagtgcccc agccttggtg gtgtcttctt gaagatggac tcttcacctc tcctcctggg 1680
   gtgggaggac attgtcacgg cccagtccct cgggctcagc ctcaaactca gcggcagttg 1740
   gcactagaag gccccacccc acccattgag tcttccaaga gtggtgaggg tcacaggtcc 1800
   tagcctctga ccgttcccag ctgccctccc acccacgctt acacctcagc ctaccacacc 1860
   atgcaccttg agtggagaga ggttagggca ggtggccccc cacagttggg agaccacagg 1920
   ccctctcttc tgcccctttt atttaataaa aaaacaaaaa taaa 1964
<210> 13
   <211> 25
   <212> DNA
   <213> Artificial
<220>
   <223> PCR primer
<400> 13
   gctttctgct gagtgactga actac 25
<210> 14
   <211> 21
   <212> DNA
   <213> Artificial
<220>
   <223> PCR primer
<400> 14
   gaattactgc tttttgccgc 21
<210> 15
   <211> 20
   <212> DNA
   <213> Artificial
<220>
   <223> PCR primer
<400> 15
   gcagcccaac tcttcagaac 20
<210> 16
   <211> 20
   <212> DNA
   <213> Artificial
<220>
   <223> PCR primer
<400> 16
   tcccctttgt ctttgtggac 20
<210> 17
   <211> 25
   <212> DNA
   <213> Artificial
<220>
   <223> PCR primer
<400> 17
   gctttctgct gagtgactga actac 25
<210> 18
   <211> 20
   <212> DNA
   <213> Artificial
<220>
   <223> PCR primer
<400> 18
   tgcgtctctt tctcctaccc 20
<210> 19
   <211> 20
   <212> DNA
   <213> Artificial
<220>
   <223> PCR primer
<400> 19
   aggcaggtgg atcatttgag 20
<210> 20
   <211> 20
   <212> DNA
   <213> Artificial
<220>
   <223> PCR primer
<400> 20
   ccaaacatct gggcttctgt 20